(19) Europäisches Patentamt
European Patent Office
Office européen des brevets

(11) EP 4 596 693 A1

(12) **EUROPEAN PATENT APPLICATION**
published in accordance with Art. 153(4) EPC

(43) Date of publication:
**06.08.2025 Bulletin 2025/32**

(21) Application number: **23866946.9**

(22) Date of filing: **18.05.2023**

(51) International Patent Classification (IPC):
***C12N 15/113** (2010.01)* ***A61K 31/713** (2006.01)*
***A61P 7/00** (2006.01)* ***A61P 7/06** (2006.01)*

(52) Cooperative Patent Classification (CPC):
**A61K 31/713; A61P 7/00; A61P 7/06; C12N 15/113**

(86) International application number:
**PCT/CN2023/095104**

(87) International publication number:
**WO 2024/060649 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.09.2022 CN 202211203337**
**19.01.2023 CN 202310088165**

(71) Applicant: **Bebetter Med Inc.**
**Guangzhou, Guangdong 510660 (CN)**

(72) Inventors:
- **LIU, Xinjian**
  **Guangzhou, Guangdong 510660 (CN)**
- **FAN, Fushun**
  **Guangzhou, Guangdong 510660 (CN)**
- **QIAN, Changgeng**
  **Guangzhou, Guangdong 510660 (CN)**

(74) Representative: **Fuchs Patentanwälte Partnerschaft mbB**
**Tower 185**
**Friedrich-Ebert-Anlage 35-37**
**60327 Frankfurt am Main (DE)**

Remarks:
The complete document including Reference Table(s) and the Sequence Listing(s) can be downloaded from the EPO website

# (54) SIRNA OR SALT THEREOF AND MEDICAMENT FOR INHIBITING EXPRESSION OF TMPRSS6 GENE, AND USE THEREOF

(57) The present invention provides an siRNA or a pharmaceutically acceptable salt thereof for inhibiting TMPRSS6 expression in human cells, as well as appropriate modifications of the siRNA to enhance target silencing efficiency and minimize off-target activity. The invention further provides biological agent or pharmaceutical compositions containing the foregoing siRNA for inhibiting TMPRSS6 expression. Through a series of in vitro and in vivo experiments, the present invention has identified siRNA sequences with potent biological activity in suppressing TMPRS S6 expression, demonstrating higher efficacy than TMPRSS6-HCM-9, which is currently the most advanced siRNA in clinical development. These findings support the potential clinical application of the invention in treating disorders associated with dysregulated TMPRSS6 expression or diseases related to iron excess or iron overload, demonstrating significant therapeutic potential.

EP 4 596 693 A1

## Description

The present invention claims the benefit of priority to Chinese Patent Application No.CN2022112033373 filed on September 29, 2022, entitled "Sirna or salt thereof and medicament for inhibiting expression of TMPRSS6 gene, and use thereof ". The entire contents of the foregoing application are hereby incorporated herein by reference. The present invention also claims the benefit of priority to Chinese Patent Application No.CN2023100881658 filed on January 19, 2023, entitled " Sirna or salt thereof and medicament for inhibiting expression of TMPRSS6 gene, and use thereof ". The entire contents of the foregoing application are also hereby incorporated herein by reference.

## BACKGROUND

The present disclosure related to the field of biomedical sciences, and in particular to an siRNA or a pharmaceutically acceptable salt thereof for inhibiting the expression of the TMPRSS6 gene, medicaments, and applications thereof.

Description of Related Art

RNA interference (RNAi) is a process in which messenger RNA (mRNA) is cleaved by small interfering RNA (siRNA), and is widely used for studying the regulation of gene expression in various cells. Delivery of siRNA into cells in vitro can significantly inhibit gene expression (Jackson et al., 2006, RNA 12:1179-1187). In recent years, RNA interference (RNAi) has become one of the most widely used gene knockout technologies (Hu et al., 2020, Signal Transduct Target Ther 5:101). Small interfering RNA (siRNA) or short single-stranded RNA such as microRNA (miRNA) forms complexes with the RNA-induced silencing complex (RISC), and subsequently binds to complementary mRNA, which is then cleaved with the help of ribonucleases to inhibit the expression of specific genes. The mechanism of RNAi can protect cells from viral infections and transposable elements (Weingartner et al., 2020, Mol Ther Nucleic Acids 21:242-250).

Beta-thalassemia is one of the most common genetic diseases, typically caused by mutations in the beta-globin gene or its promoter, leading to reduced or absent synthesis of beta-globin (Cao and Kan, 2013, Cold Spring Harb Perspect Med 3:a011775; Rivella, 2015, Haematologica 100:418-430). More than 180 different beta-globin gene mutations have been identified in beta-thalassemia patients, which may affect gene function at any level of transcription, processing of primary messenger RNA transcripts, translation, or post-translational stability of the gene product. Beta-thalassemia typically manifests as ineffective erythropoiesis accompanied by extramedullary hematopoiesis, splenomegaly, and systemic iron overload. Affected individuals exhibit extreme clinical heterogeneity, ranging from nearly asymptomatic to life-threatening severe anemia. Beta-thalassemia can be divided into beta-thalassemia major and relatively less severe anemia (non-transfusion-dependent thalassemia or thalassemia intermedia) (Musallam et al., 2021, Am J Hematol 96:1518-1531).

In thalassemia patients, the main regulator of iron homeostasis, hepcidin, is chronically suppressed. This suppression leads to uncontrolled dietary iron absorption and insufficient iron retention in the reticuloendothelial system. Patients absorb abnormally high levels of iron, exacerbating ineffective erythropoiesis and anemia, which necessitates iron chelation to prevent clinical sequelae associated with iron overload (Richard et al., 2020, Am J Hematol 95:68-77). Regulation of iron homeostasis in patients is necessary to prevent toxicity due to iron excess (Nualkaew et al., 2021, Mol Ther 29:2841-2853). If left untreated, iron overload can lead to organ failure and death. Consequently, management of iron overload in β-thalassemia and other iron-related diseases has become a major focus (Beliveau et al., 2019, Cell Chem Biol 26:1559-1572).The production of hepcidin is primarily regulated by a transmembrane serine protease encoded by the TMPRSS6 gene. TMPRSS6 (transmembrane protease, serine 6), also known as matriptase-2, is a type II serine protease. It is predominantly expressed in the liver, but high levels of TMPRSS6 mRNA have also been detected in the kidney. TMPRSS6 plays a role in iron homeostasis by inhibiting the activation of hepcidin through cleavage of hemojuvelin, and inhibition of TMPRSS6 expression can regulate iron homeostasis, reduce iron overload, and thus alleviate symptoms of thalassemia (Ginzburg and Rivella, 2011, Blood 118:4321-4330).

TMPRSS6-HCM-9 is an siRNA targeting TMPRSS6, which is currently undergoing a Phase 1 clinical trials for the treatment of thalassemia. Previous phase 1 clinical trials of SLN124 in healthy volunteers have demonstrated that SLN124 effectively reduced plasma iron levels, exhibited a favorable safety profile, and had a prolonged duration of action. The sequence of SLN124 is disclosed in patent US_11174483_B2. Currently,there are limited therapeutic options available for the treatment of thalassemia, and an siRNA-based therapy has good market prospects.

Ineffective erythropoiesis and chronic anemia result in iron metabolism disorders and systemic iron overload, which in turn leads to cardiac, hepatic, and endocrine dysfunction. Treatment options for thalassemia remain limited, with red blood cell transfusion being the primary therapeutic approach(Longo et al.,2021,Int J Mol Sci 22). There is an urgent need for novel methods to effectively treat disorders associated with iron overload.

Polycythemia is classified into secondary polycythemia and polycythemia vera. Secondary polycythemia can be

alleviated by addressing the underlying cause. Polycythemia vera (PV) is a myeloproliferative disorder (MPDS) that often leads to abnormally high hematocrit (Hct) and hemoglobin (Hb) concentrations, which can result in thromboembolic complications. Loss of function of the TMPRSS6 gene leads to increased production of hepcidin, thereby improving abnormal erythropoiesis and preventing or limiting iron overload. Targeting TMPRSS6 with oligonucleotides provides an approach for preventing or treating polycythemia. Currently, an antisense oligonucleotide drug targeting TMPRSS6, IONIS-TMPRSS6-LRx (ISIS 702843), is in phase 2 clinical trials for the treatment of polycythemia vera. Developing additional siRNA-based therapies will contribute to treating these disorders and provide more clinical options.

SUMMARY

Based on this, the objective of the present invention provides a double-stranded siRNA or a pharmaceutically acceptable salt thereof for inhibiting TMPRSS6 expression, as well as its applications, which exhibits robust in vitro and in vivo efficacy with reduced off-target activity.

The first aspect of the present invention provides an siRNA or a pharmaceutically acceptable salt thereof for inhibiting TMPRSS6 expression. The siRNA comprises a sense strand and an antisense strand, which form a double-stranded structure through base pairing. The nucleotide sequence is selected from any one of the sequences (a) to (e) as set forth herein, or a sequence differing by one, two, or three nucleotides from any one of the sequences (a) to (e):

(a) The double-stranded siRNA designated BBD-2051, comprising a sense strand having the nucleotide sequence as set forth in SEQ ID NO:103 (UGGGAACUUACUACAACUCCA), and an antisense strand having the nucleotide sequence as set forth in SEQ ID NO:104 (UGGAGUUGUAGUAAGUUCCCAGG).

(b) The double-stranded siRNA designated BBD-2083, comprising a sense strand having the nucleotide sequence as set forth in SEQ ID NO:167 (UGCUACUCUGGUAUUUCCUAA), and an antisense strand having the nucleotide sequence as set forth in SEQ ID NO:168 (UUAGGAAAUACCAGAGUAGCACC).

(c) The double-stranded siRNA designated BBD-2047, comprising a sense strand having the nucleotide sequence as set forth in SEQ ID NO:95 (CGCCUGGGAACUUACUACAAU), and an antisense strand having the nucleotide sequence as set forth in SEQ ID NO:96 (AUUGUAGUAAGUUCCCAGGCGGG).

(d) The double-stranded siRNA designated BBD-2086, comprising a sense strand having the nucleotide sequence as set forth in SEQ ID NO:173 (GGGUGCUACUCUGGUAUUUCA), and an antisense strand having the nucleotide sequence as set forth in SEQ ID NO:174 (UGAAAUACCAGAGUAGCACCCCC).

(e) The double-stranded siRNA designated BBD-2087, comprising a sense strand having the nucleotide sequence as set forth in SEQ ID NO:175 (GGGGUGCUACUCUGGUAUUUA), and an antisense strand having the nucleotide sequence as set forth in SEQ ID NO:176 (UAAAUACCAGAGUAGCACCCCCG).

In certain embodiments, the sense strand comprises no more than 3, 2, 1, or 0 unmodified nucleotides, and the modified nucleotides in the sense strand are selected from 2'-O-methyl-modified nucleotides, 2'-deoxynucleotides, and 2'-fluoro-modified nucleotides. The sense strand comprises 1 to 3 phosphorothioate linkages at the 5'-terminus.

In certain embodiments, the antisense strand comprises no more than 3, 2, 1, or 0 unmodified nucleotides. The modified nucleotides in the antisense strand are selected from 2'-O-methyl-modified nucleotides, 2'-deoxynucleotides, 2'-fluoro-modified nucleotides, 5'- (E)-vinylphosphonate nucleotides, and isomer of glycerol nucleic acid(isoGNA). The antisense strand comprises 1 to 3 phosphorothioate linkages at both the 5'-terminus and the 3'-terminus. The double-stranded RNAi agent comprises at least one strand with a 3'-overhang of 1 to 3 nucleotides.

In some preferred embodiments, the isomer of glycerol nucleic acid(isoGNA) nucleotide is located in at least one position between position 4 and 8 counted from the 5'-terminus of the antisense strand, so as to reduce off-target activity of an siRNA or a pharmaceutically acceptable salt thereof and enhance safety.

In certain embodiments, antisense strand of the complementary siRNA at positions 11 to 13 of the 5'- terminus and/ or the sense strand of the complementary siRNA at positions 9 to 11 of the 5'-terminus together comprise a total of 1 to 6 2'-deoxynucleotides..

Wherein,the isomer of glycerol nucleic acid(isoGNA) nucleotide is selected from at least one of the following structures:

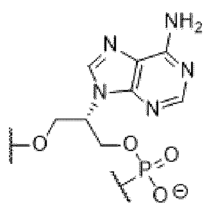
isoGNA-A

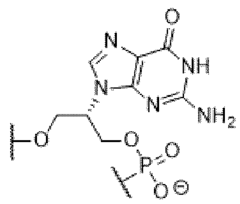
isoGNA-G

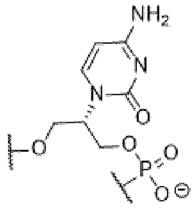
isoGNA-C

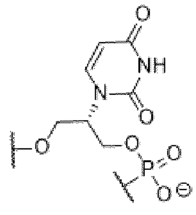
isoGNA-U

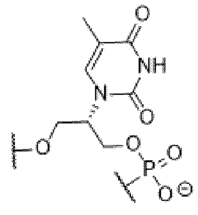
isoGNA-T .

Wherein, the 2'-deoxy modified nucleotides is selected from at least one of the following structures:

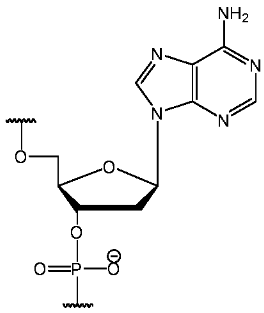
dA

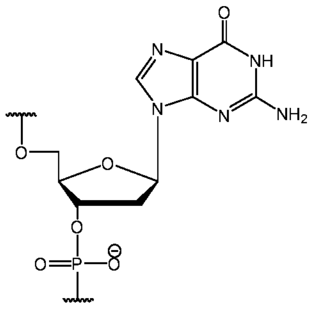
dG

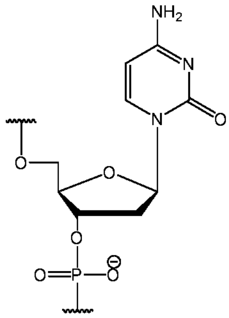
dC

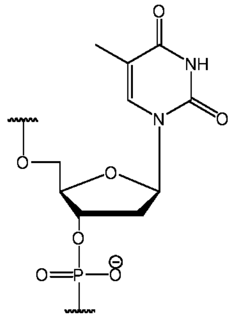
dT .

Wherein, the structure of the uridine-5'-(E)-vinylphosphonate nucleotide is as follows:

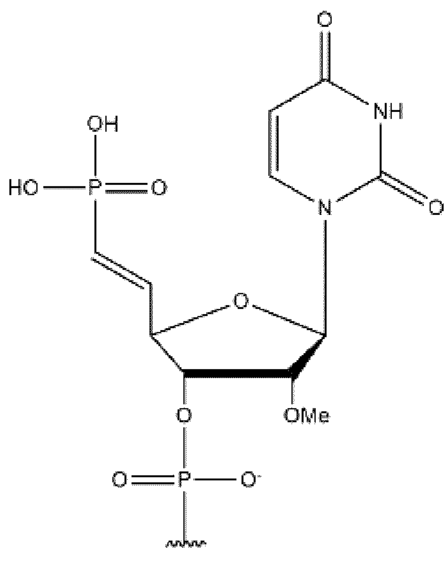
VPU

In some preferred embodiments, the modified duplex ID is selected from the group consisting BBD-2051.210, BBD-2051.211, BBD-2051.28, BBD-2083.313, BBD-2083.413, BBD-2051.25, BBD-2051.26, BBD-2051.27, BBD-2051.28, BBD-2051.29, BBD-2051.213, BBD-2051.214, BBD-2051.215, BBD-2051.217, BBD-2051.218, and BBD-2051.219; BBD-2083.41, BBD-2083.410, BBD-2083.411, BBD-2083.412, BBD-2083.414, BBD-2083.415, BBD-2083.416, BBD-2083.418, BBD-2083.419, BBD-2083.420, BBD-2083.422, BBD-2083.423, and BBD-2083.424.

In some more preferred embodiments, the modified siRNA is selected from the group consisting:

BBD-2051.210 :

sense strand : mU*mG*mGmGmAmAfCmUfUfAfCmUmAmCmAmAmCmUmCmCmA ,

Antisense strand : VPU*fG*mGmAmGisoGNA-TmUmGmUfAmGdTmAfAmGfUmUmCmCmCmA*mG*mG,

BBD-2051.211 :

sense strand : mU*mG*mGmGmAmAfCmUfUfAfCmUmAmCmAmAmCmUmCmCmA,

Antisense strand : VPU*fG*mGmAmGmUisoGNA-TmGmUfAmGdTmAfAmGfUmUmCmCmCmA*mG*mG,

BBD-2051.28 :

sense strand : mU*mG*mGmGmAmAfCmUfUfAfCmUmAmCmAmAmCmUmCmCmA,

Antisense strand : VPU*fG*mGmAmGmUmUmGmUfAmGdTmAfAmGfUmUmCmCmCmA*mG*mG,

BBD-2083.313 :

sense strand : mU*mG*mCmUmAmCfUmCfUfGfGmUmAmUmUmUmCmCmUmAmA,

Antisense strand : VPU*fU*mAmGmGmAmAmAmUfAmCdCmAfGmAfGmUmAmGmCmA*mC*mC,

BBD-2083.413 :

sense strand : mU*mG*mCmUmAmCfUmCfUfGdGmUmAmUmUmUmCmCmUmAmA,
Antisense strand : VPU*fU*mAmGmGmAmAmAmUfAmCdCmAfGmAfGmUmAmGmCmA*mC*mC ;

The second aspect of the present invention provides a pharmaceutical composition for inhibiting TMPRSS6 expression, comprising any one of the siRNAs or a pharmaceutically acceptable salt thereof as described above, and a targeting delivery ligand or delivery vehicle conjugated to or encapsulating the siRNA.
In certain embodiments, the ligand is an N-acetylgalactosamine (GalNAc) derivative.
In certain embodiments, the structure of the ligand is as follows:

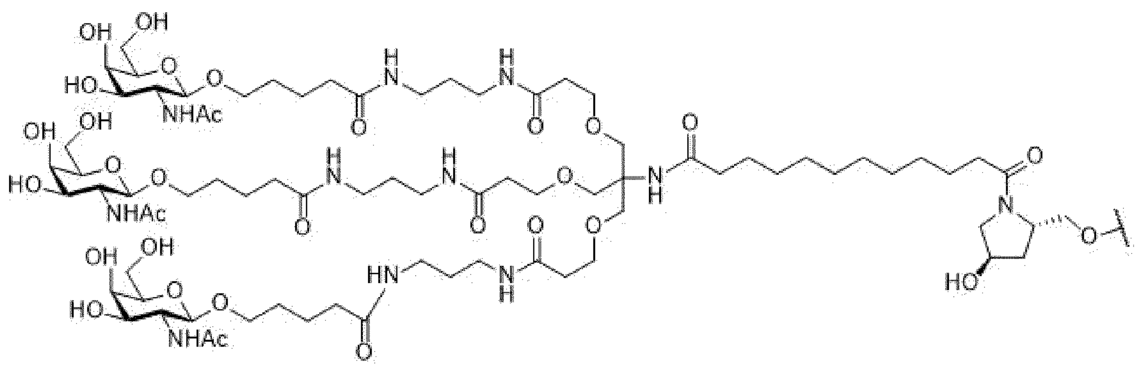

or

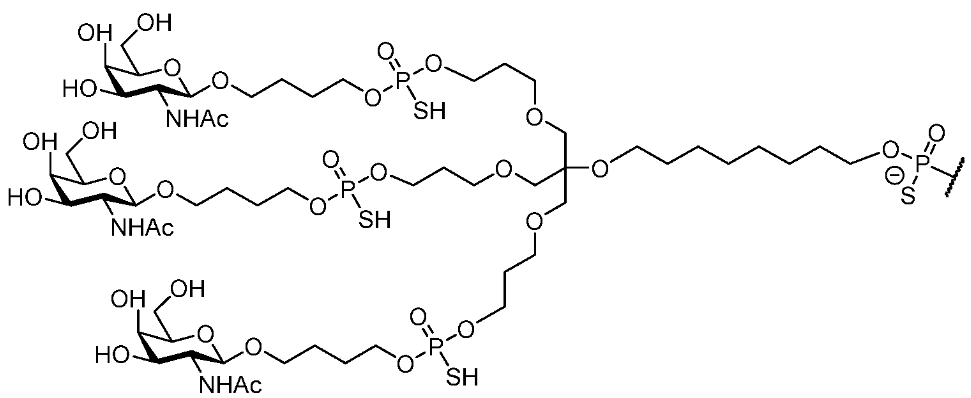

or

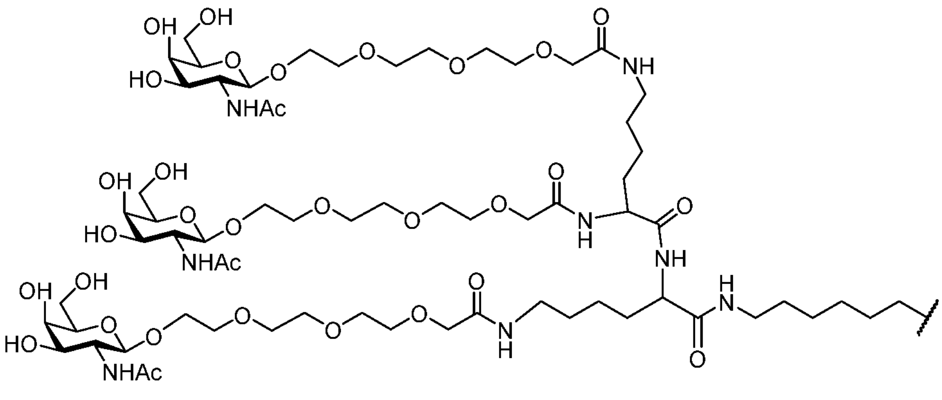

or

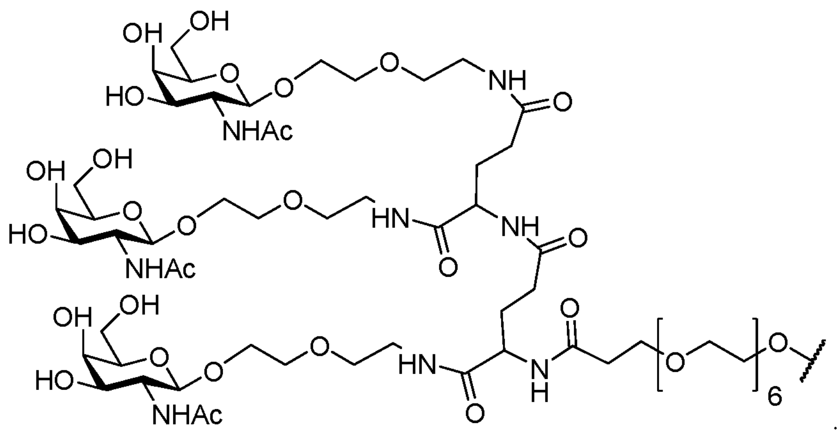

In certain embodiments, the ligand is conjugated to the 3'-terminus of the sense strand of the siRNA.

In certain embodiments, the ligand is conjugated to the 3'-terminus of the sense strand of the siRNA, as illustrated in the following schematic:

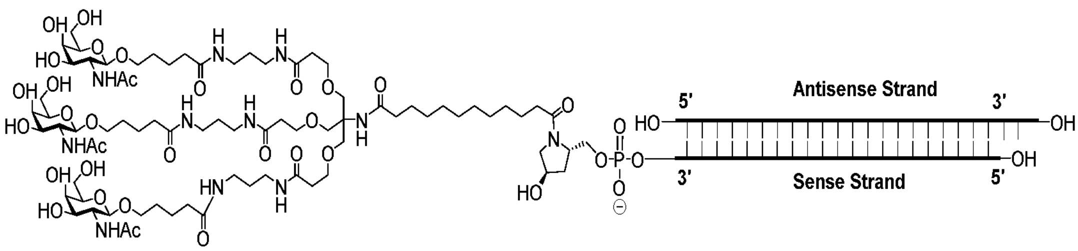


or

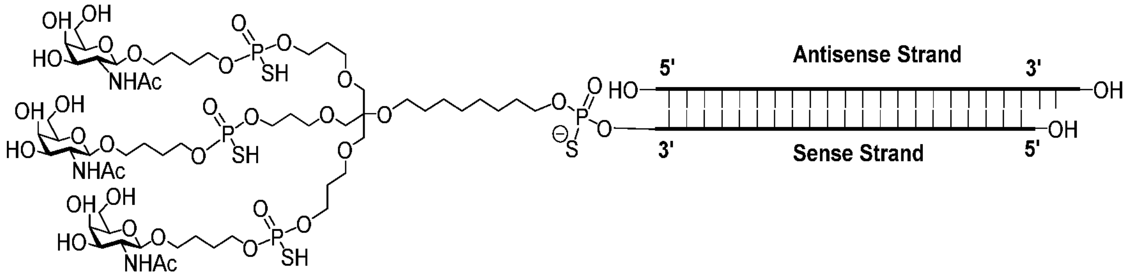


or

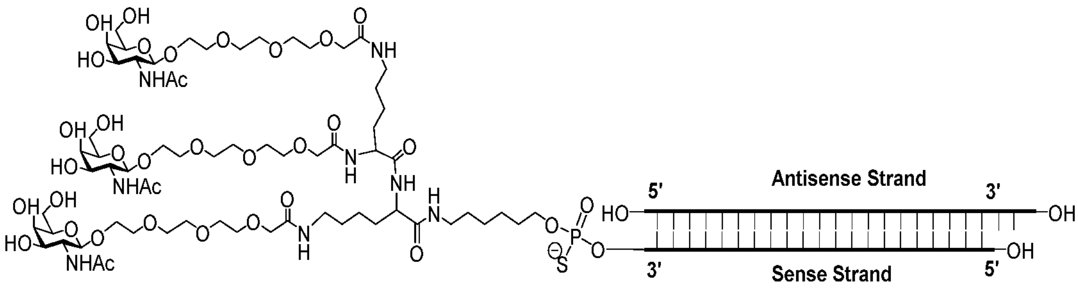

or

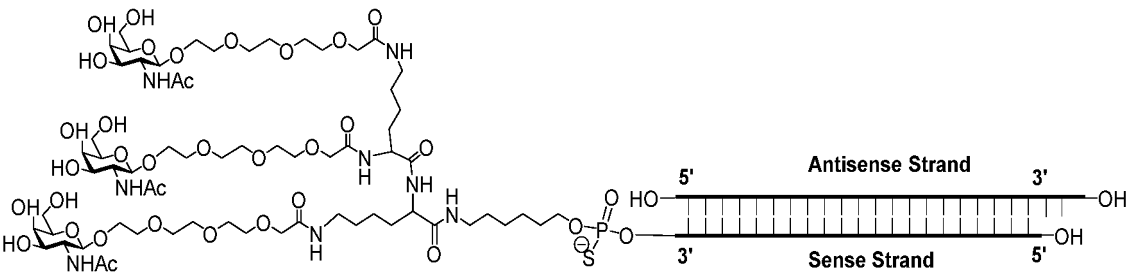

-6-

The third aspect of the present invention provides the use of the above-mentioned siRNA or the pharmaceutically acceptable salt thereof in the manufacture of a biological agent or pharmaceutical composition for inhibiting TMPRSS6 expression.

The fourth aspect of the present invention provides the use of the biological agent or the pharmaceutical composition in the manufacture of a medicament for preventing and treating a disorder associated with TMPRSS6 or a disorder associated with iron excess or iron overload.

The fifth aspect of the present invention provides a method of inhibiting TMPRSS6 expression in a cell, the method comprising:

(a) contacting the cell with any one of the foregoing siRNA or a pharmaceutically acceptable salt thereof or any of the foregoing biological agents or pharmaceutical compositions; and (b) maintaining the cells produced in step (a) for a time sufficient to obtain degradation of the mRNA transcript of a TMPRSS6 gene, thereby inhibiting expression of the TMPRSS6 gene in the cell.

The sixth aspect of the present invention provides a method of treating a subject suffering from a TMPRSS6-related disorder or a disease associated with iron excess or iron overload, comprising administering to the subject a therapeutically effective amount of any one of the foregoing siRNAs or pharmaceutically acceptable salts thereof, or any one of the foregoing biological agents or pharmaceutical compositions, thereby treating the subject.

In certain embodiments, the cell is within the subject.

In certain embodiments, the subject is a human.

In certain embodiments, the TMPRSS6 expression is inhibited by at least about 30%, about 40%, about 50%, about 60%, about 70%, about 80%, about 90%, about 95%, about 98%, or about 100%.

In certain embodiments, the serum hepcidin concentration of the subject is increased by at least about 10%; and/or the serum iron concentration of the subject is decreased by at least about 20%; and/or a percent transferrin saturation of the subject is decreased by at least about 20%.

In certain embodiments, a disorder associated with TMPRSS6 or a disease associated with iron excess or iron overload is selected from polycythemia, thalassemia, hemochromatosis, myelodysplastic syndrome, porphyria cutanea tarda, aplastic anemia, sideroblastic anemia, iron-refractory iron deficiency anemia, hereditary anemia, severe chronic hemolytic anemia, Parkinson's disease, Alzheimer's disease, Friedreich's ataxia, or microcytic anemia, transfusion-induced iron overload, and iron overload associated with non-alcoholic fatty liver disease (NAFLD).

In certain embodiments, the thalassemia is selected from α-thalassemia, β-thalassemia, and δ-thalassemia.

In certain embodiments, the polycythemia is polycythemia vera.

In certain embodiments, the hereditary anemia is selected from sickle-cell anemia, thalassemia, Fanconi anemia, Diamond-Blackfan anemia, Shwachman-Diamond syndrome, red blood cell membrane disorders, glucose-6-phosphate dehydrogenase deficiency, and hereditary hemorrhagic telangiectasia.

The inventors of the present application have conducted research on siRNA sequences and, through extensive experiments, identified multiple siRNA sequences capable of inhibiting TMPRSS6 expression. Based on these findings, appropriate modifications were further made to enhance target silencing efficiency and reduce off-target activity. Through a series of in vitro and in vivo experiments, siRNA sequences with robust biological activity in inhibiting TMPRSS6 expression were obtained, demonstrating superior activity compared to the most advanced clinical candidate, TMPRSS6-HCM-9. These siRNA sequences are expected to be applied in the clinical treatment of TMPRSS6-related disorders or diseases associated with iron overload or iron excess.

## BRIEF DESCRIPTION OF THE DRAWINGS

Figure 1 illustrates the evaluation of the effects of different modified BBD-2047 sequences, BBD-2051 sequences, BBD-2083 sequences, and TMPRSS6-HCM-9 sequence on hepatic human TMPRSS6 expression in mice expressing human TMPRSS6 (1 mg/kg).
Figure 2 illustrates the evaluation of the effects of different modified BBD - 2083 sequences and TMPRSS6 - HCM - 9 sequence on hepatic hTMPRSS6 expression in mice expressing human TMPRSS6 (3 mg/kg).
Figure 3 illustrates the evaluation of the effects of different modified BBD - 2051 sequences and TMPRSS6 - HCM - 9 sequence on hepatic hTMPRSS6 expression in mice expressing human TMPRSS6 (3 mg/kg).
Figure 4 illustrates the evaluation of the effects of different modified BBD - 2051 sequences on hepatic mTMPRSS6 expression in wild - type mice (10 mg/kg).
Figure 5 illustrates the evaluation of the effects of different modified BBD-2051 sequences on serum iron concentration in wild-type mice (10 mg/kg).
Figure 6 illustrates the evaluation of the off-target activity of different modified BBD-2051 sequences.
Figure 7 illustrates the evaluation of the off-target activity of different modified BBD-2083 sequences and AD-1554911.

## DESCRIPTION OF THE EMBODIMENTS

To facilitate understanding of the present invention, a more detailed description is provided below. The invention can be implemented in numerous forms and is not limited to the embodiments described herein. Rather, these embodiments are provided to enable a thorough and comprehensive understanding of the disclosed content of the invention.

In the following embodiments, experimental methods, unless otherwise specified, are typically conducted under conventional conditions, such as those described in Sambrook et al., Molecular Cloning: A Laboratory Manual (New York: Cold Spring Harbor Laboratory Press, 1989), or according to the conditions recommended by the manufacturer. Various commonly used chemical reagents employed in the embodiments are commercially available products.

Unless otherwise defined, all technical and scientific terms used herein have the same meanings as commonly understood by one of ordinary skill in the art to which the present invention belongs. The terminology used in the specification is provided solely for the purpose of describing specific embodiments only and is not intended to limit the invention. The term 'and/or' as used in the present invention encompasses any and all combinations of the listed items.

In order that the present invention may be more readily understood, certain terms are defined.

Iron overload refers to the excessive accumulation of iron in the body, resulting in structural damage and functional impairment of vital organs, particularly the heart, liver, pituitary gland, pancreas, and joints.

Polycythemia refers to an elevated erythrocyte count and hemoglobin concentration in a given volume of blood, exceeding the upper limit of the reference range.

Hemochromatosis is a disorder of iron metabolism characterized by excessive iron accumulation in the body, resulting from a high-iron diet, frequent blood transfusions, or systemic diseases.

Thalassemia, also known as "marine anemia", is officially termed " globin synthesis disorder anemia" as designated by China National Committee for Terminology in Sciences and Technologies. This disease is characterized by the impairment or complete inhibition of one or more globin chain syntheses, leading to abnormal hemoglobin (Hb) composition and subsequent chronic hemolytic anemia.

The expression of the TMPRSS6 gene can be assessed based on the level of any variable associated with TMPRSS6 gene expression, e.g., TMPRSS6 mRNA level in tissues or serum, TMPRSS6 protein level, hepcidin mRNA level, hepcidin protein level, transferrin saturation level, or iron level. Inhibition can be assessed by a decrease in an absolute or relative level of one or more of these variables compared with a control level. The control level may be any type of control level that used in the art, e.g., pre-dose baseline level, or a level measured from a similar subject, cell, or sample that is untreated or treated with a control.

In certain embodiments, the diseases related to TMPRSS6 expression or associated with iron excess or iron

overload are selected from polycythemia, thalassemia, hemochromatosis, myelodysplastic syndrome, porphyria cutanea tarda, aplastic anemia, sideroblastic anemia, iron-refractory iron deficiency anemia, hereditary anemia, severe chronic hemolysis, Parkinson's disease, Alzheimer's disease, Friedreich's ataxia, or microcytic anemia, transfusion-dependent iron overload, and NAFLD-associated iron overload.

The siRNA agent of the present invention can be delivered to cells, such as those within a subject (e.g., a human subject, such as one suffering from a TMPRSS6-related disorder such as hemochromatosis), via various routes. For instance, delivery can be accomplished by bringing the cells into contact with the siRNA of the present invention either *ex vivo or in vivo. In vivo* delivery can also be achieved by administering to the subject a composition comprising the siRNA or its salt.

Generally, any method of delivering a nucleic acid molecule (either *ex vivo* or *in vivo*) can be adapted for use with existing delivery technologies. For *in vivo* delivery, factors to consider in order to deliver an siRNA molecule include, for example, biological stability of the delivered molecule, prevention of non-specific effects, and accumulation of the delivered molecule in the target tissue. The non-specific effects of an iRNA can be minimized by local administration, for example, by direct injection or implantation into a tissue or topically administering the preparation. Local administration to a treatment site maximizes local concentration of the agent, limits the exposure of the agent to systemic tissues that can otherwise be harmed by the agent or that can degrade the agent, and permits a lower total dose of the iRNA molecule to be administered..

For systemic iRNA administration to treat diseases, the RNA can be modified or delivered via a drug delivery system; both methods act to prevent the rapid degradation of dsRNA by endonucleases and exonucleases in vivo. Modifications to RNA or pharmaceutical carriers can also enable the siRNA composition to target the target specific tissues and avoid off-target effects. siRNA molecules can be chemically conjugated to lipophilic moieties such as cholesterol, for example, lipid particles, to enhance cellular uptake and prevent degradation.

The present invention also includes pharmaceutical compositions and formulations

which include the iRNAs of the invention. In one embodiment, provided herein are pharmaceutical compositions containing an iRNA, as described herein, and a pharmaceutically acceptable carrier. The pharmaceutical compositions containing the iRNA are useful for treating a TMPRSS6 associated disease or disorder, e.g. hemochromatosis. Such pharmaceutical compositions are formulated based on the mode of delivery.

In certain embodiments, the compositions are formulated for systemic administration via parenteral routes, e.g.,intravenous(IV) delivery or subcutaneous (SC) delivery. In the methods of the present invention, the siRNA or its salt pharmaceutical compositions can be administered in a solution, preferably in a sterile solution, e.g., by injection.

In the present invention, the term "Therapeutically effective amount," as used herein, is intended to include the amount of an RNAi agent that, when administered to a patient for treating a TMPRSS6 associated disease, is sufficient to effect treatment of the disease (e.g., by diminishing, ameliorating or maintaining the existing disease or one or more symptoms of disease). The "therapeutically effective amount" may vary depending on the RNAi agent, how the agent is administered, the disease and its severity and the history, age, weight, family history, genetic makeup, stage of pathological processes mediated by TMPRSS6 expression, the types of preceding or concomitant treatments, if any, and other individual characteristics of the patient to be treated.

In the present invention, the term "Prophylactically effective amount," as used herein, is intended to include the amount of an RNAi agent that, when administered to a subject who does not yet experience or display symptoms of a TMPRSS6-associated disease, but who may be predisposed to the disease, is sufficient to prevent or ameliorate the disease or one or more symptoms of the disease. Ameliorating the disease includes slowing the course of the disease or reducing the severity of later-developing disease. The "prophylactically effective amount" may vary depending on the siRNA agent, how the agent is administered, the degree of risk of disease,
and the history, age, weight, family history, genetic makeup, the types of preceding or concomitant treatments, if any, and other individual characteristics of the patient to be treated.

The "therapeutically effective amount" or "prophylactically effective amount" also refer to an amount of an siRNA that produces some desired local or systemic effect at a reasonable benefit/risk ratio applicable to any treatment.

The targeted delivery ligand or other types of delivery vehicles of the present invention, wherein the ligand is preferably being an N-acetylgalactosamine derivative (GalNAc carrier), or other types of delivery vehicles, such as liposomes specifically targeting hepatocytes, may also be used in the present invention, provided that they are capable of delivering siRNA.

In recent years, the GalNAc delivery vehicle has been extensively studied. GalNAc-nucleic acid are conjugates of carbohydrate compounds and nucleic acids, where N-acetylgalactosamine is covalently attached in a trivalent manner to the 3'- and/or 5'- ends of RNA sense strands of different sequences, forming GalNAc-siRNA drugs enabling specific delivery to liver cells, and allowing cellular uptake via endocytosis to deliver the drug and exert its function.

In the following embodiments, the structure of the GalNAc delivery vehicle and its conjugation with siRNA are

illustrated in the following schematic:

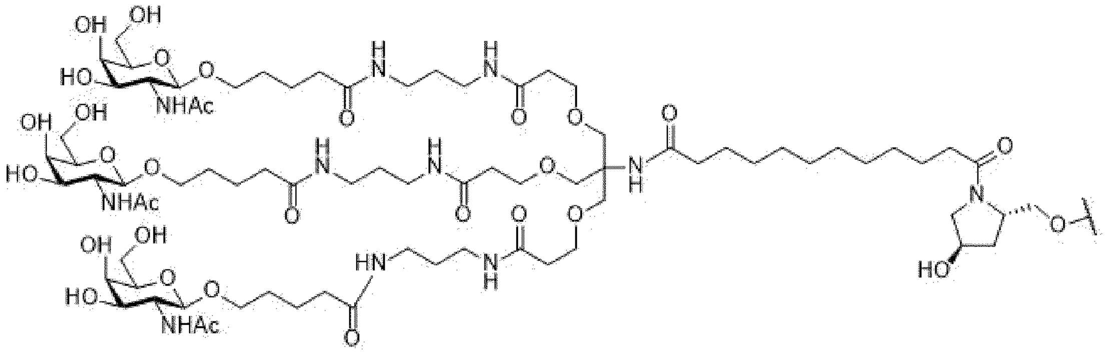

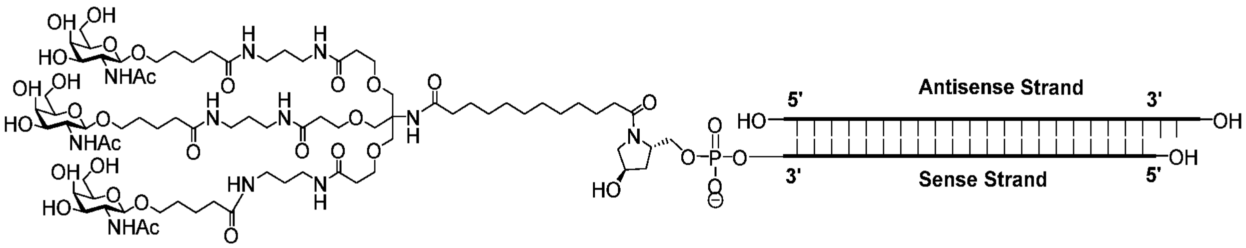


The abbreviations and structures of nucleotide monomers used in the representation of nucleic acid sequences are as follows:

| | |
|---|---|
| **A** | Adenosine-3'-phosphate |
| **T** | 5'-methyluridine-3'-phosphate |
| **C** | cytidine-3'-phosphate |
| **G** | guanosine-3'-phosphate |
| **U** | Uridine-3'-phosphate |
| **dA** | 2'-Deoxyadenosine-3'-phosphate |
| **dT** | 2'-deoxythymidine-3'-phosphate |
| **dC** | 2'-Deoxycytidine-3'-phosphate |
| **dG** | 2'-Deoxyguanosine-3'-phosphate |
| **dU** | 2'-Deoxyuridine-3'-phosphate |
| **mA** | 2'-O-Methyladenosine-3'-phosphate |
| **mC** | 2'-O-Methylcytidine-3'-phosphate |
| **mG** | 2'-O-Methylguanosine-3'-phosphate |
| **mU** | 2'-O-Methyluridine-3'-phosphate |
| **fA** | 2'-Fluoroadenosine-3'-phosphate |
| **fC** | 2'-Fluorocytidine-3'-phosphate |
| **fG** | 2'-Fluoroguanosine-3'-phosphate |
| **fU** | 2'-Fluorouridine-3'-phosphate |
| **VPU** | Uridine-5'-(E)-vinylphosphonate nucleotide |
| **isoGNA-A** | Adenosine-isoglycerol nucleotide |
| **isoGNA-T** | Thymine-isoglycerol nucleotide |
| **isoGNA-C** | Cytosine-isoglycerol nucleotide |

(continued)

| | |
|---|---|
| **isoGNA-G** | Guanosine-isoglycerol nucleotide |
| **isoGNA-U** | Uridine-isoglycerol nucleotide |
| * | Phosphorothioate linkage |

The specific structure is as follows:

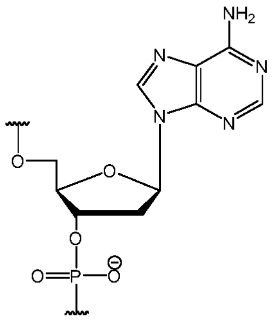
dA

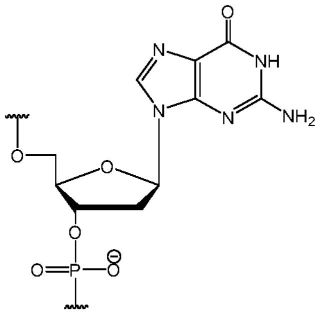
dG

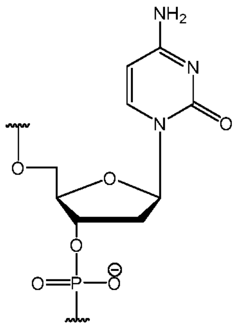
dC

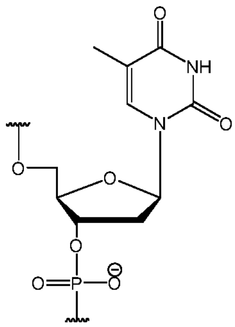
dT

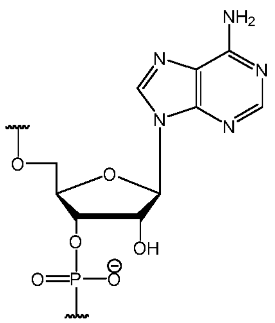
A

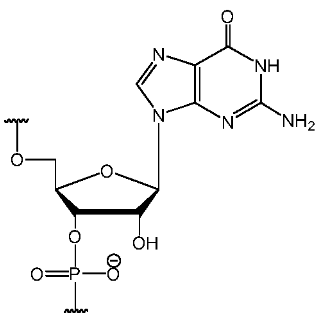
G

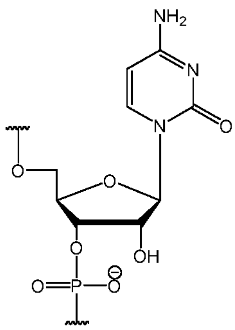
C

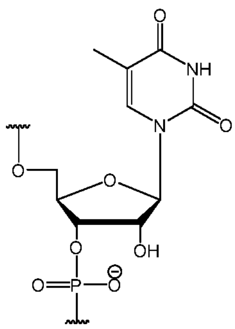
T

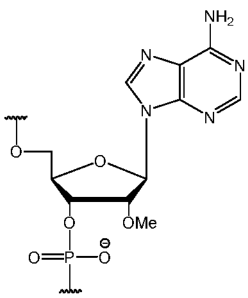
mA

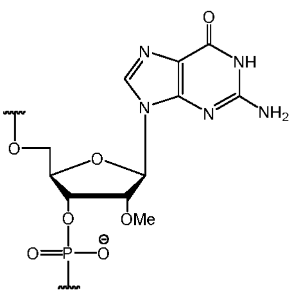
mG

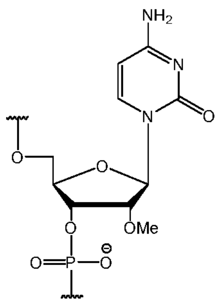
mC

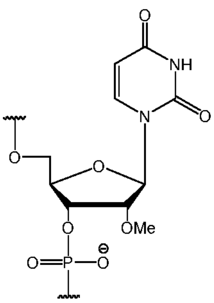
mU

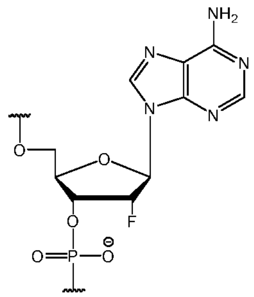
fA

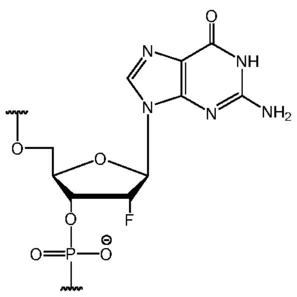
fG

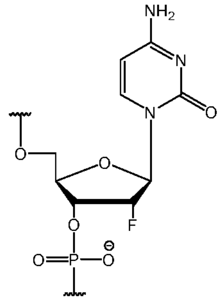
fC

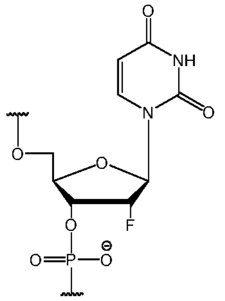
fU

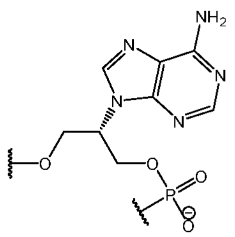
isoGNA-A

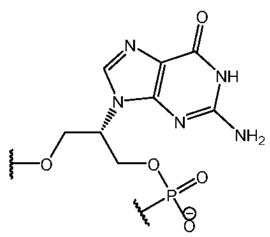
isoGNA-G

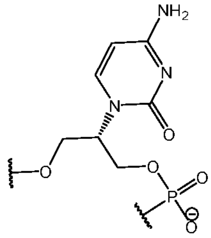
isoGNA-C

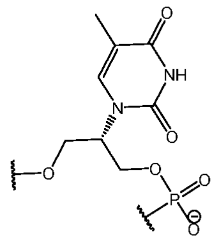
isoGNA-T

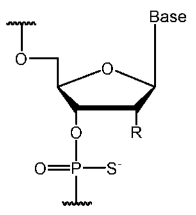
R=H, OH, F, OMe
* = Phosphorothioate modifications

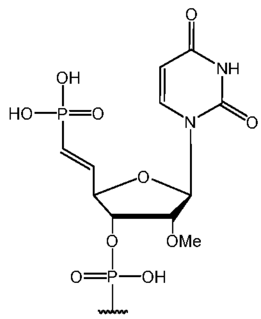
VPU(OMe)

Those skilled in the art will appreciate that the pharmaceutically acceptable salts of the siRNA may include sodium salts or potassium salts; for example, a sodium salt of the siRNA may be generated during the purification process.

The present invention is further described in detail by reference to the following specific embodiments.

### Example 1: Synthesis of siRNA

0.2-1μmol of oligonucleotides were synthesized using the solid-phase oligonucleotide synthesis protocol on a 12-channel nucleic acid synthesizer of Beijing Qingke Biotechnology Co., Ltd. For siRNA sequences containing GalNAc, synthesis was carried out CPG column preloaded with GalNAc. The synthesized oligonucleotides were treated with an ammonolysis reagent and incubated at 45-80°C to release them from the solid-phase support. The crude oligonucleotides were then precipitated with ethanol, centrifuged at high speed to remove the supernatant, and the precipitation process was repeated twice. The resulting precipitate was resuspended in DEPC-treated water. The crude oligonucleotides were purified using ion-pair HPLC, and the collected product was lyophilized in a vacuum centrifugal dryer until a powdered form was obtained. The purified product was dissolved in DEPC-treated water and analyzed using TOF LC-MS. The concentration of oligonucleotides was determined, and the volumes of equimolar sense and antisense strands were calculated accordingly. Equimolar amounts of sense and antisense strands were mixed thoroughly and annealed by heating at 95°C for 5 minutes and then cooling to room temperature naturaly to form duplex siRNA.

Table 1: Sense and antisense sequences of unmodified siRNA. TMPRSS6-HCM-9 is a clinical-stage sequence developed by Silence Therapeutics (Publication No.: CN113164768A) and serves as a positive control sequence.

| Duplex ID | Sense strand sequenceID | Sense strand sequence 5'-3' | Range within NM_1536 09.4 | Antisense strand Sequence ID | Antisense strand sequence 5'-3' | Range within NM_153 609.4 |
|---|---|---|---|---|---|---|
| TMPRSS 6-hem-9 | S0 (1) | UCACCUGCUUC UUCUGGUU | 560_578 | AS0 (2) | AACCAGAAGA AGCAGGUGA | 560_578 |
| BBD-2001 | S1 (3) | GCCAAAGCCCA GAAGAUGCUA | 463_483 | AS01 (4) | UAGCAUCUUC UGGGCUUUGG CGG | 461_ 483 |
| BBD-2002 | S2(5) | UCCUUUGGGGA GGGACCCCUA | 541_561 | AS02(6) | UAGGGGUCCC UCCCCAAAGGA AU | 539_ 561 |
| BBD-2003 | S3(7) | GUGAUCCUGGA AGCCAGUGUA | 724_744 | AS03(8) | UACACUGGCU UCCAGGAUCAC UA | 722_744 |
| BBD-2004 | S4(9) | UGAUCCUGGAA GCCAGUGUGA | 725_745 | AS04(10) | UCACACUGGCU UCCAGGAUCAC C | 723_ 745 |
| BBD-2005 | S5(11) | GAUCCUGGAAG CCAGUGUGAA | 726_746 | AS05(12) | UUCACACUGGC UUCCAGGAUC AC | 724_746 |
| BBD-2006 | S6(13) | CCCUCUCUGGA CUACGGCUUA | 1234_124 4 | AS06(14) | UAAGCCGUAG UCCAGAGAGG GCA | 1232_12 44 |
| BBD-2007 | S7(15) | GAUCCAGAACA GGAGGCUGUA | 1326_134 6 | AS07(16) | UACAGCCUCCU GUUCUGGAUC GU | 1324_13 46 |
| BBD-2008 | S8(17) | AUCCAGAACAG GAGGCUGUGU | 1327_134 7 | AS08(18) | ACACAGCCUCC UGUUCUGGAU CG | 1325_13 47 |
| BBD-2009 | S9(19) | UCCAGAACAGG AGGCUGUGUA | 1328_134 8 | AS09(20) | UACACAGCCUC CUGUUCUGGA UC | 1326_13 48 |
| BBD-2010 | S10(21) | CCAGAACAGGA GGCUGUGUGA | 1329_134 9 | AS10(22) | UCACACAGCCU CCUGUUCUGG AU | 1327_13 49 |

(continued)

| Duplex ID | Sense strand sequenceID | Sense strand sequence 5'-3' | Range within NM_1536 09.4 | Antisense strand Sequence ID | Antisense strand sequence 5'-3' | Range within NM_153 609.4 |
|---|---|---|---|---|---|---|
| BBD-2011 | S11(23) | CAGAACAGGAG GCUGUGUGGA | 1330_135 0 | AS11(24) | UCCACACAGCC UCCUGUUCUG GA | 1328_13 50 |
| BBD-2012 | S12(25) | GAACAGGAGGC UGUGUGGCUU | 1332_135 2 | AS12(26) | AAGCCACACAG CCUCCUGUUCU G | 1330_13 52 |
| BBD-2013 | S13(27) | UCACCAUCAAC UUCACCUCCA | 1406_142 6 | AS13(28) | UGGAGGUGAA GUUGAUGGUG AUC | 1404_14 26 |
| BBD-2014 | S14(29) | CACCAUCAACU UCACCUCCCA | 1407_142 7 | AS14(30) | UGGGAGGUGA AGUUGAUGGU GAU | 1405_14 27 |
| BBD-2015 | S15(31) | ACCAUCAACUU CACCUCCCAA | 1408_1428 | AS15(32) | UUGGGAGGUG AAGUUGAUGG UGA | 1406_1428 |
| BBD-2016 | S16(33) | CCAUCAACUUC ACCUCCCAGA | 1409_142 9 | AS16(34) | UCUGGGAGGU GAAGUUGAUG GUG | 1407_14 29 |
| BBD-2017 | S17(35) | CAUCAACUUCA CCUCCCAGAU | 1410_143 0 | AS17(36) | AUCUGGGAGG UGAAGUUGAU GGU | 1408_14 30 |
| BBD-2018 | S18(37) | AUCAACUUCAC CUCCCAGAUA | 1411_143 1 | AS18(38) | UAUCUGGGAG GUGAAGUUGA UGG | 1409_14 31 |
| BBD-2019 | S19(39) | UCAACUUCACC UCCCAGAUCU | 1412_143 2 | AS19(40) | AGAUCUGGGA GGUGAAGUUG AUG | 1410_14 32 |
| BBD-2020 | S20(41) | CAACUUCACCU CCCAGAUCUA | 1413_143 3 | AS20(42) | UAGAUCUGGG AGGUGAAGUU GAU | 1411_14 33 |
| BBD-2021 | S21(43) | AACUUCACCUC CCAGAUCUCA | 1414_143 4 | AS21(44) | UGAGAUCUGG GAGGUGAAGU UGA | 1412_14 34 |

(continued)

| Duplex ID | Sense strand sequenceID | Sense strand sequence 5'-3' | Range within NM_1536 09.4 | Antisense strand Sequence ID | Antisense strand sequence 5'-3' | Range within NM_153 609.4 |
|---|---|---|---|---|---|---|
| BBD-2022 | S22(45) | ACUUCACCUCC CAGAUCUCCA | 1415_143 5 | AS22(46) | UGGAGAUCUG GGAGGUGAAG UUG | 1413_14 35 |
| BBD-2023 | S23(47) | CUUCACCUCCC AGAUCUCCCU | 1416_143 6 | AS23(48) | AGGGAGAUCU GGGAGGUGAA GUU | 1414_14 36 |
| BBD-2024 | S24(49) | UUCACCUCCCA GAUCUCCCUA | 1417_143 7 | AS24(50) | UAGGGAGAUC UGGGAGGUGA AGU | 1415_14 37 |
| BBD-2025 | S25(51) | UCACCUCCCAG AUCUCCCUCA | 1418_143 8 | AS25(52) | UGAGGGAGAU CUGGGAGGUG AAG | 1416_14 38 |
| BBD-2026 | S26(53) | CACCUCCCAGA UCUCCCUCAA | 1419_143 9 | AS26(54) | UUGAGGGAGA UCUGGGAGGU GAA | 1417_14 39 |
| BBD-2027 | S27(55) | CCAGUGUGAGG ACCGGAGCUA | 1725_174 5 | AS27(56) | UAGCUCCGGUC CUCACACUGGA A | 1723_17 45 |
| BBD-2028 | S28(57) | CACUGUGACUG UGGCCUCCAA | 1810_183 0 | AS28(58) | UUGGAGGCCA CAGUCACAGU GCU | 1808_18 30 |
| BBD-2029 | S29(59) | UUCGGGGUCGA CACAUCUGUA | 1904_192 4 | AS29(60) | UACAGAUGUG UCGACCCCGAA CC | 1902_19 24 |
| BBD-2030 | S30(61) | UCGGGGUCGAC ACAUCUGUGA | 1905_192 5 | AS30(62) | UCACAGAUGU GUCGACCCCGA AC | 1903_19 25 |
| BBD-2031 | S31(63) | CGGGGUCGACA CAUCUGUGGA | 1906_192 6 | AS31(64) | UCCACAGAUG UGUCGACCCCG AA | 1904_19 26 |
| BBD-2032 | S32(65) | CCAGGAGGACA GCAUGGCCUA | 1974-1994 | AS32(66) | UAGGCCAUGC UGUCCUCCUGG AA | 1972-1994 |

(continued)

| Duplex ID | Sense strand sequenceID | Sense strand sequence 5'-3' | Range within NM_1536 09.4 | Antisense strand Sequence ID | Antisense strand sequence 5'-3' | Range within NM_153 609.4 |
|---|---|---|---|---|---|---|
| BBD-2033 | S33(67) | CUUCCAGGAGG ACAGCAUGGA | 1971-1991 | AS33(68) | UCCAUGCUGUC CUCCUGGAAGC A | 1969-1991 |
| BBD-2034 | S34(69) | GCUUCCAGGAG GACAGCAUGA | 1970-1990 | AS34(70) | UCAUGCUGUCC UCCUGGAAGC AG | 1968-1990 |
| BBD-2035 | S35(71) | UGCUUCCAGGA GGACAGCAUA | 1969-1989 | AS35(72) | UAUGCUGUCC UCCUGGAAGC AGU | 1967-1989 |
| BBD-2036 | S36(73) | CUGCUUCCAGG AGGACAGCAU | 1968-1988 | AS36(74) | AUGCUGUCCUC CUGGAAGCAG UG | 1966-1988 |
| BBD-2037 | S37(75) | GUGUCCUUCAA GGUGAGCCGA | 2056-2076 | AS37(76) | UCGGCUCACCU UGAAGGACAC CU | 2054-2076 |
| BBD-2038 | S38(77) | GGUGUCCUUCA AGGUGAGCCA | 2055-2075 | AS38(78) | UGGCUCACCUU GAAGGACACC UC | 2053-2075 |
| BBD-2039 | S39(79) | AGGUGUCCUUC AAGGUGAGCA | 2054-2074 | AS39(80) | UGCUCACCUUG AAGGACACCUC U | 2052-2074 |
| BBD-2040 | S40(81) | AGCCAUGACUA CGACGUGGCA | 2107-2127 | AS40(82) | UGCCACGUCGU AGUCAUGGCU GU | 2105-2127 |
| BBD-2041 | S41(83) | CAGCCAUGACU ACGACGUGGA | 2106-2126 | AS41(84) | UCCACGUCGUA GUCAUGGCUG UC | 2104-2126 |
| BBD-2042 | S42(85) | ACAGCCAUGAC UACGACGUGA | 2105-2125 | AS42(86) | UCACGUCGUA GUCAUGGCUG UCC | 2103-2125 |
| BBD-2043 | S43(87) | GACAGCCAUGA CUACGACGUA | 2104-2124 | AS43(88) | UACGUCGUAG UCAUGGCUGU CCU | 2102-2124 |

(continued)

| Duplex ID | Sense strand sequenceID | Sense strand sequence 5'-3' | Range within NM_1536 09.4 | Antisense strand Sequence ID | Antisense strand sequence 5'-3' | Range within NM_153 609.4 |
|---|---|---|---|---|---|---|
| BBD-2044 | S44(89) | GGACAGCCAUG ACUACGACGU | 2103-2123 | AS44(90) | ACGUCGUAGU CAUGGCUGUCC UC | 2101-2123 |
| BBD-2045 | S45(91) | AGGACAGCCAU GACUACGACA | 2102-2122 | AS45(92) | UGUCGUAGUC AUGGCUGUCC UCU | 2100-2122 |
| BBD-2046 | S46(93) | GAGGACAGCCA UGACUACGAA | 2101-2121 | AS46(94) | UUCGUAGUCA UGGCUGUCCUC UU | 2099-2121 |
| BBD-2047 | S47(95) | CGCCUGGGAAC UUACUACAAU | 505_525 | AS47(96) | AUUGUAGUAA GUUCCCAGGCG GG | 503_525 |
| BBD-2048 | S48(97) | GCCUGGGAACU UACUACAACU | 506_526 | AS48(98) | AGUUGUAGUA AGUUCCCAGGC GG | 504_526 |
| BBD-2049 | S49(99) | CCUGGGAACUU ACUACAACUA | 507_527 | AS49(100) | UAGUUGUAGU AAGUUCCCAG GCG | 505_527 |
| BBD-2050 | S50(101) | CUGGGAACUUA CUACAACUCA | 508_528 | AS50(102) | UGAGUUGUAG UAAGUUCCCA GGC | 506_528 |
| BBD-2051 | S51(103) | UGGGAACUUAC UACAACUCCA | 509_529 | AS51(104) | UGGAGUUGUA GUAAGUUCCC AGG | 507_529 |
| BBD-2052 | S52(105) | GGGAACUUACU ACAACUCCAA | 510_530 | AS52(106) | UUGGAGUUGU AGUAAGUUCC CAG | 508_530 |
| BBD-2053 | S53(107) | GGAACUUACUA CAACUCCAGA | 511_531 | AS53(108) | UCUGGAGUUG UAGUAAGUUC CCA | 509_531 |
| BBD-2054 | S54(109) | GAACUUACUAC AACUCCAGCU | 512_532 | AS54(110) | AGCUGGAGUU GUAGUAAGUU CCC | 510_532 |

(continued)

| Duplex ID | Sense strand sequenceID | Sense strand sequence 5'-3' | Range within NM_1536 09.4 | Antisense strand Sequence ID | Antisense strand sequence 5'-3' | Range within NM_153 609.4 |
|---|---|---|---|---|---|---|
| BBD-2055 | S55(111) | AACUUACUACA ACUCCAGCUA | 513_533 | AS55(112) | UAGCUGGAGU UGUAGUAAGU UCC | 511_533 |
| BBD-2056 | S56(113) | ACUUACUACAA CUCCAGCUCA | 514_534 | AS56(114) | UGAGCUGGAG UUGUAGUAAG UUC | 512_534 |
| BBD-2057 | S57(115) | CUUACUACAAC UCCAGCUCCA | 515_535 | AS57(116) | UGGAGCUGGA GUUGUAGUAA GUU | 513_535 |
| BBD-2058 | S58(117) | UUACUACAACU CCAGCUCCGU | 516_536 | AS58(118) | ACGGAGCUGG AGUUGUAGUA AGU | 514_536 |
| BBD-2059 | S59(119) | UACUACAACUC CAGCUCCGUA | 517_537 | AS59(120) | UACGGAGCUG GAGUUGUAGU AAG | 515_537 |
| BBD-2060 | S60(121) | ACUACAACUCC AGCUCCGUCU | 518_538 | AS60(122) | AGACGGAGCU GGAGUUGUAG UAA | 516_538 |
| BBD-2061 | S61(123) | CUACAACUCCA GCUCCGUCUA | 519_539 | AS61(124) | UAGACGGAGC UGGAGUUGUA GUA | 517_539 |
| BBD-2062 | S62(125) | UACAACUCCAG CUCCGUCUAU | 520_540 | AS62(126) | AUAGACGGAG CUGGAGUUGU AGU | 518_540 |
| BBD-2063 | S63(127) | ACAACUCCAGC UCCGUCUAUU | 521_541 | AS63(128) | AAUAGACGGA GCUGGAGUUG UAG | 519_541 |
| BBD-2064 | S64(129) | CAACUCCAGCU CCGUCUAUUA | 522_542 | AS64(130) | UAAUAGACGG AGCUGGAGUU GUA | 520_542 |
| BBD-2065 | S65(131) | AACUCCAGCUC CGUCUAUUCA | 523_543 | AS65(132) | UGAAUAGACG GAGCUGGAGU UGU | 521_543 |

(continued)

| Duplex ID | Sense strand sequenceID | Sense strand sequence 5'-3' | Range within NM_1536 09.4 | Antisense strand Sequence ID | Antisense strand sequence 5'-3' | Range within NM_153 609.4 |
|---|---|---|---|---|---|---|
| BBD-2066 | S66(133) | ACUCCAGCUCC GUCUAUUCCU | 524_544 | AS66(134) | AGGAAUAGAC GGAGCUGGAG UUG | 522_544 |
| BBD-2067 | S67(135) | CUCCAGCUCCG UCUAUUCCUU | 525_545 | AS67(136) | AAGGAAUAGA CGGAGCUGGA GUU | 523_545 |
| BBD-2068 | S68(137) | UCCAGGCCUGU GAAGUGAACA | 1112_113 2 | AS68(138) | UGUUCACUUC ACAGGCCUGG AAG | 1110_11 32 |
| BBD-2069 | S69(139) | CCAGGCCUGUG AAGUGAACCU | 1113_113 3 | AS69(140) | AGGUUCACUU CACAGGCCUGG AA | 1111_11 33 |
| BBD-2070 | S70(141) | CAGGCCUGUGA AGUGAACCUA | 1114_113 4 | AS70(142) | UAGGUUCACU UCACAGGCCUG GA | 1112_11 34 |
| BBD-2071 | S71(143) | AGGCCUGUGAA GUGAACCUGA | 1115_113 5 | AS71(144) | UCAGGUUCAC UUCACAGGCCU GG | 1113_11 35 |
| BBD-2072 | S72(145) | GGCCUGUGAAG UGAACCUGAA | 1116_113 6 | AS72(146) | UUCAGGUUCA CUUCACAGGCC UG | 1114_11 36 |
| BBD-2073 | S73(147) | GCCUGUGAAGU GAACCUGACA | 1117_113 7 | AS73(148) | UGUCAGGUUC ACUUCACAGGC CU | 1115_11 37 |
| BBD-2074 | S74(149) | CUGUGAAGUGA ACCUGACGCU | 1119_113 9 | AS74(150) | AGCGUCAGGU UCACUUCACAG GC | 1117_11 39 |
| BBD-2075 | S75(151) | UGUGAAGUGA ACCUGACGCUA | 1120_114 0 | AS75(152) | UAGCGUCAGG UUCACUUCACA GG | 1118_11 40 |
| BBD-2076 | S76(153) | GUGAAGUGAAC CUGACGCUGA | 1121_114 1 | AS76(154) | UCAGCGUCAG GUUCACUUCAC AG | 1119_11 41 |

(continued)

| Duplex ID | Sense strand sequenceID | Sense strand sequence 5'-3' | Range within NM_1536 09.4 | Antisense strand Sequence ID | Antisense strand sequence 5'-3' | Range within NM_153 609.4 |
|---|---|---|---|---|---|---|
| BBD-2077 | S77(155) | AUCGCUGACCG CUGGGUGAUA | 1936_195 6 | AS77(156) | UAUCACCCAGC GGUCAGCGAU GA | 1934_19 56 |
| BBD-2078 | S78(157) | UCGCUGACCGC UGGGUGAUAA | 1937_195 7 | AS78(158) | UUAUCACCCAG CGGUCAGCGA UG | 1935_19 57 |
| BBD-2079 | S79(159) | CGCUGACCGCU GGGUGAUAAU | 1938_195 8 | AS79(160) | AUUAUCACCCA GCGGUCAGCG AU | 1936_19 58 |
| BBD-2080 | S80(161) | GCUGACCGCUG GGUGAUAACA | 1939_195 9 | AS80(162) | UGUUAUCACCC AGCGGUCAGC GA | 1937_19 59 |
| BBD-2081 | S81(163) | GACCGCUGGGU GAUAACAGCU | 1942_1962 | AS81(164) | AGCUGUUAUC ACCCAGCGGUC AG | 1940_1962 |
| BBD-2082 | S82(165) | ACCGCUGGGUG AUAACAGCUA | 1943_196 3 | AS82(166) | UAGCUGUUAU CACCCAGCGGU CA | 1941_19 63 |
| BBD-2083 | S83(167) | UGCUACUCUGG UAUUUCCUAA | 326-345 | AS83(168) | UUAGGAAAUA CCAGAGUAGC ACC | 324-345 |
| BBD-2084 | S84(169) | GUGCUACUCUG GUAUUUCCUA | 325-345 | AS84(170) | UAGGAAAUAC CAGAGUAGCA CCC | 323-345 |
| BBD-2085 | S85(171) | GGUGCUACUCU GGUAUUUCCU | 324-344 | AS85(172) | AGGAAAUACC AGAGUAGCAC CCC | 322-344 |
| BBD-2086 | S86(173) | GGGUGCUACUC UGGUAUUUCA | 323-343 | AS86(174) | UGAAAUACCA GAGUAGCACCC CC | 321-343 |
| BBD-2087 | S87(175) | GGGGUGCUACU CUGGUAUUUA | 322-342 | AS87(176) | UAAAUACCAG AGUAGCACCCC CG | 320-342 |

(continued)

| Duplex ID | Sense strand sequenceID | Sense strand sequence 5'-3' | Range within NM_1536 09.4 | Antisense strand Sequence ID | Antisense strand sequence 5'-3' | Range within NM_153 609.4 |
|---|---|---|---|---|---|---|
| BBD-2088 | S88(177) | GGGGGUGCUAC UCUGGUAUUU | 321-341 | AS88(178) | AAAUACCAGA GUAGCACCCCC GC | 319-341 |
| BBD-2089 | S89(179) | CGGGGGUGCUA CUCUGGUAUU | 320-340 | AS89(180) | AAUACCAGAG UAGCACCCCCG CC | 318-340 |
| BBD-2090 | S90(181) | GCUACUCUGGU AUUUCCUAGG | 327-347 | AS90(182) | CCUAGGAAAU ACCAGAGUAG CAC | 325-347 |
| BBD-2091 | S91(183) | CUACUCUGGUA UUUCCUAGGG | 328-348 | AS91(184) | CCCUAGGAAA UACCAGAGUA GCA | 326-348 |
| BBD-2092 | S92(185) | UACUCUGGUAU UUCCUAGGGU | 329-349 | AS92 (186) | ACCCUAGGAA AUACCAGAGU AGC | 327-349 |
| AD-1554911 | (187) | UGCUACUCUGG UAUUUCCUAU | 326-345 | (188) | AUAGGAAAUA CCAGAGUAGC ACC | 324-345 |

**Example 2: In Vitro Screening of siRNA Using Liposome Transfection in Hep3B Cells**

Cell culture and transfection in 96-Well plate: In vitro experiments were conducted using Hep3B cells, which were cultured in MEM supplemented with 10% FBS, 1X penicillin-streptomycin, and 1X non-essential amino acids. When the cell confluence reached 80%, the cells were digested with trypsin and the cell concentration was measured using a Scepter automated cell counter (Millipore, #PHCC00000). Meanwhile, siRNA, Opti-MEM, and INTERFERin (Polyplus Transfection) were mixed and incubated in a 96-well plate and incubated at room temperature for 10 minutes. Subsequently, the mixture was added to each well containing Hep3B cells in complete culture medium, and the plate was incubated at 37°C with 5% $CO_2$ for 24 hours.

Unmodified siRNA was screened at a final concentration of 1 nM.

RNA extraction and reverse transcription from a 96-well plate: mRNA was extracted from cells in the 96-well plate using the Dynabeads mRNA DIRECT Kit (Ambion). The culture medium was aspirated, and the wells were rinsed once with DPBS. Then, 50-300 μL of cell lysis buffer was added to each well, followed by 20-100 μL of beads. The plate was placed on a shaker for thorough mixing. Next, the 96-well plate was placed on a magnetic stand, and the lysis buffer was aspirated. Each well was then washed with 50-300 μL of Wash Buffer A, mixed thoroughly, and placed on the magnetic stand to remove the buffer. The beads were then resuspended in Wash Buffer B, transferred to a new 96-well plate, and placed on the magnetic stand to remove the buffer. The beads were once again resuspended in Wash Buffer B and transferred to a 96-well PCR plate. Meanwhile, reverse transcription reagents were prepared. The 96-well PCR plate was placed on a magnetic stand, and the wash buffer was aspirated. Then, 20 μL of reverse transcription reagent was added to each well, and the plate was sealed with sealing film. The plate was incubated at 25°C for 10 minutes on a PCR instrument, then at 37°C for 2 hours, followed by 85°C for 5 minutes, and finally cooled to 4°C to complete the reverse transcription process.

Real-time fluorescent quantitative PCR (qPCR): After reverse transcription, the 96-well plate was placed on a magnetic stand until the beads were completely adsorbed to the bottom. The reverse transcription reagent was then aspirated, and the prepared qPCR reaction mixture was added to the 96-well PCR plate. The plate was sealed with a sealing film and subjected to qPCR analysis using the StepOnePlus Real-Time PCR System (Applied Biosystems).

The ΔΔCt method was used to analyze the data, and the test was normalized using cells transfected with a 1 nM negative control sequence.

The negative control AD-1955 sequence was:

Sense strand: CUUACGCUGAGUACUUCGAdTdT (SEQ ID NO.187)
Antisense strand: UCGAAGUACUCAGCGUAAGdTdT (SEQ ID NO.188)

Table 2 shows the screening results of unmodified siRNA transfected at a concentration of 1 nM in Hep3B cells.

| siRNA Duplex | mRNA level in Hep3B cells (%) (relative to negative control cells) | |
|---|---|---|
| | 1nM avg | SD |
| TMPRSS6-hcm-9 | 25.85 | 4.68 |
| BBD-2001 | 44.51 | 0.39 |
| BBD-2002 | 90.44 | 4.29 |
| BBD-2003 | 33.34 | 3.75 |
| BBD-2004 | 68.18 | 5.57 |
| BBD-2005 | 26.47 | 1.40 |
| BBD-2006 | 53.87 | 3.90 |
| BBD-2007 | 69.38 | 8.36 |
| BBD-2008 | 61.20 | 8.62 |
| BBD-2009 | 53.58 | 8.57 |
| BBD-2010 | 62.46 | 13.61 |
| BBD-2011 | 74.77 | 5.71 |
| BBD-2012 | 51.13 | 4.14 |
| BBD-2013 | 32.25 | 3.20 |

(continued)

| siRNA Duplex | mRNA level in Hep3B cells (%) (relative to negative control cells) | |
|---|---|---|
| | 1nM avg | SD |
| BBD-2014 | 29.14 | 4.84 |
| BBD-2015 | 50.11 | 9.25 |
| BBD-2016 | 25.86 | 2.37 |
| BBD-2017 | 40.37 | 2.03 |
| BBD-2018 | 30.26 | 5.31 |
| BBD-2019 | 33.95 | 4.77 |
| BBD-2020 | 36.15 | 3.19 |
| BBD-2021 | 65.42 | 6.82 |
| BBD-2022 | 56.01 | 5.34 |
| BBD-2023 | 45.64 | 7.76 |
| BBD-2024 | 83.71 | 7.26 |
| BBD-2025 | 87.45 | 12.37 |
| BBD-2026 | 67.00 | 6.13 |
| BBD-2027 | 80.47 | 3.76 |
| BBD-2028 | 63.95 | 10.03 |
| BBD-2029 | 99.49 | 17.48 |
| BBD-2030 | 89.02 | 7.22 |
| BBD-2031 | 84.44 | 10.02 |
| BBD-2032 | 88.48 | 5.46 |
| BBD-2033 | 100.49 | 12.70 |
| BBD-2034 | 99.74 | 23.78 |
| BBD-2035 | 67.76 | 9.77 |
| BBD-2036 | 87.30 | 8.49 |
| BBD-2037 | 59.57 | 2.84 |
| BBD-2038 | 63.93 | 2.83 |
| BBD-2039 | 63.40 | 3.87 |
| BBD-2040 | 88.84 | 4.33 |
| BBD-2041 | 74.42 | 7.48 |
| BBD-2042 | 44.17 | 7.48 |
| BBD-2043 | 41.89 | 2.87 |
| BBD-2044 | 37.04 | 4.81 |
| BBD-2045 | 53.40 | 6.91 |
| BBD-2046 | 47.74 | 9.28 |
| BBD-2047 | 18.88 | 2.71 |
| BBD-2048 | 15.07 | 3.47 |
| BBD-2049 | 21.18 | 3.87 |
| BBD-2050 | 18.90 | 2.95 |
| BBD-2051 | 11.19 | 3.86 |

(continued)

| siRNA Duplex | mRNA level in Hep3B cells (%) (relative to negative control cells) | |
|---|---|---|
| | 1nM avg | SD |
| BBD-2052 | 54.69 | 4.51 |
| BBD-2053 | 71.27 | 5.90 |
| BBD-2054 | 61.06 | 15.96 |
| BBD-2055 | 55.83 | 7.12 |
| BBD-2056 | 35.62 | 7.07 |
| BBD-2057 | 42.90 | 7.61 |
| BBD-2058 | 49.78 | 11.87 |
| BBD-2059 | 49.10 | 11.03 |
| BBD-2060 | 65.92 | 13.24 |
| BBD-2061 | 51.45 | 12.52 |
| BBD-2062 | 14.49 | 0.83 |
| BBD-2063 | 51.63 | 14.27 |
| BBD-2064 | 17.71 | 3.05 |
| BBD-2065 | 60.30 | 7.25 |
| BBD-2066 | 46.68 | 7.23 |
| BBD-2067 | 45.39 | 7.07 |
| BBD-2068 | 56.96 | 4.30 |
| BBD-2069 | 60.30 | 14.22 |
| BBD-2070 | 67.24 | 10.25 |
| BBD-2071 | 58.32 | 8.63 |
| BBD-2072 | 57.56 | 9.09 |
| BBD-2073 | 43.46 | 6.72 |
| BBD-2074 | 36.34 | 7.23 |
| BBD-2075 | 57.95 | 11.28 |
| BBD-2076 | 46.99 | 10.47 |
| BBD-2077 | 63.98 | 12.57 |
| BBD-2078 | 20.54 | 5.66 |
| BBD-2079 | 44.91 | 17.92 |
| BBD-2080 | 57.08 | 13.55 |
| BBD-2081 | 73.76 | 8.10 |
| BBD-2082 | 77.17 | 6.88 |
| BBD-2083 | 20.94 | 2.64 |

The experimental results indicate that the siRNA sequences in Table 1 inhibited TMPRSS6 expression in Hep3B cells at varying levels at a concentration of 1 nM. Following comparison and comprehensive evaluation, some of the more active sequences were selected for modification to enhance their in vivo stability and activity.

Table 3 describes multiple modified sense strand sequences of siRNA.

| modified sense strand ID | modified sense strand sequence 5'-3' |
|---|---|
| TMPRSS6-hcm-9B | fUmCfAmCfCmUfGmCfUmUfCmUfUmCfUmGfG*mU*fU |
| BBD-2003SM1 | mG*mU*mGmAmUmCfCmUdGfGfAmAfGmCmCmAmGmUmGmUmA |
| BBD-2005SM1 | mG*mA*mUmCmCmUfGmGdAfAfGmCfCmAmGmUmGmUmGmAmA |
| BBD-2013SM1 | mU*mC*mAmCmCmAfUmCdAfAfCmUfUmCmAmCmCmUmCmCmA |
| BBD-2014SM1 | mC*mA*mCmCmAmUfCmAdAfCfUmUfCmAmCmCmUmCmCmCmA |
| BBD-2015SM1 | mA*mC*mCmAmUmCfAmAdCfUfUmCfAmCmCmUmCmCmCmAmA |
| BBD-2016SM1 | mC*mC*mAmUmCmAfAmCdTfUfCmAfCmCmUmCmCmCmAmGmA |
| BBD-2017SM1 | mC*mA*mUmCmAmAfCmUdTfCfAmCfCmUmCmCmCmAmGmAmU |
| BBD-2018SM1 | mA*mU*mCmAmAmCfUmUdCfAfCmCfUmCmCmCmAmGmAmUmA |
| BBD-2019SM1 | mU*mC*mAmAmCmUfUmCdAfCfCmUfCmCmCmAmGmAmUmCmU |
| BBD-2020SM1 | mC*mA*mAmCmUmUfCmAdCfCfUmCfCmCmAmGmAmUmCmUmA |
| BBD-2023SM1 | mC*mU*mUmCmAmCfCmUdCfCfCmAfGmAmUmCmUmCmCmCmU |
| BBD-2042SM1 | mA*mC*mAmGmCmCfAmUdGfAfCmUfAmCmGmAmCmGmUmGmA |
| BBD-2043SM1 | mG*mA*mCmAmGmCfCmAdTfGfAmCfUmAmCmGmAmCmGmUmA |
| BBD-2044SM1 | mG*mG*mAmCmAmGfCmCdAfUfGmAfCmUmAmCmGmAmCmGmU |
| BBD-2047SM1 | mC*mG*mCmCmUmGfGmGdAfAfCmUfUmAmCmUmAmCmAmAmU |
| BBD-2048SM1 | mG*mC*mCmUmGmGfGmAdAfCfUmUfAmCmUmAmCmAmAmCmU |
| BBD-2049SM1 | mC*mC*mUmGmGmGfAmAdCfUfUmAfCmUmAmCmAmAmCmUmA |
| BBD-2050SM1 | mC*mU*mGmGmGmAfAmCdTfUfAmCfUmAmCmAmAmCmUmCmA |
| BBD-2051SM1 | mU*mG*mGmGmAmAfCmUdTfAfCmUfAmCmAmAmCmUmCmCmA |
| BBD-2056SM1 | mA*mC*mUmUmAmCfUmAdCfAfAmCfUmCmCmAmGmCmUmCmA |
| BBD-2057SM1 | mC*mU*mUmAmCmUfAmCdAfAfCmUfCmCmAmGmCmUmCmCmA |
| BBD-2058SM1 | mU*mU*mAmCmUmAfCmAdAfCfUmCfCmAmGmCmUmCmCmGmU |
| BBD-2059SM1 | mU*mA*mCmUmAmCfAmAdCfUfCmCfAmGmCmUmCmCmGmUmA |
| BBD-2062SM1 | mU*mA*mCmAmAmCfUmCdCfAfGmCfUmCmCmGmUmCmUmAmU |
| BBD-2064SM1 | mC*mA*mAmCmUmCfCmAdGfCfUmCfCmGmUmCmUmAmUmUmA |
| BBD-2074SM1 | mC*mU*mGmUmGmAfAmGdTfGfAmAfCmCmUmGmAmCmGmCmU |
| BBD-2078SM1 | mU*mC*mGmCmUmGfAmCdCfGfCmUfGmGmGmUmGmAmUmAmA |

(continued)

| modified sense strand ID | modified sense strand sequence 5'-3' |
|---|---|
| BBD-2078SM2 | mU*mC*mGmCmUmGfAmCfCfGdCmUfGmGmGmUmGmAmUmAmA |
| BBD-2078SM3 | mU*mC*mGmCmUmGfAmCfCfGfCmUfGmGmGmUmGmAmUmAmA |
| BBD-2083SM1 | mU*mG*mCmUmAmCfUmCdTfGfGmUfAmUmUmUmCmCmUmAmA |
| BBD-2084SM1 | mG*mU*mGmCmUmAfCmUfCfUfGmGmUmAmUmUmUmCmCmUmA |
| BBD-2085SM1 | mG*mG*mUmGmCmUfAmCfUfCfUmGmGmUmAmUmUmUmCmCmU |
| BBD-2086SM1 | mG*mG*mGmUmGmCfUmAfCfUfCmUmGmGmUmAmUmUmUmCmA |
| BBD-2087SM1 | mG*mG*mGmGmUmGfCmUfAfCfUmCmUmGmGmUmAmUmUmUmA |
| BBD-2088SM1 | mG*mG*mGmGmGmUfGmCfUfAfCmUmCmUmGmGmUmAmUmUmU |
| BBD-2089SM1 | mC*mG*mGmGmGmGfUmGfCfUfAmCmUmCmUmGmGmUmAmUmU |
| BBD-2090SM1 | mG*mC*mUmAmCmUfCmUfGfGfUmAmUmUmUmCmCmUmAmGmG |
| BBD-2091SM1 | mC*mU*mAmCmUmCfUmGfGfUfAmUmUmUmCmCmUmAmGmGmG |
| BBD-2092SM1 | mU*mA*mCmUmCmUfGmGfUfAfUmUmUmCmCmUmAmGmGmGmU |

Table 4 describes multiple modified antisense strand sequences of siRNA.

| modified antisense strand ID | modified antisense strand sequence 5'-3' |
|---|---|
| TMPRSS6-hcm-9A | mA*fA*mCfCmAfGmAfAmGfArnAfGmCfAmGfGmU*fG*mA |
| BBD-2003AM1 | mU*fA*mCmAmCmUmGmGmCfUmUdCmCfAmGfGmAmUmCmAmC*mU*mA |
| BBD-2005AM1 | mU*fU*mCmAmCmAmCmUmGfGmCdTmUfCmCfAmGmGmAmUmC*mA*mC |
| BBD-2013AM1 | mU*fG*mGmAmGmGmUmGmAfAmGdTmUfGmAfUmGmGmUmGmA*mU*mC |
| BBD-2014AM1 | mU*fG*mGmGmAmGmGmUmGfAmAdGmUfUmGfAmUmGmGmUmG*mA*mU |

(continued)

| modified antisense strand ID | modified antisense strand sequence 5'-3' |
|---|---|
| BBD-2015AM1 | mU*fU*mGmGmGmAmGmGmUfGmAdAmGfUmUfGmAmUmGmGmU*mG*mA |
| BBD-2016AM1 | mU*fC*mUmGmGmGmAmGmGfUmGdAmAfGmUfUmGmAmUmGmG*mU*mG |
| BBD-2017AM1 | mA*fU*mCmUmGmGmGmAmGfGmUdGmAfAmGfUmUmGmAmUmG*mG*mU |
| BBD-2018AM1 | mU*fA*mUmCmUmGmGmGmAfGmGdTmGfAmAfGmUmUmGmAmU*mG*mG |
| BBD-2019AM1 | mA*fG*mAmUmCmUmGmGmGfAmGdGmUfGmAfAmGmUmUmGmA*mU*mG |
| BBD-2020AM1 | mU*fA*mGmAmUmCmUmGmGfGmAdGmGfUmGfAmAmGmUmUmG*mA*mU |
| BBD-2023AM1 | mA*fG*mGmGmAmGmAmUmCfUmGdGmGfAmGfGmUmGmAmAmG*mU*mU |
| BBD-2042AM1 | mU*fC*mAmCmGmUmCmGmUfAmGdTmCfAmUfGmGmCmUmGmU*mC*mC |
| BBD-2043AM1 | mU*fA*mCmGmUmCmGmUmAfGmUdCmAfUmGfGmCmUmGmUmC*mC*mU |
| BBD-2044AM1 | mA*fC*mGmUmCmGmUmAmGfUmCdAmUfGmGfCmUmGmUmCmC*mU*mC |
| BBD-2047AM1 | mA*fU*mUmGmUmAmGmUmAfAmGdTmUfCmCfCmAmGmGmCmG*mG*mG |
| BBD-2048AM1 | mA*fG*mUmUmGmUmAmGmUfAmAdGmUfUmCfCmCmAmGmGmC*mG*mG |
| BBD-2049AM1 | mU*fA*mGmUmUmGmUmAmGfUmAdAmGfUmUfCmCmCmAmGmG*mC*mG |
| BBD-2050AM1 | mU*fG*mAmGmUmUmGmUmAfGmUdAmAfGmUfUmCmCmCmAmG*mG*mC |
| BBD-2051AM1 | mU*fG*mGmAmGmUmUmGmUfAmGdTmAfAmGfUmUmCmCmCmA*mG*mG |
| BBD-2056AM1 | mU*fG*mAmGmCmUmGmGmAfGmUdTmGfUmAfGmUmAmAmGmU*mU*mC |
| BBD-2057AM1 | mU*fG*mGmAmGmCmUmGmGfAmGdTmUfGmUfAmGmUmAmAmG*mU*mU |

(continued)

| modified antisense strand ID | modified antisense strand sequence 5'-3' |
|---|---|
| BBD-2058AM1 | mA*fC*mGmGmAmGmCmUmGfGmAdGmUfUmGfUmAmGmUmAmA*mG*mU |
| BBD-2059AM1 | mU*fA*mCmGmGmAmGmCmUfGmGdAmGfUmUfGmUmAmGmUmA*mA*mG |
| BBD-2062AM1 | mA*fU*mAmGmAmCmGmGmAfGmCdTmGfGmAfGmUmUmGmUmA*mG*mU |
| BBD-2064AM1 | mU*fA*mAmUmAmGmAmCmGfGmAdGmCfUmGfGmAmGmUmUmG*mU*mA |
| BBD-2074AM1 | mA*fG*mCmGmUmCmAmGmGfUmUdCmAfCmUfUmCmAmCmAmG*mG*mC |
| BBD-2078AM1 | mU*fU*mAmUmCmAmCmCmCfAmGdCmGfGmUfCmAmGmCmGmA*mU*mG |
| BBD-2078AM2 | VPU*fU*mAmUisoGNA-C mAmCmCmCfAmGdCmGfGmUfCmAmGmCmGmA*mU*mG |
| BBD-2078AM3 | VPU*fU*mAmUmCisoGNA-A mCmCmCfAmGdCmGfGmUfCmAmGmCmGmA*mU*mG |
| BBD-2078AM4 | VPU*fU*mAmUmCmAisoGNA-C mCmCfAmGdCmGfGmUfCmAmGmCmGmA*mU*mG |
| BBD-2083AM1 | mU*fU*mAmGmGmAmAmAmUfAmCdCmAfGmAfGmUmAmGmCmA*mC*mC |
| BBD-2084AM1 | mU*fA*mGmGmAmAmAmUmAfCmCdAmGfAmGfUmAmGmCmAmC*mC*mC |
| BBD-2085AM1 | mA*fG*mGmAmAmAmUmAmCfCmAdGmAfGmUfAmGmCmAmCmC*mC*mC |
| BBD-2086AM1 | mU*fG*mAmAmAmUmAmCmCfAmGdAmGfUmAfGmCmAmCmCmC*mC*mC |
| BBD-2087AM1 | mU*fA*mAmAmUmAmCmCmAfGmAdGmUfAmGfCmAmCmCmCmC*mC*mG |
| BBD-2088AM1 | mA*fA*mAmUmAmCmCmAmGfAmGdTmAfGmCfAmCmCmCmCmC*mG*mC |
| BBD-2089AM1 | mA*fA*mUmAmCmCmAmGmAfGmUdAmGfCmAfCmCmCmCmCmG*mC*mC |
| BBD-2090AM1 | mC*fC*mUmAmGmGmAmAmAfUmAdCmCfAmGfAmGmUmAmGmC*mA*mC |
| BBD-2091AM1 | mC*fC*mCmUmAmGmGmAmAfAmUdAmCfCmAfGmAmGmUmAmG*mC*mA |

(continued)

| modified antisense strand ID | modified antisense strand sequence 5'-3' |
|---|---|
| BBD-2092AM1 | mA*fC*mCmCmUmAmGmGmAfAmAdTmAfCmCfAmGmAmGmUmA*mG*mC |

Table 5 describes multiple modified double-stranded siRNA sequences.

| Dupl ex ID | modif ied sense stran d ID | modified sense strand sequence 5'-3' | mod ified antis ense stran d ID | modified antisense strand sequence 5'-3' |
|---|---|---|---|---|
| TM PRS S6-hcm -9 | TMP RSS6 -hcm-9B | fUmCfAmCfCmUfGmCfUmUf CmUfUmCfUmGfG*mU*fU | TM PRS S6-hcm -9A | mA*fA*mCfCmAfGmAfAmGfArn AfGmCfAmGfGmU*fG*mA |
| BB D-2003 .11 | BBD-2003 SM1 | mG*mU*mGmAmUmCfCmUd GfGfAmAfGmCmCmAmGmU mGmUmA | BB D-2003 AM 1 | mU*fA*mCmAmCmUmGmGmCf UmUdCmCfAmGfGmAmUmCmA mC*mU*mA |
| BB D-2005 .11 | BBD-2005 SM1 | mG*mA*mUmCmCmUfGmGd AfAfGmCfCmAmGmUmGmU mGmAmA | BB D-2005 AM 1 | mU*fU*mCmAmCmAmCmUmGf GmCdTmUfCmCfAmGmGmAmU mC*mA*mC |
| BB D-2013 .11 | BBD-2013 SM1 | mU*mC*mAmCmCmAfUmCd AfAfCmUfUmCmAmCmCmU mCmCmA | BB D-2013 AM 1 | mU*fG*mGmAmGmGmUmGmAf AmGdTmUfGmAfUmGmGmUmG mA*mU*mC |
| BB D-2014 .11 | BBD-2014 SM1 | mC*mA*mCmCmAmUfCmAd AfCfUmUfCmAmCmCmUmC mCmCmA | BB D-2014 AM 1 | mU*fG*mGmGmAmGmGmUmGf AmAdGmUfUmGfAmUmGmGmU mG*mA*mU |
| BB D-2015 .11 | BBD-2015 SM1 | mA*mC*mCmAmUmCfAmAd CfUfUmCfAmCmCmUmCmC mCmAmA | BB D-2015 AM 1 | mU*fU*mGmGmGmAmGmGmUf GmAdAmGfUmUfGmAmUmGmG mU*mG*mA |
| BB D-2016 .11 | BBD-2016 SM1 | mC*mC*mAmUmCmAfAmCd TfUfCmAfCmCmUmCmCmC mAmGmA | BB D-2016 AM 1 | mU*fC*mUmGmGmGmAmGmGf UmGdAmAfGmUfUmGmAmUmG mG*mU*mG |
| BB D-2017 .11 | BBD-2017 SM1 | mC*mA*mUmCmAmAfCmUd TfCfAmCfCmUmCmCmCmA mGmAmU | BB D-2017 AM 1 | mA*fU*mCmUmGmGmGmAmGf GmUdGmAfAmGfUmUmGmAmU mG*mG*mU |

(continued)

| Dupl ex ID | modif ied sense stran d ID | modified sense strand sequence 5'-3' | mod ified antis ense stran d ID | modified antisense strand sequence 5'-3' |
|---|---|---|---|---|
| BB D-2018 .11 | BBD-2018 SM1 | mA*mU*mCmAmAmCfUmUdCfAfCmCfUmCmCmCmAmGmAmUmA | BB D-2018 AM 1 | mU*fA*mUmCmUmGmGmGmAfGmGdTmGfAmAfGmUmUmGmAmU*mG*mG |
| BB D-2019 .11 | BBD-2019 SM1 | mU*mC*mAmAmCmUfUmCdAfCfCmUfCmCmCmAmGmAmUmCmU | BB D-2019 AM 1 | mA*fG*mAmUmCmUmGmGmGfAmGdGmUfGmAfAmGmUmUmGmA*mU*mG |
| BB D-2020 .11 | BBD-2020 SM1 | mC*mA*mAmCmUmUfCmAdCfCfUmCfCmCmAmGmAmUmCmUmA | BB D-2020 AM 1 | mU*fA*mGmAmUmCmUmGmGfGmAdGmGfUmGfAmAmGmUmUmG*mA*mU |
| BB D-2023 .11 | BBD-2023 SM1 | mC*mU*mUmCmAmCfCmUdCfCfCmAfGmAmUmCmUmCmCmCmU | BB D-2023 AM 1 | mA*fG*mGmGmAmGmAmUmCfUmGdGmGfAmGfGmUmGmAmAmG*mU*mU |
| BB D-2042 .11 | BBD-2042 SM1 | mA*mC*mAmGmCmCfAmUdGfAfCmUfAmCmGmAmCmGmUmGmA | BB D-2042 AM 1 | mU*fC*mAmCmGmUmCmGmUfAmGdTmCfAmUfGmGmCmUmGmU*mC*mC |
| BB D-2043 .11 | BBD-2043 SM1 | mG*mA*mCmAmGmCfCmAdTfGfAmCfUmAmCmGmAmCmGmUmA | BB D-2043 AM 1 | mU*fA*mCmGmUmCmGmUmAfGmUdCmAfUmGfGmCmUmGmUmC*mC*mU |
| BB D-2044 .11 | BBD-2044 SM1 | mG*mG*mAmCmAmGfCmCdAfUfGmAfCmUmAmCmGmAmCmGmU | BB D-2044 AM 1 | mA*fC*mGmUmCmGmUmAmGfUmCdAmUfGmGfCmUmGmUmCmC*mU*mC |
| BB D-2047 .11 | BBD-2047 SM1 | mC*mG*mCmCmUmGfGmGdAfAfCmUfUmAmCmUmAmCmAmAmU | BB D-2047 AM 1 | mA*fU*mUmGmUmAmGmUmAfAmGdTmUfCmCfCmAmGmGmCmG*mG*mG |

(continued)

| Dupl ex ID | modif ied sense stran d ID | modified sense strand sequence 5'-3' | mod ified antis ense stran d ID | modified antisense strand sequence 5'-3' |
|---|---|---|---|---|
| BB D-2048 .11 | BBD-2048 SM1 | mG*mC*mCmUmGmGfGmAdAfCfUmUfAmCmUmAmCmAmAmCmU | BB D-2048 AM 1 | mA*fG*mUmUmGmUmAmGmUfAmAdGmUfUmCfCmCmAmGmGmC*mG*mG |
| BB D-2049 .11 | BBD-2049 SM1 | mC*mC*mUmGmGmGfAmAdCfUfUmAfCmUmAmCmAmAmCmUmA | BB D-2049 AM 1 | mU*fA*mGmUmUmGmUmAmGfUmAdAmGfUmUfCmCmCmAmGmG*mC*mG |
| BB D-2050 .11 | BBD-2050 SM1 | mC*mU*mGmGmGmAfAmCdTfUfAmCfUmAmCmAmAmCmUmCmA | BB D-2050 AM 1 | mU*fG*mAmGmUmUmGmUmAfGmUdAmAfGmUfUmCmCmCmAmG*mG*mC |
| BB D-2051 .11 | BBD-2051 SM1 | mU*mG*mGmGmAmAfCmUdTfAfCmUfAmCmAmAmCmUmCmCmA | BB D-2051 AM 1 | mU*fG*mGmAmGmUmUmGmUfAmGdTmAfAmGfUmUmCmCmCmA*mG*mG |
| BB D-2056 .11 | BBD-2056 SM1 | mA*mC*mUmUmAmCfUmAdCfAfAmCfUmCmCmAmGmCmUmCmA | BB D-2056 AM 1 | mU*fG*mAmGmCmUmGmGmAfGmUdTmGfUmAfGmUmAmAmGmU*mU*mC |
| BB D-2057 .11 | BBD-2057 SM1 | mC*mU*mUmAmCmUfAmCdAfAfCmUfCmCmAmGmCmUmCmCmA | BB D-2057 AM 1 | mU*fG*mGmAmGmCmUmGmGfAmGdTmUfGmUfAmGmUmAmAmG*mU*mU |
| BB D-2058 .11 | BBD-2058 SM1 | mU*mU*mAmCmUmAfCmAdAfCfUmCfCmAmGmCmUmCmCmGmU | BB D-2058 AM 1 | mA*fC*mGmGmAmGmCmUmGfGmAdGmUfUmGfUmAmGmUmAmA*mG*mU |
| BB D-2059 .11 | BBD-2059 SM1 | mU*mA*mCmUmAmCfAmAdCfUfCmCfAmGmCmUmCmCmGmUmA | BB D-2059 AM 1 | mU*fA*mCmGmGmAmGmCmUfGmGdAmGfUmUfGmUmAmGmUmA*mA*mG |

(continued)

| Dupl ex ID | modif ied sense stran d ID | modified sense strand sequence 5'-3' | mod ified antis ense stran d ID | modified antisense strand sequence 5'-3' |
|---|---|---|---|---|
| BB D-2062 .11 | BBD-2062 SM1 | mU*mA*mCmAmAmCfUmCdCfAfGmCfUmCmCmGmUmCmUmAmU | BB D-2062 AM 1 | mA*fU*mAmGmAmCmGmGmAfGmCdTmGfGmAfGmUmUmGmUmA*mG*mU |
| BB D-2064 .11 | BBD-2064 SM1 | mC*mA*mAmCmUmCfCmAdGfCfUmCfCmGmUmCmUmAmUmUmA | BB D-2064 AM 1 | mU*fA*mAmUmAmGmAmCmGfGmAdGmCfUmGfGmAmGmUmUmG*mU*mA |
| BB D-2074 .11 | BBD-2074 SM1 | mC*mU*mGmUmGmAfAmGdTfGfAmAfCmCmUmGmAmCmGmCmU | BB D-2074 AM 1 | mA*fG*mCmGmUmCmAmGmGfUmUdCmAfCmUfUmCmAmCmAmG*mG*mC |
| BB D-2078 .11 | BBD-2078 SM1 | mU*mC*mGmCmUmGfAmCdCfGfCmUfGmGmGmUmGmAmUmAmA | BB D-2078 AM 1 | mU*fU*mAmUmCmAmCmCmCfAmGdCmGfGmUfCmAmGmCmGmA*mU*mG |
| BB D-2078 .12 | BBD-2078 SM1 | mU*mC*mGmCmUmGfAmCdCfGfCmUfGmGmGmUmGmAmUmAmA | BB D-2078 AM 2 | VPU*fU*mAmUisoGNA-CmAmCmCmCfAmGdCmGfGmUfCmAmGmCmGmA*mU*mG |
| BB D-2078 .13 | BBD-2078 SM1 | mU*mC*mGmCmUmGfAmCdCfGfCmUfGmGmGmUmGmAmUmAmA | BB D-2078 AM 3 | VPU*fU*mAmUmCisoGNA-AmCmCmCfAmGdCmGfGmUfCmAmGmCmGmA*mU*mG |
| BB D-2078 .14 | BBD-2078 SM1 | mU*mC*mGmCmUmGfAmCdCfGfCmUfGmGmGmUmGmAmUmAmA | BB D-2078 AM 4 | VPU*fU*mAmUmCmAisoGNA-CmCmCfAmGdCmGfGmUfCmAmGmCmGmA*mU*mG |
| BB D-2078 .21 | BBD-2078 SM2 | mU*mC*mGmCmUmGfAmCfCfGdCmUfGmGmGmUmGmAmUmAmA | BB D-2078 AM 1 | mU*fU*mAmUmCmAmCmCmCfAmGdCmGfGmUfCmAmGmCmGmA*mU*mG |

(continued)

| Dupl ex ID | modif ied sense stran d ID | modified sense strand sequence 5'-3' | mod ified antis ense stran d ID | modified antisense strand sequence 5'-3' |
|---|---|---|---|---|
| BB D-2078 .22 | BBD-2078 SM2 | mU*mC*mGmCmUmGfAmCf CfGdCmUfGmGmGmUmGmA mUmAmA | BB D-2078 AM 2 | VPU*fU*mAmUisoGNA-C mAmCmCmCfAmGdCmGfGmUfC mAmGmCmGmA*mU*mG |
| BB D-2078 .23 | BBD-2078 SM2 | mU*mC*mGmCmUmGfAmCf CfGdCmUfGmGmGmUmGmA mUmAmA | BB D-2078 AM 3 | VPU*fU*mAmUmCisoGNA-A mCmCmCfAmGdCmGfGmUfCmA mGmCmGmA*mU*mG |
| BB D-2078 .24 | BBD-2078 SM2 | mU*mC*mGmCmUmGfAmCf CfGdCmUfGmGmGmUmGmA mUmAmA | BB D-2078 AM 4 | VPU*fU*mAmUmCmAisoGNA-C mCmCfAmGdCmGfGmUfCmAmG mCmGmA*mU*mG |
| BB D-2078 .31 | BBD-2078 SM3 | mU*mC*mGmCmUmGfAmCf CfGfCmUfGmGmGmUmGmA mUmAmA | BB D-2078 AM 1 | mU*fU*mAmUmCmAmCmCmCf AmGdCmGfGmUfCmAmGmCmG mA*mU*mG |
| BB D-2078 .32 | BBD-2078 SM3 | mU*mC*mGmCmUmGfAmCf CfGfCmUfGmGmGmUmGmA mUmAmA | BB D-2078 AM 2 | VPU*fU*mAmUisoGNA-C mAmCmCmCfAmGdCmGfGmUfC mAmGmCmGmA*mU*mG |
| BB D-2078 .33 | BBD-2078 SM3 | mU*mC*mGmCmUmGfAmCf CfGfCmUfGmGmGmUmGmA mUmAmA | BB D-2078 AM 3 | VPU*fU*mAmUmCisoGNA-A mCmCmCfAmGdCmGfGmUfCmA mGmCmGmA*mU*mG |
| BB D-2078 .34 | BBD-2078 SM3 | mU*mC*mGmCmUmGfAmCf CfGfCmUfGmGmGmUmGmA mUmAmA | BB D-2078 AM 4 | VPU*fU*mAmUmCmAisoGNA-C mCmCfAmGdCmGfGmUfCmAmG mCmGmA*mU*mG |
| BB D-2083 .11 | BBD-2083 SM1 | mU*mG*mCmUmAmCfUmCd TfGfGmUfAmUmUmUmCmC mUmAmA | BB D-2083 AM 1 | mU*fU*mAmGmGmAmAmAmUf AmCdCmAfGmAfGmUmAmGmC mA*mC*mC |

(continued)

| Dupl ex ID | modif ied sense stran d ID | modified sense strand sequence 5'-3' | mod ified antis ense stran d ID | modified antisense strand sequence 5'-3' |
|---|---|---|---|---|
| BB D-2084 .11 | BBD-2084 SM1 | mG*mU*mGmCmUmAfCmUf CfUfGmGmUmAmUmUmUm CmCmUmA | BB D-2084 AM 1 | mU*fA*mGmGmAmAmAmUmAf CmCdAmGfAmGfUmAmGmCmA mC*mC*mC |
| BB D-2085 .11 | BBD-2085 SM1 | mG*mG*mUmGmCmUfAmCf UfCfUmGmGmUmAmUmUm UmCmCmU | BB D-2085 AM 1 | mA*fG*mGmAmAmAmUmAmCf CmAdGmAfGmUfAmGmCmAmC mC*mC*mC |
| BB D-2086 .11 | BBD-2086 SM1 | mG*mG*mGmUmGmCfUmAf CfUfCmUmGmGmUmAmUm UmUmCmA | BB D-2086 AM 1 | mU*fG*mAmAmAmUmAmCmCf AmGdAmGfUmAfGmCmAmCmC mC*mC*mC |
| BB D-2087 .11 | BBD-2087 SM1 | mG*mG*mGmGmUmGfCmUf AfCfUmCmUmGmGmUmAm UmUmUmA | BB D-2087 AM 1 | mU*fA*mAmAmUmAmCmCmAf GmAdGmUfAmGfCmAmCmCmC mC*mC*mG |
| BB D-2088 .11 | BBD-2088 SM1 | mG*mG*mGmGmGmUfGmCf UfAfCmUmCmUmGmGmUm AmUmUmU | BB D-2088 AM 1 | mA*fA*mAmUmAmCmCmAmGf AmGdTmAfGmCfAmCmCmCmC mC*mG*mC |
| BB D-2089 .11 | BBD-2089 SM1 | mC*mG*mGmGmGmGfUmGf CfUfAmCmUmCmUmGmGm UmAmUmU | BB D-2089 AM 1 | mA*fA*mUmAmCmCmAmGmAf GmUdAmGfCmAfCmCmCmCmC mG*mC*mC |
| BB D-2090 .11 | BBD-2090 SM1 | mG*mC*mUmAmCmUfCmUf GfGfUmAmUmUmUmCmCm UmAmGmG | BB D-2090 AM 1 | mC*fC*mUmAmGmGmAmAmAf UmAdCmCfAmGfAmGmUmAmG mC*mA*mC |
| BB D-2091 .11 | BBD-2091 SM1 | mC*mU*mAmCmUmCfUmGf GfUfAmUmUmUmCmCmUm AmGmGmG | BB D-2091 AM 1 | mC*fC*mCmUmAmGmGmAmAf AmUdAmCfCmAfGmAmGmUmA mG*mC*mA |

(continued)

| Dupl ex ID | modif ied sense stran d ID | modified sense strand sequence 5'-3' | mod ified antis ense stran d ID | modified antisense strand sequence 5'-3' |
|---|---|---|---|---|
| BB D-2092 .11 | BBD-2092 SM1 | mU*mA*mCmUmCmUfGmGfUfAfUmUmUmCmCmUmAmGmGmGmU | BB D-2092 AM 1 | mA*fC*mCmCmUmAmGmGmAfAmAdTmAfCmCfAmGmAmGmUmA*mG*mC |

Table 6 describes the experimental results of modified double-stranded siRNA screened at 1 nM transfection concentration in Hep3B cells.

| siRNA Duplex | mRNA level in Hep3B cells (%) (relative to negative control cells) | |
|---|---|---|
| | 1nM avg | SD |
| TMPRSS6-hcm-9 | 47.00 | 16.38 |
| BBD-2003.11 | 81.59 | 1.17 |
| BBD-2005.11 | 58.15 | 1.80 |
| BBD-2013.11 | 64.28 | 12.64 |
| BBD-2014.11 | 72.25 | 10.82 |
| BBD-2015.11 | 57.40 | 2.93 |
| BBD-2016.11 | 94.41 | 4.46 |
| BBD-2017.11 | 54.37 | 7.90 |
| BBD-2018.11 | 62.84 | 6.74 |
| BBD-2019.11 | 60.63 | 8.20 |
| BBD-2020.11 | 62.93 | 2.90 |
| BBD-2023.11 | 66.26 | 15.88 |
| BBD-2042.11 | 80.91 | 1.56 |
| BBD-2043.11 | 53.02 | 4.03 |
| BBD-2044.11 | 81.53 | 17.27 |
| BBD-2047.11 | 35.02 | 9.98 |
| BBD-2048.11 | 42.86 | 6.25 |
| BBD-2049.11 | 56.69 | 23.22 |
| BBD-2050.11 | 41.65 | 11.26 |

Table 7 describes the experimental results of modified double-stranded siRNA screened at 10 nM transfection concentration in Hep3B cells.

| siRNA Duplex | mRNA level in Hep3B cells (%) (relative to negative control cells) | |
|---|---|---|
| | 10nM avg | SD |
| TMPRSS6-hcm-9 | 24.30 | 3.93 |
| BBD-2005.11 | 36.69 | 4.05 |
| BBD-2015.11 | 32.09 | 8.36 |
| BBD-2017.11 | 32.70 | 2.87 |
| BBD-2019.11 | 33.95 | 5.49 |
| BBD-2043.11 | 43.49 | 2.69 |
| BBD-2047.11 | 20.24 | 1.84 |
| BBD-2048.11 | 23.29 | 2.37 |
| BBD-2049.11 | 24.35 | 4.23 |
| BBD-2050.11 | 22.60 | 2.49 |
| BBD-2051.11 | 19.69 | 2.09 |
| BBD-2056.11 | 34.01 | 8.13 |
| BBD-2057.11 | 35.74 | 4.25 |
| BBD-2058.11 | 31.67 | 6.16 |

(continued)

| siRNA Duplex | mRNA level in Hep3B cells (%) (relative to negative control cells) | |
|---|---|---|
| | 10nM avg | SD |
| BBD-2059.11 | 26.23 | 1.98 |
| BBD-2062.11 | 20.22 | 2.50 |
| BBD-2064.11 | 15.02 | 1.88 |
| BBD-2074.11 | 35.48 | 1.93 |
| BBD-2078.11 | 18.56 | 1.59 |
| BBD-2083.11 | 25.09 | 3.81 |
| BBD-2084.11 | 61.88 | 9.44 |
| BBD-2085.11 | 67.91 | 3.21 |
| BBD-2086.11 | 22.26 | 3.33 |
| BBD-2087.11 | 26.69 | 1.43 |
| BBD-2088.11 | 49.22 | 3.00 |
| BBD-2089.11 | 45.02 | 4.75 |
| BBD-2090.11 | 104.19 | 16.66 |
| BBD-2091.11 | 155.06 | 15.06 |
| BBD-2092.11 | 75.44 | 2.58 |

Next, the three sequences BBD-2051.11, BBD-2083.11, and BBD-2047.11,with relatively high activity, were further modified to identify the most optimal modification pattern.

Table 8 describes multiple modified sense strand sequences of BBD-2051.

| modified sense | modified sense strand sequence 5'-3' |
|---|---|
| strand ID | |
| BBD-2051SM1 | mU*mG*mGmGmAmAfCmUdTfAfCmUfAmCmAmAmCmUmCmCmA |
| BBD-2051SM2 | mU*mG*mGmGmAmAfCmUfUfAfCmUmAmCmAmAmCmUmCmCmA |
| BBD-2051SM3 | mU*mG*mGmGmAmAfCmUfUfAdCmUmAmCmAmAmCmUmCmCmA |
| BBD-2051SM4 | mU*mG*mGmGmAmAfCmUfUdAfCmUmAmCmAmAmCmUmCmCmA |
| BBD-2051SM5 | mU*mG*mGmGmAmAfCmUdTfAfCmUmAmCmAmAmCmUmCmCmA |

Table 9 describes multiple modified antisense strand sequences of BBD-2051.

| modified antisense strand ID | modified antisense strand sequence 5'-3' |
|---|---|
| BBD-2051AM1 | mU*fG*mGmAmGmUmUmGmUfAmGdTmAfAmGfUmUmCmCmCmA*mG*mG |

(continued)

| modified antisense strand ID | modified antisense strand sequence 5'-3' |
|---|---|
| BBD-2051AM2 | mU*fG*mGmAmGmUmUmGmUfAdGmUmAfAmGfUmUmCmCmCmA*mG*mG |
| BBD-2051AM3 | mU*fG*mGmAmGmUmUmGmUfAmGmUdAfAmGfUmUmCmCmCmA*mG*mG |
| BBD-2051AM4 | mU*fG*mGmAmGmUmUmGmUfAmGmUmAfAmGfUmUmCmCmCmA*mG*mG |
| BBD-2051AM5 | mU*fG*mGmAisoGNA-GmUmUmGmUfAmGdTmAfAmGfUmUmCmCmCmA*mG*mG |
| BBD-2051AM6 | mU*fG*mGmAmGisoGNA-TmUmGmUfAmGdTmAfAmGfUmUmCmCmCmA*mG*mG |
| BBD-2051AM7 | mU*fG*mGmAmGmUisoGNA-TmGmUfAmGdTmAfAmGfUmUmCmCmCmA*mG*mG |
| BBD-2051AM8 | VPU*fG*mGmAmGmUmUmGmUfAmGdTmAfAmGfUmUmCmCmCmA*mG*mG |
| **BBD-**2051AM9 | VPU*fG*mGmAisoGNA-GmUmUmGmUfAmGdTmAfAmGfUmUmCmCmCmA*mG*mG |
| BBD-2051AM10 | VPU*fG*mGmAmGisoGNA-TmUmGmUfAmGdTmAfAmGfUmUmCmCmCmA*mG*mG |
| BBD-2051AM11 | VPU*fG*mGmAmGmUisoGNA-TmGmUfAmGdTmAfAmGfUmUmCmCmCmA*mG*mG |
| BBD-2051AM12 | mU*dG*mGmAmGmUmUmGmUmAmGdTmAfAmGfUmUmCmCmCmA*mG*mG |
| BBD-2051AM13 | mU*dG*mGmAisoGNA-GmUmUmGmUmAmGdTmAfAmGfUmUmCmCmCmA*mG*mG |
| BBD-2051AM14 | mU*dG*mGmAmGisoGNA-TmUmGmUmAmGdTmAfAmGfUmUmCmCmCmA*mG*mG |
| BBD-2051AM15 | mU*dG*mGmAmGmUisoGNA-TmGmUmAmGdTmAfAmGfUmUmCmCmCmA*mG*mG |
| BBD-2051AM16 | VPU*dG*mGmAmGmUmUmGmUmAmGdTmAfAmGfUmUmCmCmCmA*mG*mG |
| BBD-2051AM17 | VPU*dG*mGmAisoGNA-GmUmUmGmUmAmGdTmAfAmGfUmUmCmCmCmA*mG*mG |
| BBD-2051AM18 | VPU*dG*mGmAmGisoGNA-TmUmGmUmAmGdTmAfAmGfUmUmCmCmCmA*mG*mG |

(continued)

| modified antisense strand ID | modified antisense strand sequence 5'-3' |
|---|---|
| BBD-2051AM19 | VPU*dG*mGmAmGmUisoGNA-TmGmUmAmGdTmAfAmGfUmUmCmCmCmA*mG*mG |

Table 10 describes multiple modified double-stranded siRNA sequences of BBD-2051.

| duplex ID | modified sense strand ID | modified sense strand sequence 5'-3' | modifie d antisens e strand ID | modified antisense strand sequence 5'-3' |
|---|---|---|---|---|
| BBD-2051.2 1 | BBD-2051SM 2 | mU*mG*mGmGmAmAfC mUfUfAfCmUmAmCmA mAmCmUmCmCmA | BBD-2051A M1 | mU*fG*mGmAmGmUmUmGmUfA mGdTmAfAmGfUmUmCmCmCmA *mG*mG |
| BBD-2051.2 5 | BBD-2051SM 2 | mU*mG*mGmGmAmAfC mUfUfAfCmUmAmCmA mAmCmUmCmCmA | BBD-2051A M5 | mU*fG*mGmAisoGNA-GmUmUmGmUfAmGdTmAfAmGf UmUmCmCmCmA*mG*mG |
| BBD-2051.2 6 | BBD-2051SM 2 | mU*mG*mGmGmAmAfC mUfUfAfCmUmAmCmA mAmCmUmCmCmA | BBD-2051A M6 | mU*fG*mGmAmGisoGNA-TmUmGmUfAmGdTmAfAmGfUm UmCmCmCmA*mG*mG |
| BBD-2051.2 7 | BBD-2051SM 2 | mU*mG*mGmGmAmAfC mUfUfAfCmUmAmCmA mAmCmUmCmCmA | BBD-2051A M7 | mU*fG*mGmAmGmUisoGNA-TmGmUfAmGdTmAfAmGfUmUm CmCmCmA*mG*mG |
| BBD-2051.2 8 | BBD-2051SM 2 | mU*mG*mGmGmAmAfC mUfUfAfCmUmAmCmA mAmCmUmCmCmA | BBD-2051A M8 | VPU*fG*mGmAmGmUmUmGmUf AmGdTmAfAmGfUmUmCmCmCm A*mG*mG |
| BBD-2051.2 9 | BBD-2051SM 2 | mU*mG*mGmGmAmAfC mUfUfAfCmUmAmCmA mAmCmUmCmCmA | BBD-2051A M9 | VPU*fG*mGmAisoGNA-GmUmUmGmUfAmGdTmAfAmGf UmUmCmCmCmA*mG*mG |
| BBD-2051.2 10 | BBD-2051SM 2 | mU*mG*mGmGmAmAfC mUfUfAfCmUmAmCmA mAmCmUmCmCmA | BBD-2051A M10 | VPU*fG*mGmAmGisoGNA-TmUmGmUfAmGdTmAfAmGfUm UmCmCmCmA*mG*mG |
| BBD-2051.2 11 | BBD-2051SM 2 | mU*mG*mGmGmAmAfC mUfUfAfCmUmAmCmA mAmCmUmCmCmA | BBD-2051A M11 | VPU*fG*mGmAmGmUisoGNA-TmGmUfAmGdTmAfAmGfUmUm CmCmCmA*mG*mG |

(continued)

| duplex ID | modified sense strand ID | modified sense strand sequence 5'-3' | modifie d antisens e strand ID | modified antisense strand sequence 5'-3' |
|---|---|---|---|---|
| BBD-2051.2 12 | BBD-2051SM 2 | mU*mG*mGmGmAmAfC mUfUfAfCmUmAmCmA mAmCmUmCmCmA | BBD-2051A M12 | mU*dG*mGmAmGmUmUmGmUm AmGdTmAfAmGfUmUmCmCmCm A*mG*mG |
| BBD-2051.2 13 | BBD-2051SM 2 | mU*mG*mGmGmAmAfC mUfUfAfCmUmAmCmA mAmCmUmCmCmA | BBD-2051A M13 | mU*dG*mGmAisoGNA-GmUmUmGmUmAmGdTmAfAmGf UmUmCmCmCmA*mG*mG |
| BBD-2051.2 14 | BBD-2051SM 2 | mU*mG*mGmGmAmAfC mUfUfAfCmUmAmCmA mAmCmUmCmCmA | BBD-2051A M14 | mU*dG*mGmAmGisoGNA-TmUmGmUmAmGdTmAfAmGfUm UmCmCmCmA*mG*mG |
| BBD-2051.2 15 | BBD-2051SM 2 | mU*mG*mGmGmAmAfC mUfUfAfCmUmAmCmA mAmCmUmCmCmA | BBD-2051A M15 | mU*dG*mGmAmGmUisoGNA-TmGmUmAmGdTmAfAmGfUmUm CmCmCmA*mG*mG |
| BBD-2051.216 | BBD-2051SM 2 | mU*mG*mGmGmAmAfC mUfUfAfCmUmAmCmA mAmCmUmCmCmA | BBD-2051AM16 | VPU*dG*mGmAmGmUmUmGmU mAmGdTmAfAmGfUmUmCmCmC mA*mG*mG |
| BBD-2051.2 17 | BBD-2051SM 2 | mU*mG*mGmGmAmAfC mUfUfAfCmUmAmCmA mAmCmUmCmCmA | BBD-2051A M17 | VPU*dG*mGmAisoGNA-GmUmUmGmUmAmGdTmAfAmGf UmUmCmCmCmA*mG*mG |
| BBD-2051.2 18 | BBD-2051SM 2 | mU*mG*mGmGmAmAfC mUfUfAfCmUmAmCmA mAmCmUmCmCmA | BBD-2051A M18 | VPU*dG*mGmAmGisoGNA-TmUmGmUmAmGdTmAfAmGfUm UmCmCmCmA*mG*mG |
| BBD-2051.2 19 | BBD-2051SM 2 | mU*mG*mGmGmAmAfC mUfUfAfCmUmAmCmA mAmCmUmCmCmA | BBD-2051A M19 | VPU*dG*mGmAmGmUisoGNA-TmGmUmAmGdTmAfAmGfUmUm CmCmCmA*mG*mG |

(continued)

| duplex ID | modified sense strand ID | modified sense strand sequence 5'-3' | modifie d antisens e strand ID | modified antisense strand sequence 5'-3' |
|---|---|---|---|---|
| BBD-2051.3 1 | BBD-2051SM 3 | mU*mG*mGmGmAmAfCmUfUfAdCmUmAmCmAmAmCmUmCmCmA | BBD-2051A M1 | mU*fG*mGmAmGmUmUmGmUfAmGdTmAfAmGfUmUmCmCmCmA*mG*mG |
| BBD-2051.3 5 | BBD-2051SM 3 | mU*mG*mGmGmAmAfCmUfUfAdCmUmAmCmAmAmCmUmCmCmA | BBD-2051A M5 | mU*fG*mGmAisoGNA-GmUmUmGmUfAmGdTmAfAmGfUmUmCmCmCmA*mG*mG |
| BBD-2051.3 6 | BBD-2051SM 3 | mU*mG*mGmGmAmAfCmUfUfAdCmUmAmCmAmAmCmUmCmCmA | BBD-2051A M6 | mU*fG*mGmAmGisoGNA-TmUmGmUfAmGdTmAfAmGfUmUmCmCmCmA*mG*mG |
| BBD-2051.3 7 | BBD-2051SM 3 | mU*mG*mGmGmAmAfCmUfUfAdCmUmAmCmAmAmCmUmCmCmA | BBD-2051A M7 | mU*fG*mGmAmGmUisoGNA-TmGmUfAmGdTmAfAmGfUmUmCmCmCmA*mG*mG |
| BBD-2051.3 8 | BBD-2051SM 3 | mU*mG*mGmGmAmAfCmUfUfAdCmUmAmCmAmAmCmUmCmCmA | BBD-2051A M8 | VPU*fG*mGmAmGmUmUmGmUfAmGdTmAfAmGfUmUmCmCmCmA*mG*mG |
| BBD-2051.3 9 | BBD-2051SM 3 | mU*mG*mGmGmAmAfCmUfUfAdCmUmAmCmAmAmCmUmCmCmA | BBD-2051A M9 | VPU*fG*mGmAisoGNA-GmUmUmGmUfAmGdTmAfAmGfUmUmCmCmCmA*mG*mG |
| BBD-2051.3 10 | BBD-2051SM 3 | mU*mG*mGmGmAmAfCmUfUfAdCmUmAmCmAmAmCmUmCmCmA | BBD-2051A M10 | VPU*fG*mGmAmGisoGNA-TmUmGmUfAmGdTmAfAmGfUmUmCmCmCmA*mG*mG |
| BBD-2051.3 11 | BBD-2051SM 3 | mU*mG*mGmGmAmAfCmUfUfAdCmUmAmCmAmAmCmUmCmCmA | BBD-2051A M11 | VPU*fG*mGmAmGmUisoGNA-TmGmUfAmGdTmAfAmGfUmUmCmCmCmA*mG*mG |

(continued)

| duplex ID | modified sense strand ID | modified sense strand sequence 5'-3' | modifie d antisens e strand ID | modified antisense strand sequence 5'-3' |
|---|---|---|---|---|
| BBD-2051.3 12 | BBD-2051SM 3 | mU*mG*mGmGmAmAfC mUfUfAdCmUmAmCmA mAmCmUmCmCmA | BBD-2051A M12 | mU*dG*mGmAmGmUmUmGmUm AmGdTmAfAmGfUmUmCmCmCm A*mG*mG |
| BBD-2051.3 13 | BBD-2051SM 3 | mU*mG*mGmGmAmAfC mUfUfAdCmUmAmCmA mAmCmUmCmCmA | BBD-2051A M13 | mU*dG*mGmAisoGNA-GmUmUmGmUmAmGdTmAfAmGf UmUmCmCmCmA*mG*mG |
| BBD-2051.3 14 | BBD-2051SM 3 | mU*mG*mGmGmAmAfC mUfUfAdCmUmAmCmA mAmCmUmCmCmA | BBD-2051A M14 | mU*dG*mGmAmGisoGNA-TmUmGmUmAmGdTmAfAmGfUm UmCmCmCmA*mG*mG |
| BBD-2051.3 15 | BBD-2051SM 3 | mU*mG*mGmGmAmAfC mUfUfAdCmUmAmCmA mAmCmUmCmCmA | BBD-2051A M15 | mU*dG*mGmAmGmUisoGNA-TmGmUmAmGdTmAfAmGfUmUm CmCmCmA*mG*mG |
| BBD-2051.3 16 | BBD-2051SM 3 | mU*mG*mGmGmAmAfC mUfUfAdCmUmAmCmA mAmCmUmCmCmA | BBD-2051A M16 | VPU*dG*mGmAmGmUmUmGmU mAmGdTmAfAmGfUmUmCmCmC mA*mG*mG |
| BBD-2051.3 17 | BBD-2051SM 3 | mU*mG*mGmGmAmAfC mUfUfAdCmUmAmCmA mAmCmUmCmCmA | BBD-2051A M17 | VPU*dG*mGmAisoGNA-GmUmUmGmUmAmGdTmAfAmGf UmUmCmCmCmA*mG*mG |
| BBD-2051.3 18 | BBD-2051SM 3 | mU*mG*mGmGmAmAfC mUfUfAdCmUmAmCmA mAmCmUmCmCmA | BBD-2051A M18 | VPU*dG*mGmAmGisoGNA-TmUmGmUmAmGdTmAfAmGfUm UmCmCmCmA*mG*mG |
| BBD-2051.3 19 | BBD-2051SM 3 | mU*mG*mGmGmAmAfC mUfUfAdCmUmAmCmA mAmCmUmCmCmA | BBD-2051A M19 | VPU*dG*mGmAmGmUisoGNA-TmGmUmAmGdTmAfAmGfUmUm CmCmCmA*mG*mG |

Example 3: Integration of the hTMPRSS6 (human TMPRSS6) gene into a liver-targeted AAV8 expression vector, followed by production of the virus (VectorBuilder, AAV8LP), and subsequent infection of mice to generate transgenic mice with stabe hTMPRSS6 expression.

Primary mouse hepatocyte extraction: Primary hepatocytes from mouse were isolated using inferior vena cava perfusion, followed by collagenase digestion. The digested liver tissue was filtered through a tissue cell strainer (BIOLOGIX, 15-1070) to obtain viable and healthy primary mouse hepatocytes, which were resuspended in DMEM supplemented with 10% FBS and 1X penicillin-streptomycin. The cell density was determined using a Scepter automated cell counter (Millipore).

96-Well plate transfection: In vitro experiments were conducted using primary mouse hepatocytes. siRNA, Opti-MEM, and INTERFERin (Polyplus Transfection) were combined and incubated in a 96-well plate and incubated at room temperature for 10 minutes (for free uptake siRNA, INTERFERin was not required). Complete culture medium containing primary mouse hepatocytes was then added to each well. The 96-well plate was incubated at 37°C in a 5% $CO_2$ incubator for 24 hours.

RNA extraction and reverse transcription from a 96-well plate: mRNA was extracted from cells in the 96-well plate using the Dynabeads mRNA DIRECT Kit (Ambion). The culture medium was aspirated, and the wells were rinsed once with DPBS. Then, 50-300 μL of cell lysis buffer was added to each well, followed by 20-100 μL of beads. The plate was placed on a shaker for thorough mixing. Next, the 96-well plate was placed on a magnetic stand, and the lysis buffer was aspirated. Each well was then washed with 50-300 μL of Wash Buffer A, mixed thoroughly, and placed on the magnetic stand to remove the buffer. The beads were then resuspended in Wash Buffer B, transferred to a new 96-well plate, and placed on the magnetic stand to remove the buffer. The beads were once again resuspended in Wash Buffer B and transferred to a 96-well PCR plate.Meanwhile, reverse transcription reagents were prepared. The 96-well PCR plate was placed on a magnetic stand, and the wash buffer was aspirated. Then, 20 μL of reverse transcription reagent was added to each well, and the plate was sealed with sealing film. The plate was incubated at 25°C for 10 minutes on a PCR instrument, then at 37°C for 2 hours, followed by 85°C for 5 minutes, and finally cooled to 4°C to complete the reverse transcription process.

Real-time fluorescent quantitative PCR (qPCR): After reverse transcription, the 96-well plate was placed on a magnetic stand until the beads were completely adsorbed to the bottom. The reverse transcription reagent was then aspirated, and the prepared qPCR reaction mixture was added to the 96-well PCR plate. The plate was sealed with a sealing film and subjected to qPCR analysis using the StepOnePlus Real-Time PCR System (Applied Biosystems).The ΔΔCt method was used to analyze the data, and the test was normalized using cells transfected with a 1 nM negative control sequence.

Table 11 describes the experimental results of multiple modified BBD-2051 double-stranded siRNA sequences delivered via GalNAc at a concentration of 1 nM in primary hepatocytes derived from hTMPRSS6-expressing mice.

| siRNA Duplex | hTMPRSS6 mRNA level in primary mouse hepatocytes (%) | |
|---|---|---|
| | (relative to negative control cells) | |
| | 1nM avg | SD |
| BBD-2051.31-Galnac | 68.10 | 21.32 |
| BBD-2051.35-Galnac | 5.33 | 3.94 |
| BBD-2051.36-Galnac | 24.19 | 5.96 |
| BBD-2051.37-Galnac | 17.61 | 2.60 |
| BBD-2051.21-Galnac | 31.55 | 4.88 |
| BBD-2051.25-Galnac | 0.65 | 0.10 |
| BBD-2051.26-Galnac | 3.03 | 0.85 |

Table 12 describes the experimental results of modified BBD-2051 double-stranded siRNA sequences screened at 1 nM transfection concentration in Hep3B cells.

| siRNA Duplex | mRNA level in Hep3B cells (%) (relative to negative control cells) | |
|---|---|---|
| | 1nM avg | SD |
| BBD-2051.21-Galnac | 20.05 | 6.29 |
| BBD-2051.25-Galnac | 12.77 | 1.47 |

(continued)

| siRNA Duplex | mRNA level in Hep3B cells (%) (relative to negative control cells) | |
|---|---|---|
| | 1nM avg | SD |
| BBD-2051.26-Galnac | 13.90 | 2.50 |
| BBD-2051.27-Galnac | 15.63 | 3.54 |
| BBD-2051.29-Galnac | 14.90 | 3.23 |
| BBD-2051.210-Galnac | 15.23 | 1.88 |
| BBD-2051.211-Galnac | 9.40 | 0.51 |

Table 13 describes the experimental results of modified BBD-2051 double-stranded siRNA sequences delivered via GalNAc and screened at various concentrations in primary hepatocytes derived from hTMPRSS6-expressing mice.

| siRNA Duplex | hTMPRSS6 mRNA level in primary mouse hepatocytes (%) (relative to negative control cells) | | | | | |
|---|---|---|---|---|---|---|
| | 10nM avg | SD | 2nM avg | SD | 0.4nM avg | SD |
| BBD-2051.29-Galnac | 15.20 | 1.35 | 14.77 | 3.20 | 66.23 | 29.81 |
| BBD-2051.210-Galnac | 1.30 | 0.36 | 15.00 | 3.71 | 51.80 | 6.52 |
| BBD-2051.211-Galnac | 1.77 | 0.49 | 14.53 | 1.46 | 47.47 | 18.07 |
| BBD-2051.28-Galnac | 2.30 | 1.50 | 26.13 | 3.59 | 63.13 | 14.84 |

Table 14 describes multiple modified sense strand sequences of BBD-2083.

| modified sense strand ID | modified sense strand sequence 5'-3' |
|---|---|
| AD-1554911-S | mU*mG*mCmUmAmCmUmCfUfGfGmUmAmUmUmUmCmCmUmA mU |
| BBD-2083SM1 | mU*mG*mCmUmAmCfUmCdTfGfGmUfAmUmUmUmCmCmUmAm A |
| BBD-2083 SM2 | mU*mG*mCmUmAmCfUmCfUfGdGmUfAmUmUmUmCmCmUmAm A |
| BBD-2083 SM3 | mU*mG*mCmUmAmCfUmCfUfGfGmUmAmUmUmUmCmCmUmAm A |
| BBD-2083SM4 | mU*mG*mCmUmAmCfUmCfUfGdGmUmAmUmUmUmCmCmUmAm A |
| BBD-2083 SM5 | mU*mG*mCmUmAmCfUmCfUdGfGmUmAmUmUmUmCmCmUmAm A |
| BBD-2083 SM6 | mU*mG*mCmUmAmCfUmCdTfGfGmUmAmUmUmUmCmCmUmAm A |

Table 15 describes multiple modified antisense strand sequences of BBD-2083.

| modified antisense strand ID | modified antisense strand sequence 5'-3' |
|---|---|
| AD-1554911-A | mA*dT*mAmGdGmAdAmAmUmAmCdCmAfGmAmGmUmAmGmCmA*mC*mC |
| BBD-2083AM1 | mU*fU*mAmGmGmAmAmAmUfAmCdCmAfGmAfGmUmAmGmCmA*mC*mC |
| BBD-2083AM2 | mU*fU*mAfGfGfAmAfAmUmAmCmCmAfGmAfGmUfAmGmCmA*mC*mC |
| BBD-2083AM3 | mU*fU*mAmGmGmAmAmAmUfAmCfCmAfGmAfGmUmAmGmCmA*mC*mC |
| BBD-2083AM4 | mU*fU*mAmGmGmAmAmAmUfAmCmCmAfGmAfGmUmAmGmCmA*mC*mC |
| BBD-2083AMS | mU*fU*mAmGmGmAmAmAmUmAmCfCmAfGmAfGmUmAmGmCmA*mC*mC |
| BBD-2083AM6 | mU*fU*mAmGmGmAmAmAmUfAfCmCmAfGmAfGmUmAmGmCmA*mC*mC |
| BBD-2083AM7 | mU*fU*mAmGmGmAmAmAmUmAfCmCmAfGmAfGmUmAmGmCmA*mC*mC |
| BBD-2083AM8 | mU*fU*mAmGmGmAmAmAmUfAdCmCmAfGmAfGmUmAmGmCmA*mC*mC |
| BBD-2083AM9 | mU*fU*mAmGmGmAmAmAmUfAmCmCdAfGmAfGmUmAmGmCmA*mC*mC |
| BBD-2083AM10 | mU*fU*mAmGisoGNA-GmAmAmAmUfAmCdCmAfGmAfGmUmAmGmCmA*mC*mC |
| BBD-2083AM11 | mU*fU*mAmGmGisoGNA-AmAmAmUfAmCdCmAfGmAfGmUmAmGmCmA*mC*mC |
| BBD-2083AM12 | mU*fU*mAmGmGmAisoGNA-AmAmUfAmCdCmAfGmAfGmUmAmGmCmA*mC*mC |
| BBD-2083AM13 | VPU*fU*mAmGmGmAmAmAmUfAmCdCmAfGmAfGmUmAmGmCmA*mC*mC |
| BBD-2083AM14 | VPU*fU*mAmGisoGNA-GmAmAmAmUfAmCdCmAfGmAfGmUmAmGmCmA*mC*mC |
| BBD-2083AM15 | VPU*fU*mAmGmGisoGNA-AmAmAmUfAmCdCmAfGmAfGmUmAmGmCmA*mC*mC |
| BBD-2083AM16 | VPU*fU*mAmGmGmAisoGNA-AmAmUfAmCdCmAfGmAfGmUmAmGmCmA*mC*mC |

(continued)

| modified antisense strand ID | modified antisense strand sequence 5'-3' |
|---|---|
| BBD-2083AM17 | mU*dT*mAmGmGmAmAmAmUmAmCdCmAfGmAfGmUmAmGmCmA*mC*mC |
| BBD-2083AM18 | mU*dT*mAmGisoGNA-GmAmAmAmUmAmCdCmAfGmAfGmUmAmGmCmA*mC*mC |
| BBD-2083AM19 | mU*dT*mAmGmGisoGNA-AmAmAmUmAmCdCmAfGmAfGmUmAmGmCmA*mC*mC |
| BBD-2083AM20 | mU*dT*mAmGmGmAisoGNA-AmAmUmAmCdCmAfGmAfGmUmAmGmCmA*mC*mC |
| BBD-2083AM21 | VPU*dT*mAmGmGmAmAmAmUmAmCdCmAfGmAfGmUmAmGmCmA*mC*mC |
| BBD-2083AM22 | VPU*dT*mAmGisoGNA-GmAmAmAmUmAmCdCmAfGmAfGmUmAmGmCmA*mC*mC |
| BBD-2083AM23 | VPU*dT*mAmGmGisoGNA-AmAmAmUmAmCdCmAfGmAfGmUmAmGmCmA*mC*mC |
| BBD-2083AM24 | VPU*dT*mAmGmGmAisoGNA-AmAmUmAmCdCmAfGmAfGmUmAmGmCmA*mC*mC |

Table 16 describes multiple modified double-stranded siRNA sequences of BBD-2083.

| dup lex ID | mo difi ed sens e stra nd ID | modified sense strand sequence 5'-3' | mod ified antis ense stran d ID | modified antisense strand sequence 5'-3' |
|---|---|---|---|---|
| AD - 155 491 1 | AD - 155 491 1-S | mU*mG*mCmUmAmCmUmCfUfGfGmUmAmUmUmUmCmCmUmAmU | AD-1554 911-A | mA*dT*mAmGdGmAdAmAmUmAmCdCmAfGmAmGmUmAmGmCmA*mC*mC |
| BB D-208 3.2 1 | BB D-208 3S M2 | mU*mG*mCmUmAmCfUmCfUfGdGmUfAmUmUmUmCmCmUmAmA | BB D-2083 AM 1 | mU*fU*mAmGmGmAmAmAmUfAmCdCmAfGmAfGmUmAmGmCmA*mC*mC |
| BB D-208 3.3 1 | BB D-208 3S M3 | mU*mG*mCmUmAmCfUmCfUfGfGmUmAmUmUmUmCmCmUmAmA | BB D-2083 AM 1 | mU*fU*mAmGmGmAmAmAmUfAmCdCmAfGmAfGmUmAmGmCmA*mC*mC |
| BB D-208 3.3 3 | BB D-208 3S M3 | mU*mG*mCmUmAmCfUmCfUfGfGmUmAmUmUmUmCmCmUmAmA | BB D-2083 AM 3 | mU*fU*mAmGmGmAmAmAmUfAmCfCmAfGmAfGmUmAmGmCmA*mC*mC |
| BB D-208 3.4 3 | BB D-208 3S M4 | mU*mG*mCmUmAmCfUmCfUfGdGmUmAmUmUmUmCmCmUmAmA | BB D-2083 AM 3 | mU*fU*mAmGmGmAmAmAmUfAmCfCmAfGmAfGmUmAmGmCmA*mC*mC |
| BB D-208 3.3 4 | BB D-208 3S M3 | mU*mG*mCmUmAmCfUmCfUfGfGmUmAmUmUmUmCmCmUmAmA | BB D-2083 AM 4 | mU*fU*mAmGmGmAmAmAmUfAmCmCmAfGmAfGmUmAmGmCmA*mC*mC |
| BB D-208 3.3 5 | BB D-208 3S M3 | mU*mG*mCmUmAmCfUmCfUfGfGmUmAmUmUmUmCmCmUmAmA | BB D-2083 AM 5 | mU*fU*mAmGmGmAmAmAmUmAmCfCmAfGmAfGmUmAmGmCmA*mC*mC |
| BB D-208 3.3 6 | BB D-208 3S M3 | mU*mG*mCmUmAmCfUmCfUfGfGmUmAmUmUmUmCmCmUmAmA | BB D-2083 AM 6 | mU*fU*mAmGmGmAmAmAmUfAfCmCmAfGmAfGmUmAmGmCmA*mC*mC |

(continued)

| dup lex ID | mo difi ed sens e stra nd ID | modified sense strand sequence 5'-3' | mod ified antis ense stran d ID | modified antisense strand sequence 5'-3' |
|---|---|---|---|---|
| BB D-208 3.3 7 | BB D-208 3S M3 | mU*mG*mCmUmAmCfUmCfUfGfGmUmAmUmUmUmCmCmUmAmA | BB D-2083 AM 7 | mU*fU*mAmGmGmAmAmAmUmAfCmCmAfGmAfGmUmAmGmCmA*mC*mC |
| BB D-208 3.3 8 | BB D-208 3S M3 | mU*mG*mCmUmAmCfUmCfUfGfGmUmAmUmUmUmCmCmUmAmA | BB D-2083 AM 8 | mU*fU*mAmGmGmAmAmAmUfAdCmCmAfGmAfGmUmAmGmCmA*mC*mC |
| BB D-208 3.3 9 | BB D-208 3S M3 | mU*mG*mCmUmAmCfUmCfUfGfGmUmAmUmUmUmCmCmUmAmA | BB D-2083 AM 9 | mU*fU*mAmGmGmAmAmAmUfAmCmCdAfGmAfGmUmAmGmCmA*mC*mC |
| BB D-208 3.4 4 | BB D-208 3S M4 | mU*mG*mCmUmAmCfUmCfUfGdGmUmAmUmUmUmCmCmUmAmA | BB D-2083 AM 4 | mU*fU*mAmGmGmAmAmAmUfAmCmCmAfGmAfGmUmAmGmCmA*mC*mC |
| BB D-208 3.5 4 | BB D-208 3S M5 | mU*mG*mCmUmAmCfUmCfUdGfGmUmAmUmUmUmCmCmUmAmA | BB D-2083 AM 4 | mU*fU*mAmGmGmAmAmAmUfAmCmCmAfGmAfGmUmAmGmCmA*mC*mC |
| BB D-208 3.6 4 | BB D-208 3S M6 | mU*mG*mCmUmAmCfUmCdTfGfGmUmAmUmUmUmCmCmUmAmA | BB D-2083 AM 4 | mU*fU*mAmGmGmAmAmAmUfAmCmCmAfGmAfGmUmAmGmCmA*mC*mC |
| BB D-208 3.3 10 | BB D-208 3S M3 | mU*mG*mCmUmAmCfUmCfUfGfGmUmAmUmUmUmCmCmUmAmA | BB D-2083 AM 10 | mU*fU*mAmGisoGNA-GmAmAmAmUfAmCdCmAfGmAfGmUmAmGmCmA*mC*mC |
| BB D-208 3.3 11 | BB D-208 3S M3 | mU*mG*mCmUmAmCfUmCfUfGfGmUmAmUmUmUmCmCmUmAmA | BB D-2083 AM 11 | mU*fU*mAmGmGisoGNA-AmAmAmUfAmCdCmAfGmAfGmUmAmGmCmA*mC*mC |

(continued)

| dup lex ID | mo difi ed sens e stra nd ID | modified sense strand sequence 5'-3' | mod ified antis ense stran d ID | modified antisense strand sequence 5'-3' |
|---|---|---|---|---|
| BB D-208 3.3 12 | BB D-208 3S M3 | mU*mG*mCmUmAmCfUmCfUfGfGmUmAmUmUmUmCmCmUmAmA | BB D-2083 AM 12 | mU*fU*mAmGmGmAisoGNA-AmAmUfAmCdCmAfGmAfGmUmAmGmCmA*mC*mC |
| BB D-208 3.3 13 | BB D-208 3S M3 | mU*mG*mCmUmAmCfUmCfUfGfGmUmAmUmUmUmCmCmUmAmA | BB D-2083 AM 13 | VPU*fU*mAmGmGmAmAmAmUfAmCdCmAfGmAfGmUmAmGmCmA*mC*mC |
| BB D-208 3.3 14 | BB D-208 3S M3 | mU*mG*mCmUmAmCfUmCfUfGfGmUmAmUmUmUmCmCmUmAmA | BB D-2083 AM 14 | VPU*fU*mAmGisoGNA-GmAmAmAmUfAmCdCmAfGmAfGmUmAmGmCmA*mC*mC |
| BB D-208 3.3 15 | BB D-208 3S M3 | mU*mG*mCmUmAmCfUmCfUfGfGmUmAmUmUmUmCmCmUmAmA | BB D-2083 AM 15 | VPU*fU*mAmGmGisoGNA-AmAmAmUfAmCdCmAfGmAfGmUmAmGmCmA*mC*mC |
| BB D-208 3.3 16 | BB D-208 3S M3 | mU*mG*mCmUmAmCfUmCfUfGfGmUmAmUmUmUmCmCmUmAmA | BB D-2083 AM 16 | VPU*fU*mAmGmGmAisoGNA-AmAmUfAmCdCmAfGmAfGmUmAmGmCmA*mC*mC |
| BB D-208 3.3 17 | BB D-208 3S M3 | mU*mG*mCmUmAmCfUmCfUfGfGmUmAmUmUmUmCmCmUmAmA | BB D-2083 AM 17 | mU*dT*mAmGmGmAmAmAmUmAmCdCmAfGmAfGmUmAmGmCmA*mC*mC |
| BB D-208 3.3 18 | BB D-208 3S M3 | mU*mG*mCmUmAmCfUmCfUfGfGmUmAmUmUmUmCmCmUmAmA | BB D-2083 AM 18 | mU*dT*mAmGisoGNA-GmAmAmAmUmAmCdCmAfGmAfGmUmAmGmCmA*mC*mC |
| BB D-208 3.3 19 | BB D-208 3S M3 | mU*mG*mCmUmAmCfUmCfUfGfGmUmAmUmUmUmCmCmUmAmA | BB D-2083 AM 19 | mU*dT*mAmGmGisoGNA-AmAmAmUmAmCdCmAfGmAfGmUmAmGmCmA*mC*mC |

(continued)

| dup lex ID | mo difi ed sens e stra nd ID | modified sense strand sequence 5'-3' | mod ified antis ense stran d ID | modified antisense strand sequence 5'-3' |
|---|---|---|---|---|
| BB D-208 3.3 20 | BB D-208 3S M3 | mU*mG*mCmUmAmCfUmCfUfGfGmUmAmUmUmUmCmCmUmAmA | BB D-2083 AM 20 | mU*dT*mAmGmGmAisoGNA-AmAmUmAmCdCmAfGmAfGmUmAmGmCmA*mC*mC |
| BB D-208 3.3 21 | BB D-208 3S M3 | mU*mG*mCmUmAmCfUmCfUfGfGmUmAmUmUmUmCmCmUmAmA | BB D-2083 AM 21 | VPU*dT*mAmGmGmAmAmAmUmAmCdCmAfGmAfGmUmAmGmCmA*mC*mC |
| BB D-208 3.3 22 | BB D-208 3S M3 | mU*mG*mCmUmAmCfUmCfUfGfGmUmAmUmUmUmCmCmUmAmA | BB D-2083 AM 22 | VPU*dT*mAmGisoGNA-GmAmAmAmUmAmCdCmAfGmAfGmUmAmGmCmA*mC*mC |
| BB D-208 3.3 23 | BB D-208 3S M3 | mU*mG*mCmUmAmCfUmCfUfGfGmUmAmUmUmUmCmCmUmAmA | BB D-2083 AM 23 | VPU*dT*mAmGmGisoGNA-AmAmAmUmAmCdCmAfGmAfGmUmAmGmCmA*mC*mC |
| BB D-208 3.3 24 | BB D-208 3S M3 | mU*mG*mCmUmAmCfUmCfUfGfGmUmAmUmUmUmCmCmUmAmA | BB D-2083 AM 24 | VPU*dT*mAmGmGmAisoGNA-AmAmUmAmCdCmAfGmAfGmUmAmGmCmA*mC*mC |
| BB D-208 3.4 1 | BB D-208 3S M4 | mU*mG*mCmUmAmCfUmCfUfGdGmUmAmUmUmUmCmCmUmAmA | BB D-2083 AM 1 | mU*fU*mAmGmGmAmAmAmUfAmCdCmAfGmAfGmUmAmGmCmA*mC*mC |
| BB D-208 3.4 10 | BB D-208 3S M4 | mU*mG*mCmUmAmCfUmCfUfGdGmUmAmUmUmUmCmCmUmAmA | BB D-2083 AM 10 | mU*fU*mAmGisoGNA-GmAmAmAmUfAmCdCmAfGmAfGmUmAmGmCmA*mC*mC |
| BB D-208 3.4 11 | BB D-208 3S M4 | mU*mG*mCmUmAmCfUmCfUfGdGmUmAmUmUmUmCmCmUmAmA | BB D-2083 AM 11 | mU*fU*mAmGmGisoGNA-AmAmAmUfAmCdCmAfGmAfGmUmAmGmCmA*mC*mC |

(continued)

| dup lex ID | mo difi ed sens e stra nd ID | modified sense strand sequence 5'-3' | mod ified antis ense stran d ID | modified antisense strand sequence 5'-3' |
|---|---|---|---|---|
| BB D-208 3.4 12 | BB D-208 3S M4 | mU*mG*mCmUmAmCfUmCfUfGdGmUmAmUmUmUmCmCmUmAmA | BB D-2083 AM 12 | mU*fU*mAmGmGmAisoGNA-AmAmUfAmCdCmAfGmAfGmUmAmGmCmA*mC*mC |
| BB D-208 3.4 13 | BB D-208 3S M4 | mU*mG*mCmUmAmCfUmCfUfGdGmUmAmUmUmUmCmCmUmAmA | BB D-2083 AM 13 | VPU*fU*mAmGmGmAmAmAmUfAmCdCmAfGmAfGmUmAmGmCmA*mC*mC |
| BB D-208 3.4 14 | BB D-208 3S M4 | mU*mG*mCmUmAmCfUmCfUfGdGmUmAmUmUmUmCmCmUmAmA | BB D-2083 AM 14 | VPU*fU*mAmGisoGNA-GmAmAmAmUfAmCdCmAfGmAfGmUmAmGmCmA*mC*mC |
| BB D-208 3.4 15 | BB D-208 3S M4 | mU*mG*mCmUmAmCfUmCfUfGdGmUmAmUmUmUmCmCmUmAmA | BB D-2083 AM 15 | VPU*fU*mAmGmGisoGNA-AmAmAmUfAmCdCmAfGmAfGmUmAmGmCmA*mC*mC |
| BB D-208 3.4 16 | BB D-208 3S M4 | mU*mG*mCmUmAmCfUmCfUfGdGmUmAmUmUmUmCmCmUmAmA | BB D-2083 AM 16 | VPU*fU*mAmGmGmAisoGNA-AmAmUfAmCdCmAfGmAfGmUmAmGmCmA*mC*mC |
| BB D-208 3.4 17 | BB D-208 3S M4 | mU*mG*mCmUmAmCfUmCfUfGdGmUmAmUmUmUmCmCmUmAmA | BB D-2083 AM 17 | mU*dT*mAmGmGmAmAmAmUmAmCdCmAfGmAfGmUmAmGmCmA*mC*mC |
| BB D-208 3.4 18 | BB D-208 3S M4 | mU*mG*mCmUmAmCfUmCfUfGdGmUmAmUmUmUmCmCmUmAmA | BB D-2083 AM 18 | mU*dT*mAmGisoGNA-GmAmAmAmUmAmCdCmAfGmAfGmUmAmGmCmA*mC*mC |
| BB D-208 3.4 19 | BB D-208 3S M4 | mU*mG*mCmUmAmCfUmCfUfGdGmUmAmUmUmUmCmCmUmAmA | BB D-2083 AM 19 | mU*dT*mAmGmGisoGNA-AmAmAmUmAmCdCmAfGmAfGmUmAmGmCmA*mC*mC |

(continued)

| dup lex ID | mo difi ed sens e stra nd ID | modified sense strand sequence 5'-3' | mod ified antis ense stran d ID | modified antisense strand sequence 5'-3' |
|---|---|---|---|---|
| BB D-208 3.4 20 | BB D-208 3S M4 | mU*mG*mCmUmAmCfUmCfUfGdGmUmAmUmUmUmCmCmUmAmA | BB D-2083 AM 20 | mU*dT*mAmGmGmAisoGNA-AmAmUmAmCdCmAfGmAfGmUmAmGmCmA*mC*mC |
| BB D-208 3.4 21 | BB D-208 3S M4 | mU*mG*mCmUmAmCfUmCfUfGdGmUmAmUmUmUmCmCmUmAmA | BB D-2083 AM 21 | VPU*dT*mAmGmGmAmAmAmUmAmCdCmAfGmAfGmUmAmGmCmA*mC*mC |
| BB D-208 3.4 22 | BB D-208 3S M4 | mU*mG*mCmUmAmCfUmCfUfGdGmUmAmUmUmUmCmCmUmAmA | BB D-2083 AM 22 | VPU*dT*mAmGisoGNA-GmAmAmAmUmAmCdCmAfGmAfGmUmAmGmCmA*mC*mC |
| BB D-208 3.4 23 | BB D-208 3S M4 | mU*mG*mCmUmAmCfUmCfUfGdGmUmAmUmUmUmCmCmUmAmA | BB D-2083 AM 23 | VPU*dT*mAmGmGisoGNA-AmAmAmUmAmCdCmAfGmAfGmUmAmGmCmA*mC*mC |
| BB D-208 3.4 24 | BB D-208 3S M4 | mU*mG*mCmUmAmCfUmCfUfGdGmUmAmUmUmUmCmCmUmAmA | BB D-2083 AM 24 | VPU*dT*mAmGmGmAisoGNA-AmAmUmAmCdCmAfGmAfGmUmAmGmCmA*mC*mC |
| AD - 155 491 1 | AD - 155 491 1-S | mU*mG*mCmUmAmCmUmCfUfGfGmUmAmUmUmUmCmCmUmAmU | AD-1554 911-A | mA*dT*mAmGdGmAdAmAmUmAmCdCmAfGmAmGmUmAmGmCmA*mC*mC |

Table 17 describes the experimental results of multiple modified BBD-2083 double-stranded siRNA sequences and AD-1554911 delivered via GalNAc at a concentration of 1 nM in primary hepatocytes derived from hTMPRSS6-expressing mice.

| siRNA Duplex | hTMPRSS6 mRNA level in primary mouse hepatocytes (%) (relative to negative control cells | |
|---|---|---|
| | 1nM avg | SD |
| TMPRSS6-hcm-9- | 27.69 | 7.00 |
| Galnac | | |
| BBD-2083.21-Galnac | 19.61 | 6.00 |
| BBD-2083.31-Galnac | 8.71 | 1.13 |
| BBD-2083.33-Galnac | 13.36 | 6.68 |
| BBD-2083.43-Galnac | 19.13 | 8.74 |
| BBD-2083.34-Galnac | 13.53 | 7.78 |
| BBD-2083.44-Galnac | 21.65 | 3.11 |
| BBD-2083.35-Galnac | 13.42 | 6.68 |
| BBD-2083.36-Galnac | 8.63 | 2.61 |
| BBD-2083.37-Galnac | 15.09 | 0.41 |
| BBD-2083.38-Galnac | 38.70 | 10.53 |
| BBD-2083.39-Galnac | 12.75 | 2.51 |
| BBD-2083.54-Galnac | 28.72 | 8.42 |
| BBD-2083.64-Galnac | 30.53 | 7.52 |
| BBD-2083.310-Galnac | 5.95 | 2.20 |
| BBD-2083.311-Galnac | 6.37 | 0.99 |
| BBD-2083.312-Galnac | 14.20 | 3.37 |
| BBD-2083.313-Galnac | 0.77 | 0.16 |
| BBD-2083.41-Galnac | 4.48 | 1.46 |
| BBD-2083.410-Galnac | 7.70 | 1.50 |
| BBD-2083.411-Galnac | 13.35 | 11.73 |
| BBD-2083.412-Galnac | 28.42 | 17.90 |
| BBD-2083.413-Galnac | 0.98 | 0.53 |
| AD-1554911-Galnac | 14.60 | 5.68 |

Table 18 describes the experimental results of modified BBD-2083 double-stranded siRNA sequences and AD-1554911 screened at 1 nM transfection concentration in Hep3B cells.

| siRNA Duplex | mRNA level in Hep3B cells (%) (relative to negative control cells) | |
|---|---|---|
| | 1nM avg | SD |
| BBD-2083.31-Galnac | 17.27 | 2.21 |
| BBD-2083.38-Galnac | 46.75 | 7.71 |
| BBD-2083.39-Galnac | 45.10 | 5.65 |
| BBD-2083.34-Galnac | 40.95 | 3.94 |
| BBD-2083.44-Galnac | 47.13 | 5.55 |
| BBD-2083.54-Galnac | 53.17 | 9.15 |
| BBD-2083.64-Galnac | 37.84 | 3.27 |

(continued)

| siRNA Duplex | mRNA level in Hep3B cells (%) (relative to negative control cells) | |
|---|---|---|
| | 1nM avg | SD |
| BBD-2083.310-Galnac | 8.66 | 0.23 |
| BBD-2083.311-Galnac | 13.47 | 3.50 |
| BBD-2083.312-Galnac | 11.08 | 0.50 |
| BBD-2083.41-Galnac | 14.94 | 7.68 |
| AD-1554911-Galnac | 19.46 | 8.51 |

Table 19 describes the experimental results of modified BBD-2083 double-stranded siRNA sequences and AD-1554911 screened at 0.3 nM transfection concentration in Hep3B cells

| siRNA Duplex | mRNA level in Hep3B cells (%) (relative to negative control cells) | |
|---|---|---|
| | 0. 3 rA4 avg | SD |
| BBD-2083.313-Galnac | 33.48 | 7.71 |
| BBD-2083.413-Galnac | 42.68 | 10.02 |
| BBD-2083.414-Galnac | 43.21 | 11.53 |
| BBD-2083.415-Galnac | 52.25 | 13.02 |
| AD-1554911-Galnac | 59.07 | 4.07 |

Through the above experiments, we found that the activity of the 2083 sequence was significantly enhanced in vitro after the addition of VP modifications.

Table 20 describes multiple modified sense strand sequences of BBD-2047.

| modified sense strand ID | modified sense strand sequence 5'-3' |
|---|---|
| BBD-2047SM1 | mC*mG*mCmCmUmGfGmGdAfAfCmUfUmAmCmUmAmCmAmAmU |
| BBD-2047SM2 | mC*mG*mCmCmUmGfGmGfAfAdCmUfUmAmCmUmAmCmAmAmU |
| BBD-2047SM3 | mC*fG*mCfCmUfGmGfGmAfAmCfUmUfAmCfUmAfCmAfAmU |
| BBD-2047SM4 | mC*mG*mCmCmUmGfGmGfAfAfCmUfUmAmCmUmAmCmAmAmU |
| BBD-2047SM5 | mC*mG*mCmCmUmGfGmGfAfAdCmUfUmAmCmUmAmCmAmAmU |
| BBD-2047SM6 | mC*mG*mCmCmUmGfGmGfAmAdCmUfUmAmCmUmAmCmAmAmU |
| BBD-2047SM7 | mC*mG*mCmCmUmGfGmGfAfAdCmUmUmAmCmUmAmCmAmAmU |
| BBD-2047SM8 | mC*mG*mCmCmUmGfGmGfAfAfCmUmUmAmCmUmAmCmAmAmU |
| BBD-2047SM9 | mC*mG*mCmCmUmGfGmGfAdAfCmUmUmAmCmUmAmCmAmAmU |
| BBD-2047SM10 | mC*mG*mCmCmUmGfGmGdAfAfCmUmUmAmCmUmAmCmAmAmU |

Table 21 describes multiple modified antisense strand sequences of BBD-2047.

| modified antisense strand ID | modified antisense strand sequence 5'-3' |
|---|---|
| BBD-2047AM1 | mA*fU*mUmGmUmAmGmUmAfAmGdTmUfCmCfCmAmGmGmCmG*mG*mG |
| BBD-2047AM2 | mA*fU*mUfGfUfAmGfUmAmAmGmUmUfCmCfCmAfGmGmCmG*mG*mG |
| BBD-2047AM3 | fA*mU*fUmGfUmAfGmUmAfAmGdTmUfCmCfCmAmGmGmCmG*mG*m G |
| BBD-2047AM4 | mA*fU*mUmGfUmAmGmUmAfAmGdTmUfCmCfCmAmGmGmCmG*mG*mG |
| BBD-2047AM5 | fA*mU*fUmGfUmAfGmUmAfAmGdTmUfCmCfCmAfGmGmCmG*mG*mG |
| BBD-2047AM6 | fA*mU*fUmGfUmAfGmUfAmAmGdTmUfCmCfCmAfGmGmCmG*mG*mG |
| BBD-2047AM7 | fA*mU*fUmGfUmAfGmUfAmAdGmUmUfCmCfCmAfGmGmCmG*mG*mG |
| BBD-2047AM8 | fA*mU*fUmGfUmAfGmUfAmAdGmUmUfCmCfCmAfGmGfCmG*fG*mG |
| BBD-2047AM9 | fA*mU*fUmGfUmAfGmUmAfAmGmUmUfCmCfCmAmGmGmCmG*mG*mG |
| BBD-2047AM1 0 | fA*mU*fUmGfUmAfGmUmAfAmGmUmUfCmCfCmAmGmGmCmG*mG*mG<br>fA*mU*fUmGfUmAfGmUmAmAmGmUmUfCmCfCmAmGmGmCmG*mG*mG |
| BBD-2047AM1 1 | mA*fU*mUfGfUfAmGfUmAmAmGdTmUfCmCfCmAfGmGmCmG*mG*mG |
| BBD-2047AM1 2 | mA*fU*mUfGfUfAmGfUmAmAdGmUmUfCmCfCmAfGmGmCmG*mG*mG |
| BBD-2047AM1 3 | mA*fU*mUfGfUfAmGfUmAfAmGdTmUfCmCfCmAfGmGmCmG*mG*mG |
| BBD-2047AM1 4 | mA*fU*mUfGfUfAmGfUmAmAmGfUmUfCmCfCmAfGmGmCmG*mG*mG |
| BBD-2047AM1 5 | mA*fU*mUfGfUfAmGfUmAmAfGmUmUfCmCfCmAfGmGmCmG*mG*mG |
| BBD-2047AM1 6 | mA*fU*mUfGfUfAmGfUmAfAmGfUmUfCmCfCmAfGmGmCmG*mG*mG |
| BBD-2047AM1 7 | mA*fU*mUfGfUfAmGfUmAfAfGmUmUfCmCfCmAfGmGmCmG*mG*mG |
| BBD-2047AM1 8 | mA*fU*mUfGfUfAmGfUmAfAmGmUmUfCmCfCmAfGmGmCmG*mG*mG |
| BBD-2047AM1 9 | mA*fU*mUfGfUfAmGfUfAmAmGmUmUfCmCfCmAfGmGmCmG*mG*mG |

(continued)

| modified antisense strand ID | modified antisense strand sequence 5'-3' |
|---|---|
| BBD-2047AM2 0 | mA*fU*mUfGfUfAmGfUmAmAmGmUmUfCmCfCmAmGmGmCmG*mG*mG |
| BBD-2047AM2 1 | mA*fU*mUfGfUfAmGfUmAmAmGmUmUfCmCfCmAfGfGmCmG*mG*mG |
| BBD-2047AM2 2 | mA*fU*mUfGfUfAmGfUmAfAmGmUmUfCmCfCmAmGmGmCmG*mG*mG |
| BBD-2047AM2 3 | mA*fU*mUmGmUmAmGmUmAfAmGmUmUfCmCfCmAmGmGmCmG*mG*mG |
| BBD-2047AM2 4 | mA*fU*mUmGmUmAmGmUmAfAmGfUmUfCmCfCmAmGmGmCmG*mG*mG |
| BBD-2047AM2 5 | mA*fU*mUmGmUmAmGmUmAfAdGmUmUfCmCfCmAmGmGmCmG*mG*mG |
| BBD-2047AM2 6 | mA*fU*mUmGmUmAmGmUmAfAmGmUdTfCmCfCmAmGmGmCmG*mG*mG |

Table 22 describes multiple modified double-stranded siRNA sequences of BBD-2047.

| duplex ID | modified sense strand ID | modified sense strand sequence 5'-3' | modified antisense strand ID | modified antisense strand sequence 5'-3' |
|---|---|---|---|---|
| BBD-2047.14 | BBD-2047SM1 | mC*mG*mCmCmUmGfGmGdAfAfCmUfUmAmCmUmAmCmAmAmU | BBD-2047AM4 | mA*fU*mUmGfUmAmGmUmAfAmGdTmUfCmCfCmAmGmGmCmG*mG*mG |
| BBD-2047.13 | BBD-2047SM1 | mC*mG*mCmCmUmGfGmGdAfAfCmUfUmAmCmUmAmCmAmAmU | BBD-2047AM3 | fA*mU*fUmGfUmAfGmUmAfAmGdTmUfCmCfCmAmGmGmCmG*mG*mG |
| BBD-2047.12 | BBD-2047SM1 | mC*mG*mCmCmUmGfGmGdAfAfCmUfUmAmCmUmAmCmAmAmU | BBD-2047AM2 | mA*fU*mUfGfUfAmGfUmAmAmGmUmUfCmCfCmAfGmGmCmG*mG*mG |
| BBD-2047.42 | BBD-2047SM4 | mC*mG*mCmCmUmGfGmGfAfAfCmUfUmAmCmUmAmCmAmAmU | BBD-2047AM2 | mA*fU*mUfGfUfAmGfUmAmAmGmUmUfCmCfCmAfGmGmCmG*mG*mG |
| BBD-2047.43 | BBD-2047SM4 | mC*mG*mCmCmUmGfGmGfAfAfCmUfUmAmCmUmAmCmAmAmU | BBD-2047AM3 | fA*mU*fUmGfUmAfGmUmAfAmGdTmUfCmCfCmAmGmGmCmG*mG*mG |
| BBD-2047.53 | BBD-2047SM5 | mC*mG*mCmCmUmGfGmGfAfAdCmUfUmAmCmUmAmCmAmAmU | BBD-2047AM3 | fA*mU*fUmGfUmAfGmUmAfAmGdTmUfCmCfCmAmGmGmCmG*mG*mG |
| BBD-2047.55 | BBD-2047SM5 | mC*mG*mCmCmUmGfGmGfAfAdCmUfUmAmCmUmAmCmAmAmU | BBD-2047AM5 | fA*mU*fUmGfUmAfGmUmAfAmGdTmUfCmCfCmAfGmGmCmG*mG*mG |
| BBD-2047.56 | BBD-2047SM5 | mC*mG*mCmCmUmGfGmGfAfAdCmUfUmAmCmUmAmCmAmAmU | BBD-2047AM6 | fA*mU*fUmGfUmAfGmUfAmAmGdTmUfCmCfCmAfGmGmCmG*mG*mG |

(continued)

| duplex ID | modified sense strand ID | modified sense strand sequence 5'-3' | modified antisense strand ID | modified antisense strand sequence 5'-3' |
|---|---|---|---|---|
| BBD-2047.57 | BBD-2047SM5 | mC*mG*mCmCmUmGfGmGfAfAdCmUfUmAmCmUmAmCmAmAmU | BBD-2047AM7 | fA*mU*fUmGfUmAfGmUfAmAdGmUmUfCmCfCmAfGmGmCmG*mG*mG |
| BBD-2047.58 | BBD-2047SM5 | mC*mG*mCmCmUmGfGmGfAfAdCmUfUmAmCmUmAmCmAmAmU | BBD-2047AM8 | fA*mU*fUmGfUmAfGmUfAmAdGmUmUfCmCfCmAfGmGfCmG*fG*mG |
| BBD-2047.59 | BBD-2047SM5 | mC*mG*mCmCmUmGfGmGfAfAdCmUfUmAmCmUmAmCmAmAmU | BBD-2047AM9 | fA*mU*fUmGfUmAfGmUmAfAmGmUmUfCmCfCmAmGmGmCmG*mG*mG |
| BBD-2047.510 | BBD-2047SM5 | mC*mG*mCmCmUmGfGmGfAfAdCmUfUmAmCmUmAmCmAmAmU | BBD-2047AM10 | fA*mU*fUmGfUmAfGmUmAmAmGmUmUfCmCfCmAmGmGmCmG*mG*mG |
| BBD-2047.511 | BBD-2047SM5 | mC*mG*mCmCmUmGfGmGfAfAdCmUfUmAmCmUmAmCmAmAmU | BBD-2047AM11 | mA*fU*mUfGfUfAmGfUmAmAmGdTmUfCmCfCmAfGmGmCmG*mG*mG |
| BBD-2047.512 | BBD-2047SM5 | mC*mG*mCmCmUmGfGmGfAfAdCmUfUmAmCmUmAmCmAmAmU | BBD-2047AM12 | mA*fU*mUfGfUfAmGfUmAmAdGmUmUfCmCfCmAfGmGmCmG*mG*mG |
| BBD-2047.513 | BBD-2047SM5 | mC*mG*mCmCmUmGfGmGfAfAdCmUfUmAmCmUmAmCmAmAmU | BBD-2047AM13 | mA*fU*mUfGfUfAmGfUmAfAmGdTmUfCmCfCmAfGmGmCmG*mG*mG |
| BBD-2047.411 | BBD-2047SM4 | mC*mG*mCmCmUmGfGmGfAfAfCmUfUmAmCmUmAmCmAmAmU | BBD-2047AM11 | mA*fU*mUfGfUfAmGfUmAmAmGdTmUfCmCfCmAfGmGmCmG*mG*mG |

(continued)

| duplex ID | modified sense strand ID | modified sense strand sequence 5'-3' | modified antisense strand ID | modified antisense strand sequence 5'-3' |
|---|---|---|---|---|
| BBD-2047.52 | BBD-2047SM5 | mC*mG*mCmCmUmGfGmGfAfAdCmUfUmAmCmUmAmCmAmAmU | BBD-2047AM2 | mA*fU*mUfGfUfAmGfUmAmAmGmUmUfCmCfCmAfGmGmCmG*mG*mG |
| BBD-2047.514 | BBD-2047SM5 | mC*mG*mCmCmUmGfGmGfAfAdCmUfUmAmCmUmAmCmAmAmU | BBD-2047AM14 | mA*fU*mUfGfUfAmGfUmAmAmGfUmUfCmCfCmAfGmGmCmG*mG*mG |
| BBD-2047.516 | BBD-2047SM5 | mC*mG*mCmCmUmGfGmGfAfAdCmUfUmAmCmUmAmCmAmAmU | BBD-2047AM16 | mA*fU*mUfGfUfAmGfUmAfAmGfUmUfCmCfCmAfGmGmCmG*mG*mG |
| BBD-2047.517 | BBD-2047SM5 | mC*mG*mCmCmUmGfGmGfAfAdCmUfUmAmCmUmAmCmAmAmU | BBD-2047AM17 | mA*fU*mUfGfUfAmGfUmAfAfGmUmUfCmCfCmAfGmGmCmG*mG*mG |
| BBD-2047.518 | BBD-2047SM5 | mC*mG*mCmCmUmGfGmGfAfAdCmUfUmAmCmUmAmCmAmAmU | BBD-2047AM18 | mA*fU*mUfGfUfAmGfUmAfAmGmUmUfCmCfCmAfGmGmCmG*mG*mG |
| BBD-2047.519 | BBD-2047SM5 | mC*mG*mCmCmUmGfGmGfAfAdCmUfUmAmCmUmAmCmAmAmU | BBD-2047AM19 | mA*fU*mUfGfUfAmGfUfAmAmGmUmUfCmCfCmAfGmGmCmG*mG*mG |
| BBD-2047.520 | BBD-2047SM5 | mC*mG*mCmCmUmGfGmGfAfAdCmUfUmAmCmUmAmCmAmAmU | BBD-2047AM20 | mA*fU*mUfGfUfAmGfUmAmAmGmUmUfCmCfCmAmGmGmCmG*mG*mG |
| BBD-2047.521 | BBD-2047SM5 | mC*mG*mCmCmUmGfGmGfAfAdCmUfUmAmCmUmAmCmAmAmU | BBD-2047AM21 | mA*fU*mUfGfUfAmGfUmAmAmGmUmUfCmCfCmAfGfGmCmG*mG*mG |

(continued)

| duplex ID | modified sense strand ID | modified sense strand sequence 5'-3' | modified antisense strand ID | modified antisense strand sequence 5'-3' |
|---|---|---|---|---|
| BBD-2047.611 | BBD-2047SM6 | mC*mG*mCmCmUmGfGmGfAmAdCmUfUmAmCmUmAmCmAmAmU | BBD-2047AM11 | mA*fU*mUfGfUfAmGfUmAmAmGdTmUfCmCfCmAfGmGmCmG*mG*mG |
| BBD-2047.612 | BBD-2047SM6 | mC*mG*mCmCmUmGfGmGfAmAdCmUfUmAmCmUmAmCmAmAmU | BBD-2047AM12 | mA*fU*mUfGfUfAmGfUmAmAdGmUmUfCmCfCmAfGmGmCmG*mG*mG |
| BBD-2047.613 | BBD-2047SM6 | mC*mG*mCmCmUmGfGmGfAmAdCmUfUmAmCmUmAmCmAmAmU | BBD-2047AM13 | mA*fU*mUfGfUfAmGfUmAfAmGdTmUfCmCfCmAfGmGmCmG*mG*mG |
| BBD-2047.614 | BBD-2047SM6 | mC*mG*mCmCmUmGfGmGfAmAdCmUfUmAmCmUmAmCmAmAmU | BBD-2047AM14 | mA*fU*mUfGfUfAmGfUmAmAmGfUmUfCmCfCmAfGmGmCmG*mG*mG |
| BBD-2047.615 | BBD-2047SM6 | mC*mG*mCmCmUmGfGmGfAmAdCmUfUmAmCmUmAmCmAmAmU | BBD-2047AM15 | mA*fU*mUfGfUfAmGfUmAmAfGmUmUfCmCfCmAfGmGmCmG*mG*mG |
| BBD-2047.616 | BBD-2047SM6 | mC*mG*mCmCmUmGfGmGfAmAdCmUfUmAmCmUmAmCmAmAmU | BBD-2047AM16 | mA*fU*mUfGfUfAmGfUmAfAmGfUmUfCmCfCmAfGmGmCmG*mG*mG |
| BBD-2047.617 | BBD-2047SM6 | mC*mG*mCmCmUmGfGmGfAmAdCmUfUmAmCmUmAmCmAmAmU | BBD-2047AM17 | mA*fU*mUfGfUfAmGfUmAfAfGmUmUfCmCfCmAfGmGmCmG*mG*mG |
| BBD-2047.618 | BBD-2047SM6 | mC*mG*mCmCmUmGfGmGfAmAdCmUfUmAmCmUmAmCmAmAmU | BBD-2047AM18 | mA*fU*mUfGfUfAmGfUmAfAmGmUmUfCmCfCmAfGmGmCmG*mG*mG |

(continued)

| duplex ID | modified sense strand ID | modified sense strand sequence 5'-3' | modified antisense strand ID | modified antisense strand sequence 5'-3' |
|---|---|---|---|---|
| BBD-2047.713 | BBD-2047SM7 | mC*mG*mCmCmUmGfGmGfAfAdCmUmUmAmCmUmAmCmAmAmU | BBD-2047AM13 | mA*fU*mUfGfUfAmGfUmAfAmGdTmUfCmCfCmAfGmGmCmG*mG*mG |
| BBD-2047.716 | BBD-2047SM7 | mC*mG*mCmCmUmGfGmGfAfAdCmUmUmAmCmUmAmCmAmAmU | BBD-2047AM16 | mA*fU*mUfGfUfAmGfUmAfAmGfUmUfCmCfCmAfGmGmCmG*mG*mG |
| BBD-2047.72 | BBD-2047SM7 | mC*mG*mCmCmUmGfGmGfAfAdCmUmUmAmCmUmAmCmAmAmU | BBD-2047AM2 | mA*fU*mUfGfUfAmGfUmAmAmGmUmUfCmCfCmAfGmGmCmG*mG*mG |
| BBD-2047.723 | BBD-2047SM7 | mC*mG*mCmCmUmGfGmGfAfAdCmUmUmAmCmUmAmCmAmAmU | BBD-2047AM23 | mA*fU*mUmGmUmAmGmUmAfAmGmUmUfCmCfCmAmGmGmCmG*mG*mG |
| BBD-2047.823 | BBD-2047SM8 | mC*mG*mCmCmUmGfGmGfAfAfCmUmUmAmCmUmAmCmAmAmU | BBD-2047AM23 | mA*fU*mUmGmUmAmGmUmAfAmGmUmUfCmCfCmAmGmGmCmG*mG*mG |
| BBD-2047.824 | BBD-2047SM8 | mC*mG*mCmCmUmGfGmGfAfAfCmUmUmAmCmUmAmCmAmAmU | BBD-2047AM24 | mA*fU*mUmGmUmAmGmUmAfAmGfUmUfCmCfCmAmGmGmCmG*mG*mG |
| BBD-2047.81 | BBD-2047SM8 | mC*mG*mCmCmUmGfGmGfAfAfCmUmUmAmCmUmAmCmAmAmU | BBD-2047AM1 | mA*fU*mUmGmUmAmGmUmAfAmGdTmUfCmCfCmAmGmGmCmG*mG*mG |
| BBD-2047.522 | BBD-2047SM5 | mC*mG*mCmCmUmGfGmGfAfAdCmUfUmAmCmUmAmCmAmAmU | BBD-2047AM22 | mA*fU*mUfGfUfAmGfUmAfAmGmUmUfCmCfCmAmGmGmCmG*mG*mG |

(continued)

| duplex ID | modified sense strand ID | modified sense strand sequence 5'-3' | modified antisense strand ID | modified antisense strand sequence 5'-3' |
|---|---|---|---|---|
| BBD-2047.718 | BBD-2047SM7 | mC*mG*mCmCmUmGfGmGfAfAdCmUmUmAmCmUmAmCmAmAmU | BBD-2047AM18 | mA*fU*mUfGfUfAmGfUmAfAmGmUmUfCmCfCmAfGmGmCmG*mG*mG |
| BBD-2047.720 | BBD-2047SM7 | mC*mG*mCmCmUmGfGmGfAfAdCmUmUmAmCmUmAmCmAmAmU | BBD-2047AM20 | mA*fU*mUfGfUfAmGfUmAmAmGmUmUfCmCfCmAmGmGmCmG*mG*mG |
| BBD-2047.722 | BBD-2047SM7 | mC*mG*mCmCmUmGfGmGfAfAdCmUmUmAmCmUmAmCmAmAmU | BBD-2047AM22 | mA*fU*mUfGfUfAmGfUmAfAmGmUmUfCmCfCmAmGmGmCmG*mG*mG |
| BBD-2047.82 | BBD-2047SM8 | mC*mG*mCmCmUmGfGmGfAfAfCmUmUmAmCmUmAmCmAmAmU | BBD-2047AM2 | mA*fU*mUfGfUfAmGfUmAmAmGmUmUfCmCfCmAfGmGmCmG*mG*mG |
| BBD-2047.822 | BBD-2047SM8 | mC*mG*mCmCmUmGfGmGfAfAfCmUmUmAmCmUmAmCmAmAmU | BBD-2047AM22 | mA*fU*mUfGfUfAmGfUmAfAmGmUmUfCmCfCmAmGmGmCmG*mG*mG |
| BBD-2047.923 | BBD-2047SM9 | mC*mG*mCmCmUmGfGmGfAdAfCmUmUmAmCmUmAmCmAmAmU | BBD-2047AM23 | mA*fU*mUmGmUmAmGmUmAfAmGmUmUfCmCfCmAmGmGmCmG*mG*mG |
| BBD-2047.102 3 | BBD-2047SM10 | mC*mG*mCmCmUmGfGmGdAfAfCmUmUmAmCmUmAmCmAmAmU | BBD-2047AM23 | mA*fU*mUmGmUmAmGmUmAfAmGmUmUfCmCfCmAmGmGmCmG*mG*mG |
| BBD-2047.825 | BBD-2047SM8 | mC*mG*mCmCmUmGfGmGfAfAfCmUmUmAmCmUmAmCmAmAmU | BBD-2047AM25 | mA*fU*mUmGmUmAmGmUmAfAdGmUmUfCmCfCmAmGmGmCmG*mG*mG |

(continued)

| duplex ID | modified sense strand ID | modified sense strand sequence 5'-3' | modified antisense strand ID | modified antisense strand sequence 5'-3' |
|---|---|---|---|---|
| BBD-2047.826 | BBD-2047SM8 | mC*mG*mCmC mUmGfGmGfAf AfCmUmUmAm CmUmAmCmA mAmU | BBD-2047AM26 | mA*fU*mUmG mUmAmGmUm AfAmGmUdTfC mCfCmAmGmG mCmG*mG*mG |

Table 23 describes the experimental results of multiple modified BBD-2047 double-stranded siRNA sequences delivered via GalNAc at a concentration of 10 nM in primary hepatocytes derived from hTMPRSS6-expressing mice.

| siRNA Duplex | hTMPRSS6 mRNA level in primary mouse hepatocytes (%) (relative to negative control cells) | |
|---|---|---|
| | 10nM avg | SD |
| TMPRSS6-hcm-9-Galnac | 13.87 | 1.86 |
| BBD-2047.14-Galnac | 6.03 | 1.13 |
| BBD-2047.13-Galnac | 17.90 | 0.79 |
| BBD-2047.12-Galnac | 4.37 | 0.89 |
| BBD-2047.42-Galnac | 4.09 | 0.76 |
| BBD-2047.43-Galnac | 31.04 | 6.71 |
| BBD-2047.53-Galnac | 34.47 | 3.22 |
| BBD-2047.55-Galnac | 18.63 | 1.62 |
| BBD-2047.56-Galnac | 18.57 | 0.32 |
| BBD-2047.57-Galnac | 43.98 | 14.65 |
| BBD-2047.58-Galnac | 48.34 | 12.99 |
| BBD-2047.59-Galnac | 40.37 | 16.07 |
| BBD-2047.510-Galnac | 33.04 | 1.95 |
| BBD-2047.511-Galnac | 4.22 | 0.49 |
| BBD-2047.512-Galnac | 6.08 | 2.02 |
| BBD-2047.513-Galnac | 4.25 | 1.89 |

Table 24 describes the experimental results of multiple modified BBD-2047 double-stranded siRNA sequences delivered via GalNAc at a concentration of 5 nM in primary hepatocytes derived from hTMPRSS6-expressing mice.

| siRNA Duplex | hTMPRSS6 mRNA level in primary mouse hepatocytes (%) (relative to negative control cells) | |
|---|---|---|
| | 5nM avg | SD |
| TMPRSS6-hcm-9-Galnac | 20.97 | 1.26 |
| BBD-2047.42-Galnac | 5.78 | 1.91 |
| BBD-2047.411-Galnac | 9.79 | 6.34 |
| BBD-2047.52-Galnac | 2.88 | 1.51 |
| BBD-2047.511-Galnac | 17.68 | 6.29 |
| BBD-2047.513-Galnac | 6.11 | 3.97 |
| BBD-2047.514-Galnac | 15.70 | 6.19 |

(continued)

| siRNA Duplex | hTMPRSS6 mRNA level in primary mouse hepatocytes (%) (relative to negative control cells) | |
|---|---|---|
| | 5nM avg | SD |
| BBD-2047.516-Galnac | 7.50 | 1.83 |
| BBD-2047.517-Galnac | 19.90 | 0.76 |
| BBD-2047.518-Galnac | 0.83 | 0.77 |
| BBD-2047.519-Galnac | 2.08 | 0.60 |
| BBD-2047.520-Galnac | 1.25 | 1.62 |
| BBD-2047.521-Galnac | 3.41 | 1.75 |
| BBD-2047.611-Galnac | 12.77 | 3.84 |
| BBD-2047.612-Galnac | 29.73 | 1.21 |
| BBD-2047.613-Galnac | 32.68 | 6.36 |
| BBD-2047.614-Galnac | 23.82 | 11.44 |
| BBD-2047.615-Galnac | 33.94 | 9.72 |
| BBD-2047.616-Galnac | 22.24 | 12.59 |
| BBD-2047.617-Galnac | 29.30 | 4.28 |
| BBD-2047.618-Galnac | 30.85 | 12.61 |
| BBD-2047.713-Galnac | 2.93 | 1.50 |
| BBD-2047.716-Galnac | 2.72 | 2.84 |
| BBD-2047.72-Galnac | 1.18 | 0.61 |
| BBD-2047.723-Galnac | 0.67 | 0.37 |
| BBD-2047.823-Galnac | 1.50 | 0.62 |
| BBD-2047.824-Galnac | 0.73 | 0.15 |
| BBD-2047.81-Galnac | 3.91 | 1.12 |

Table 25 describes the experimental results of multiple modified BBD-2047 double-stranded siRNA sequences delivered via GalNAc at a concentration of 1 nM in primary hepatocytes derived from hTMPRSS6-expressing mice.

| siRNA | | hTMPRSS6 mRNA level in primary |
|---|---|---|
| Duplex | mouse hepatocytes (%) (relative to negative control cells) | |
| | 1nM avg | SD |
| TMPRSS6-hcm-9-Galnac | 27.69 | 8.97 |
| BBD-2047.52-Galnac | 9.02 | 7.06 |
| BBD-2047.522-Galnac | 13.78 | 8.71 |
| BBD-2047.72-Galnac | 10.44 | 3.25 |
| BBD-2047.718-Galnac | 16.27 | 3.47 |
| BBD-2047.723-Galnac | 5.28 | 1.79 |
| BBD-2047.720-Galnac | 39.13 | 11.85 |
| BBD-2047.722-Galnac | 7.19 | 3.85 |
| BBD-2047.72-Galnac | 13.88 | 1.70 |
| BBD-2047.82-Galnac | 12.11 | 6.81 |
| BBD-2047.822-Galnac | 11.31 | 2.81 |

(continued)

| siRNA Duplex | hTMPRSS6 mRNA level in primary mouse hepatocytes (%) (relative to negative control cells) | |
|---|---|---|
| | 1nM avg | SD |
| BBD-2047.823-Galnac | 5.27 | 2.68 |
| BBD-2047.824-Galnac | 10.75 | 1.63 |

Table 26 describes the experimental results of multiple modified BBD-2047 double-stranded siRNA sequences delivered via GalNAc at a concentration of 0.5 nM in primary hepatocytes derived from hTMPRSS6-expressing mice.

| siRNA Duplex | hTMPRSS6 mRNA level in primary mouse hepatocytes (%) (relative to negative control cells) | |
|---|---|---|
| | 0.5nM avg | SD |
| TMPRSS6-hcm-9-Galnac | 57.20 | 16.97 |
| BBD-2047.52-Galnac | 87.16 | 35.19 |
| BBD-2047.518-Galnac | 23.75 | 12.87 |
| BBD-2047.520-Galnac | 26.51 | 12.58 |
| BBD-2047.72-Galnac | 18.07 | 4.88 |
| BBD-2047.716-Galnac | 78.41 | 6.72 |
| BBD-2047.722-Galnac | 28.27 | 28.46 |
| BBD-2047.723-Galnac | 19.50 | 2.07 |
| BBD-2047.823-Galnac | 23.49 | 25.06 |
| BBD-2047.824-Galnac | 10.01 | 3.03 |

Table 27 describes the experimental results of modified BBD-2047 double-stranded siRNA sequences and TMPRSS6-HCM-9 screened at a concentration of 10 nM in Hep3B cells.

| siRNA Duplex | hTMPRSS6 mRNA level in Hep3B cells (%) (relative to negative control cells) | |
|---|---|---|
| | 10nM avg | SD |
| TMPRSS6-hcm-9 | 21.53 | 2.60 |
| BBD-2047.723 | 17.90 | 2.38 |
| BBD-2047.923 | 18.76 | 1.39 |
| BBD-2047.1023 | 18.16 | 1.78 |

**Example 4: Evaluation of IC50 for Inhibition of hTMPRSS6 Expression by Different Sequences in Hep3B Cells**

Cell culture and 96-Well plate transfection: In vitro experiments were conducted using Hep3B cells, which were cultured in MEM supplemented with 10% FBS, 1X penicillin-streptomycin (Gibco, Cat.15070-063), and 1X non-essential amino acids (Cell Cook, Cat.CM1008L). When the cell confluence reached 80%, the cells were digested with trypsin, and the cell density was measured using a Scepter automated cell counter (Millipore, #PHCC00000). Meanwhile, siRNA, Opti-MEM, and INTERFERin (Polyplus Transfection) were mixed in a 96-well plate and incubated at room temperature for 10 minutes. Complete culture medium with Hep3B cells was then added to each well, and the 96-well plate was incubated at 37°C in a 5% $CO_2$ incubator for 24 hours.

siRNA was tested at a maximum concentration of 100 nM, with eight concentration points prepared by three-fold serial dilution.

RNA extraction and reverse transcription from a 96-well plate: mRNA was extracted from cells in the 96-well plate using the Dynabeads mRNA DIRECT Kit (Ambion). The culture medium was aspirated, and the wells were rinsed once with DPBS. Then, 50-300 μL of cell lysis buffer was added to each well, followed by 20-100 μL of beads. The plate was placed on a shaker for thorough mixing. Next, the 96-well plate was placed on a magnetic stand, and the lysis buffer was

aspirated. Each well was then washed with 50-300 μL of Wash Buffer A, mixed thoroughly, and placed on the magnetic stand to remove the buffer. The beads were then resuspended in Wash Buffer B, transferred to a new 96-well plate, and placed on the magnetic stand to remove the buffer. The beads were once again resuspended in Wash Buffer B and transferred to a 96-well PCR plate.Meanwhile, reverse transcription reagents were prepared. The 96-well PCR plate was placed on a magnetic stand, and the wash buffer was aspirated. Then, 20 μL of reverse transcription reagent was added to each well, and the plate was sealed with sealing film. The plate was incubated at 25°C for 10 minutes on a PCR instrument, then at 37°C for 2 hours, followed by 85°C for 5 minutes, and finally cooled to 4°C to complete the reverse transcription process.

Real-time fluorescent quantitative PCR (qPCR): After reverse transcription, the 96-well plate was placed on a magnetic stand until the beads were completely adsorbed to the bottom. The reverse transcription reagent was then aspirated, and the prepared qPCR reaction mixture was added to the 96-well PCR plate. The plate was sealed with a sealing film and subjected to qPCR analysis using the StepOnePlus Real-Time PCR System (Applied Biosystems).

The ΔΔCt method was used to analyze the data, and the test was normalized using cells transfected with a 1 nM negative control sequence.

Table 28 describes the activity results of different modified double-stranded sequences in Hep3B cells.

| siRNA duplex | HEP3B(n=3) | | | |
|---|---|---|---|---|
| | BOTTOM | Top | SLOPE | $IC_{50}$ (nM) |
| TMPRSS6-hcm-9-Galnac | 27.6 | 93.9 | -1.1 | 0.442 |
| BBD-2083.415-Galnac | 24.0 | = 100.0 | -1.1 | 0.07841 |
| BBD-2051.210-Galnac | 18.8 | = 100.0 | -1.4 | 0.03669 |

Example 5: Evaluation of the Effect of Different Modified BBD-2047, BBD-2051, and BBD-2083 Sequences on hTMPRSS6 Expression in the Liver of hTMPRSS6-Expressing Mice

1. Integration of hTMPRSS6 via Adenovirus

Mice were infected with $1\times10^{11}$ to $10\times10^{11}$ purified recombinant AAV8 viral particles to generate transgenic mice with stable hTMPRSS6 expression.

2. Drug Administration

Fourteen days after viral injection, the mice were evenly divided into groups, with four mice per group. The siRNA was dissolved in saline and administered via subcutaneous injection at doses of 1 mg/kg and 3 mg/kg.

3. Liver Sampling and Detection

At different time points after siRNA injection, liver samples were collected. Total RNA was extracted from liver tissue using TRI REAGENT (MRC, Cat.No. TR118), and the extracted RNA was reverse transcribed into cDNA using the PrimeScript RT Reagent Kit (Takara, Cat. RR047A). The prepared qPCR reaction mixture was added to a 96-well PCR plate, sealed, and subjected to qPCR analysis using the StepOnePlus Real-Time PCR System (Applied Biosystems) to quantify hTMPRSS6 expression.

Fourteen days after siRNA injection, hTMPRSS6 expression levels were measured, and the results are shown in Figure 1. At a dose of 1 mg/kg, different modified sequences exhibited varied in vivo activity, with multiple sequences demonstrating significant gene silencing effects in mice, showing greater activity of TMPRSS6-hcm-9-GalNAc.

At different time points after siRNA injection, hTMPRSS6 expression levels were measured, and the results are shown in Figure 2. At a dose of 3 mg/kg, BBD-2083.31-GalNAc and BBD-2083.313-GalNAc exhibited a longer duration of pharmacodynamic effects compared to TMPRSS6-hcm-9-GalNAc and AD-1554911-GalNAc. Additionally, we observed that incorporating a VP modification into BBD-2083 further enhanced its in vivo activity (BBD-2083.313-GalNAc vs. BBD-2083.31-GalNAc).

At different time points after siRNA injection, hTMPRSS6 expression levels were measured, and the results are shown in Figure 3. At a dose of 3 mg/kg, all modified BBD-2051 sequences exhibited high activity, with a longer duration of pharmacodynamic effect compared to TMPRSS6-hcm-9-GalNAc.

**Example 6: Evaluation of the Effects of Different Modified BBD-2051 Sequences on mTMPRSS6 and mHepcidin1 Expression in the Liver and Serum Iron Levels in Wild-Type Mice**

Wild-type mice were subcutaneously administered 10 mg/kg of siRNA, and liver samples were collected on days 7 and 21. Total RNA was extracted from liver tissue using TRI REAGENT (MRC, Cat.No. TR118), and the extracted RNA was reverse transcribed into cDNA using the PrimeScript RT reagent Kit (Takara, Cat. RR047A). The prepared qPCR reaction mixture was added to a 96-well PCR plate, sealed, and subjected to qPCR analysis using the StepOnePlus Real-Time PCR System (Applied Biosystems) to quantify mTMPRSS6 expression levels. The results are shown in Figure 4. The findings indicate that BBD-2051.210 and BBD-2051.211 effectively reduced endogenous TMPRSS6 expression in wild-type mice.

Wild-type mice were subcutaneously administered 10 mg/kg of siRNA. On day 7, a small amount of blood was collected from the tail. The blood was allowed to clot at room temperature for 30 minutes, then centrifuged at 1000×g for 10 minutes, and the supernatant serum was collected. The serum was diluted and processed using the Iron Assay Kit - Colorimetric according to the manufacturer's instructions. Reducer solution was added, and the samples were incubated at 37°C for 15 minutes, followed by the addition of 100 μL of Probe Solution, and further incubation at 37°C for 1 hour. The absorbance at 593 nm was measured, and serum iron concentration was calculated based on a standard curve. The results are shown in Figure 5. The findings indicate that BBD-2051.210 and BBD-2051.211 effctively reduced serum iron levels in wild-type mice.

Example 7: Evaluation of Off-Target Activity of Different Modified BBD-2051 and BBD-2083 Sequences

siRNA-specific off-target sequences were designed, synthesized, and cloned into the psiCheck2 vector. The psiCheck2-Off-target plasmid was co-transfected with siRNA in 24-well plates. Prior to transfection, cells were seeded in 0.5 mL of growth medium per well for 6-8 hours. Using a transfection reagent, 50 ng of psiCheck2-Off-target plasmid and siRNA at concentrations ranging from 0.0001 nM to 100 nM were transfected and incubated at 37°C for 24 hours.

Fluorescence signal values of Renilla luciferase and firefly luciferase were measured using the Dual-Luciferase Reporter Assay System (Promega, Cat#E1980). The normalized value was calculated as follows:

$$\text{Normalized value} = \text{Renilla luciferase reading} / \text{Firefly luciferase reading}$$

Relative expression fold change = Normalized value of the experimental group / Normalized value of the control group

Experimental results, as shown in Figure 6, indicate that introducing ISOGNA at positions 5, 6, and 7 of the antisense strand significantly reduced off-target activity. In vitro experiments demonstrated that the modified BBD-2051 sequences (particularly BBD-2051.29 and BBD-2051.210) exhibited minimal off-target activity and a favorable safety profile.

As shown in Figure 7, in vitro experimental results demonstrated that the modified BBD-2083 sequences exhibited minimal off-target activity and a favorable safety profile.

**Example 8: Evaluation of the In Vitro Metabolic Stability of BBD-2051.210 in Serum and Liver S9 Fractions**

BBD-2051.210-GalNAc was incubated in vitro at 37°C for 24 hours with serum or liver S9 fractions derived from mice, rats, dogs, monkeys, and humans to evaluate its metabolic stability. Experimental data, as shown in Table 29, indicate that in serum from different species, the stability ranking of the antisense strand was mouse > monkey > rat > human > dog, while the stability ranking of the sense strand was monkey > rat > mouse > human > dog. As shown in Table 30, in liver S9 fractions from different species, the stability ranking of the antisense strand was dog > human > monkey > mouse > rat, while the stability ranking of the sense strand was dog > mouse > human > monkey > rat.

Table 29: Serum Stability Results of BBD-2051.210-GalNAc

| 24h residual rate(%) serum | mouse | rat | dog | monkey | human |
|---|---|---|---|---|---|
| Antisense | 102.99 | 91.03 | 71.30 | 96.87 | 87.35 |
| sense | 94.63 | 95.28 | 71.76 | 98.94 | 87.92 |

Table 30: Liver S9 Stability Results of BBD-2051.210-GalNAc

| 24h residual rate(%) liver S9 | mouse | rat | dog | monkey | human |
|---|---|---|---|---|---|
| Antisense | 83.47 | 74.37 | 104.55 | 95.17 | 95.31 |
| sense | 99.47 | 85.95 | 105.21 | 95.40 | 96.02 |

**Example 9: Evaluation of the In Vitro Metabolic Stability of BBD-2051.210 in Liver Homogenate**

BBD-2051.210-GalNAc was incubated in vitro at 37°C for 72 hours with rat liver homogenate to evaluate its metabolic stability. After 72 hours of incubation, the remaining percentage of the antisense strand was 94.98%, while the remaining percentage of the sense strand was 54.24%. These data indicate that BBD-2051.210-GalNAc exhibits stability in liver homogenate.

The embodiments described above represent only certain implementations of the present invention. While provided in detail, they should not be interpreted as limiting the scope of the invention. Those skilled in the art may make various modifications, adaptations, or improvements without departing from the spirit and scope of the invention. Accordingly, the protection scope of the present invention shall be defined solely by the appended claims.

## Claims

**1.** An siRNA or a pharmaceutically acceptable salt thereof for inhibiting the expression of TMPRSS6 in a cell, comprising a sense strand and an antisense strand, wherein the nucleotide sequence is selected from any one of (a) to (e) as set forth herein :

(a) BBD-2051, wherein the sense strand sequence is as set forth in SEQ ID NO:103, and the antisense strand sequence is as set forth in SEQ ID NO: 104;
(b) BBD-2083, wherein the sense strand sequence is as set forth in SEQ ID NO:167, and the antisense strand sequence is as set forth in SEQ ID NO: 168;
(c) BBD-2047, wherein the sense strand sequence is as set forth in SEQ ID NO:95, and the antisense strand sequence is as set forth in SEQ ID NO:96;
(d) BBD-2086, wherein the sense strand sequence is as set forth in SEQ ID NO: 173, and the antisense strand sequence is as set forth in SEQ ID NO: 174;
(e) BBD-2087, wherein the sense strand sequence is as set forth in SEQ ID NO: 175, and the antisense strand sequence is as set forth in SEQ ID NO: 176.

**2.** The siRNA or the pharmaceutically acceptable salt thereof according to claim 1, wherein the sense strand comprises no more than 3, 2, 1, or 0 unmodified nucleotides, wherein the modified nucleotides in the sense strand are independently selected from 2'-O-methyl-modified nucleotides, 2'-deoxynucleotides, and 2'-fluoro-modified nucleotides; wherein the sense strand comprises 1, 2, or 3 phosphorothioate linkages at the 5'-terminus; and wherein the 2'-deoxynucleotide is selected from at least one of the following structures:

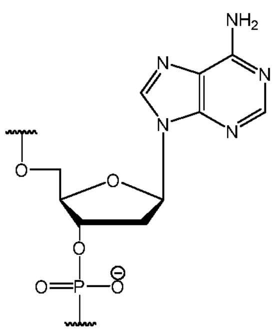
dA

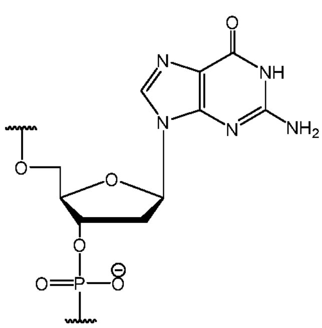
dG

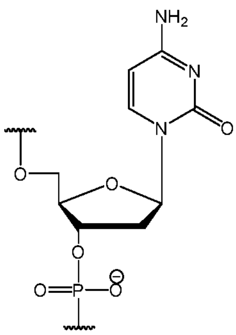
dC

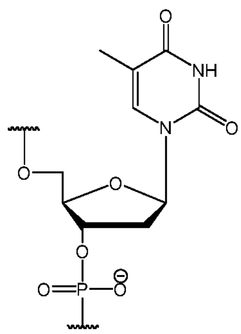
dT ;

wherein the antisense strand comprises no more than 3, 2, 1, or 0 unmodified nucleotides, wherein the modified

nucleotides in the antisense strand are independently selected from 2'-O-methyl-modified nucleotides, 2'-deoxynucleotides, 2'-fluoro-modified nucleotides, 5'-(E)-vinylphosphonate nucleotides, and isomer of glycerol nucleic acid(isoGNA); wherein the antisense strand comprises 1 to 3 phosphorothioate linkages at both the 5'-terminus and the 3'-terminus; and wherein isomer of glycerol nucleic acid(isoGNA) is selected from one of the following structures:

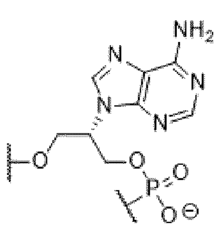
isoGNA-A

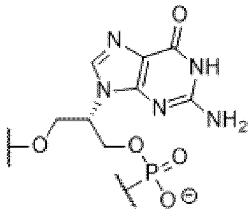
isoGNA-G

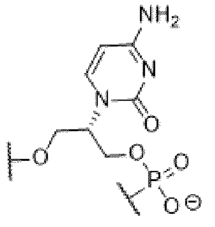
isoGNA-C

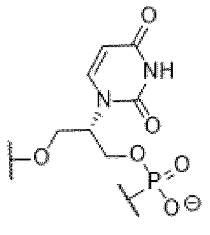
isoGNA-U

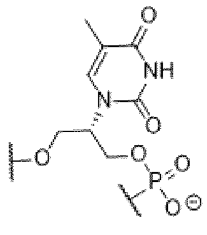
isoGNA-T

;

wherein, the structure of the uridine-5'-(E)-vinylphosphonate nucleotides is as follows:

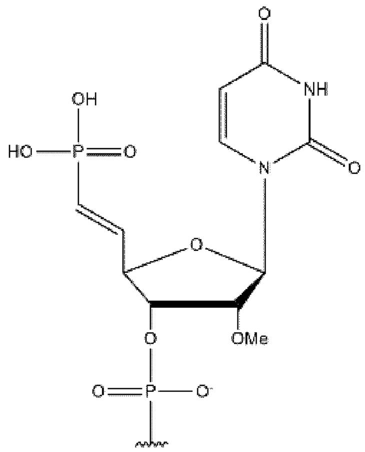
VPU

3. The siRNA or the pharmaceutically acceptable salt thereof according to claim 2, wherein the isoGNA is located in at least one position between position 4 and 8 counted from the 5'-terminus of the antisense strand.

4. The siRNA or the pharmaceutically acceptable salt thereof of any one of claims 1-3, wherein at least one strand of the siRNA comprises a 3'-overhang of at least 1 to 3 nucleotides; and wherein antisense strand of the complementary siRNA at positions 11 to 13 of the 5'- terminus and/ or the sense strand of the complementary siRNA at positions 9 to 11 of the 5'- terminus together comprise a total of 1 to 6 2'-deoxynucleotides.

5. The siRNA or the pharmaceutically acceptable salt thereof of any one of claims 1-3, wherein the modified siRNA is selected from the group consisting of:

BBD-2051.210 :

Sense strand : mU*mG*mGmGmAmAfCmUfUfAfCmUmAmCmAmAmCmUmCmCmA,
Antisense strand : VPU*fG*mGmAmGisoGNA-TmUmGmUfAmGdTmAfAmGfUmUmCmCmCmA*mG*mG,

BBD-2051.211 :

Sense strand : mU*mG*mGmGmAmAfCmUfUfAfCmUmAmCmAmAmCmUmCmCmA,
Antisense strand : VPU*fG*mGmAmGmUisoGNA-TmGmUfAmGdTmAfAmGfUmUmCmCmCmA*mG*mG,

BBD-2051.28 :

Sense strand : mU*mG*mGmGmAmAfCmUfUfAfCmUmAmCmAmAmCmUmCmCmA,
Antisense strand : VPU*fG*mGmAmGmUmUmGmUfAmGdTmAfAmGfUmUmCmCmCmA*mG*mG,

BBD-2083.313 :

Sense strand : mU*mG*mCmUmAmCfUmCfUfGfGmUmAmUmUmUmCmCmUmAmA,
Antisense strand : VPU*fU*mAmGmGmAmAmAmUfAmCdCmAfGmAfGmUmAmGmCmA *mC*mC,

BBD-2083.413 :

Sense strand : mU*mG*mCmUmAmCfUmCfUfGdGmUmAmUmUmUmCmCmUmAmA,
Antisense strand : VPU*fU*mAmGmGmAmAmAmUfAmCdCmAfGmAfGmUmAmGmCmA*mC*mC,

BBD-2051.25 :

Sense strand : mU*mG*mGmGmAmAfCmUfUfAfCmUmAmCmAmAmCmUmCmCmA,
Antisense strand : mU*fG*mGmAisoGNA-GmUmUmGmUfAmGdTmAfAmGfUmUmCmCmCmA*mG*mG,

BBD-2051.26 :

Sense strand : mU*mG*mGmGmAmAfCmUfUfAfCmUmAmCmAmAmCmUmCmCmA,
Antisense strand : mU*fG*mGmAmGisoGNA-TmUmGmUfAmGdTmAfAmGfUmUmCmCmCmA*mG*mG,

BBD-2051.27 :

Sense strand : mU*mG*mGmGmAmAfCmUfUfAfCmUmAmCmAmAmCmUmCmCmA,
Antisense strand : mU*fG*mGmAmGmUisoGNA-TmGmUfAmGdTmAfAmGfUmUmCmCmCmA*mG*mG,

BBD-2051.29 :

Sense strand : mU*mG*mGmGmAmAfCmUfUfAfCmUmAmCmAmAmCmUmCmCmA,
Antisense strand : VPU*fG*mGmAisoGNA-GmUmUmGmUfAmGdTmAfAmGfUmUmCmCmCmA*mG*mG,

BBD-2051.213 :

Sense strand : mU*mG*mGmGmAmAfCmUfUfAfCmUmAmCmAmAmCmUmCmCmA,
Antisense strand : mU*dG*mGmAisoGNA-GmUmUmGmUmAmGdTmAfAmGfUmUmCmCmCmA*mG*mG,

BBD-2051.214 :

Sense strand : mU*mG*mGmGmAmAfCmUfUfAfCmUmAmCmAmAmCmUmCmCmA,
Antisense strand : mU*dG*mGmAmGisoGNA-TmUmGmUmAmGdTmAfAmGfUmUmCmCmCmA*mG*mG,

BBD-2051.215 :

Sense strand : mU*mG*mGmGmAmAfCmUfUfAfCmUmAmCmAmAmCmUmCmCmA,
Antisense strand : mU*dG*mGmAmGmUisoGNA-TmGmUmAmGdTmAfAmGfUmUmCmCmCmA*mG*mG,

BBD-2051.217 :

Sense strand : mU*mG*mGmGmAmAfCmUfUfAfCmUmAmCmAmAmCmUmCmCmA,
Antisense strand : VPU*dG*mGmAisoGNA-GmUmUmGmUmAmGdTmAfAmGfUmUmCmCmCmA*mG*mG,

BBD-2051.218 :

Sense strand : mU*mG*mGmGmAmAfCmUfUfAfCmUmAmCmAmAmCmUmCmCmA,
Antisense strand : VPU*dG*mGmAmGisoGNA-TmUmGmUmAmGdTmAfAmGfUmUmCmCmCmA*mG*mG,

BBD-2051.219 :

Sense strand : mU*mG*mGmGmAmAfCmUfUfAfCmUmAmCmAmAmCmUmCmCmA,
Antisense strand : VPU*dG*mGmAmGmUisoGNA-TmGmUmAmGdTmAfAmGfUmUmCmCmCmA*mG*mG,

BBD-2083.41 :

Sense strand : mU*mG*mCmUmAmCfUmCfUfGdGmUmAmUmUmUmCmCmUmAmA,
Antisense strand : mU*fU*mAmGmGmAmAmAmUfAmCdCmAfGmAfGmUmAmGmMCmA*mC*mC,

BBD-2083.410 :

Sense strand : mU*mG*mCmUmAmCfUmCfUfGdGmUmAmUmUmUmCmCmUmAmA,
Antisense strand : mU*fU*mAmGisoGNA-GmAmAmAmUfAmCdCmAfGmAfGmUmAmGmCmA*mC*mC,

BBD-2083.411 :

Sense strand : mU*mG*mCmUmAmCfUmCfUfGdGmUmAmUmUmUmCmCmUmAmA,
Antisense strand : mU*fU*mAmGmGisoGNA-AmAmAmUfAmCdCmAfGmAfGmUmAmGmCmA*mC*mC,

BBD-2083.412 :

Sense strand : mU*mG*mCmUmAmCfUmCfUfGdGmUmAmUmUmUmCmCmUmAmA,
Antisense strand : mU*fU*mAmGmGmAisoGNA-AmAmUfAmCdCmAfGmAfGmUmAmGmCmA*mC*mC,

BBD-2083.414 :

Sense strand : mU*mG*mCmUmAmCfUmCfUfGdGmUmAmUmUmUmCmCmUmAmA,
Antisense strand : VPU*fU*mAmGisoGNA-GmAmAmAmUfAmCdCmAfGmAfGmUmAmGmCmA*mC*mC,

BBD-2083.415 :

Sense strand : mU*mG*mCmUmAmCfUmCfUfGdGmUmAmUmUmUmCmCmUmAmA,
Antisense strand : VPU*fU*mAmGmGisoGNA-AmAmAmUfAmCdCmAfGmAfGmUmAmGmCmA*mC*mC,

BBD-2083.416 :

Sense strand : mU*mG*mCmUmAmCfUmCfUfGdGmUmAmUmUmUmCmCmUmAmA,
Antisense strand : VPU*fU*mAmGmGmAisoGNA-AmAmUfAmCdCmAfGmAfGmUmAmGmCmA*mC*mC,

BBD-2083.418 :

Sense strand : mU*mG*mCmUmAmCfUmCfUfGdGmUmAmUmUmUmCmCmUmAmA,
Antisense strand : mU*dT*mAmGisoGNA-GmAmAmAmUmAmCdCmAfGmAfGmUmAmGmCmA*mC*mC,

BBD-2083.419 :

Sense strand : mU*mG*mCmUmAmCfUmCfUfGdGmUmAmUmUmUmCmCmUmAmA,
Antisense strand : mU*dT*mAmGmGisoGNA-AmAmAmUmAmCdCmAfGmAfGmUmAmGmCmA*mC*mC,

BBD-2083.420 :

Sense strand : mU*mG*mCmUmAmCfUmCfUfGdGmUmAmUmUmUmCmCmUmAmA,
Antisense strand : mU*dT*mAmGmGmAisoGNA-AmAmUmAmCdCmAfGmAfGmUmAmGmCmA*mC*mC,

BBD-2083.422 :

Sense strand : mU*mG*mCmUmAmCfUmCfUfGdGmUmAmUmUmUmCmCmUmAmA,
Antisense strand : VPU*dT*mAmGisoGNA-GmAmAmAmUmAmCdCmAfGmAfGmUmAmGmCmA*mC*mC,

BBD-2083.423 :

Sense strand : mU*mG*mCmUmAmCfUmCfUfGdGmUmAmUmUmUmCmCmUmAmA,
Antisense strand : VPU*dT*mAmGmGisoGNA-AmAmAmUmAmCdCmAfGmAfGmUmAmGmCmA*mC*mC,

BBD-2083.424 :

Sense strand : mU*mG*mCmUmAmCfUmCfUfGdGmUmAmUmUmUmCmCmUmAmA,
Antisense strand : VPU*dT*mAmGmGmAisoGNA-AmAmUmAmCdCmAfGmAfGmUmAmGmCmA*mC*mC.

**6.** The siRNA or the pharmaceutically acceptable salt thereof according to any one of claims 1-5, wherein the pharmaceutically acceptable salt is selected from a sodium salt or a potassium salt.

**7.** The siRNA or pharmaceutically acceptable salt thereof according to any one of claims 1 to 6, for use in the manufacture of a biological agent or pharmaceutical composition for the inhibition of TMPRSS6 expression.

**8.** A biological agent or pharmaceutical composition for inhibiting the expression of TMPRSS6, wherein, the biological agent or pharmaceutical composition comprises the siRNA or the pharmaceutically acceptable salt thereof according to any one of claims 1 to 6, and a targeting delivery ligand or delivery vehicle conjugated to or encapsulating the siRNA.

**9.** The biological agent or pharmaceutical composition of claim 8, wherein the ligand is an N-acetylgalactosamine derivative; or the delivery vehicle is a liposome, peptide, or antibody specifically targeted to liver cells.

**10.** The biological agent or pharmaceutical composition according to claim 9, wherein the structure of the N-acetylgalactosamine derivative is as follows:

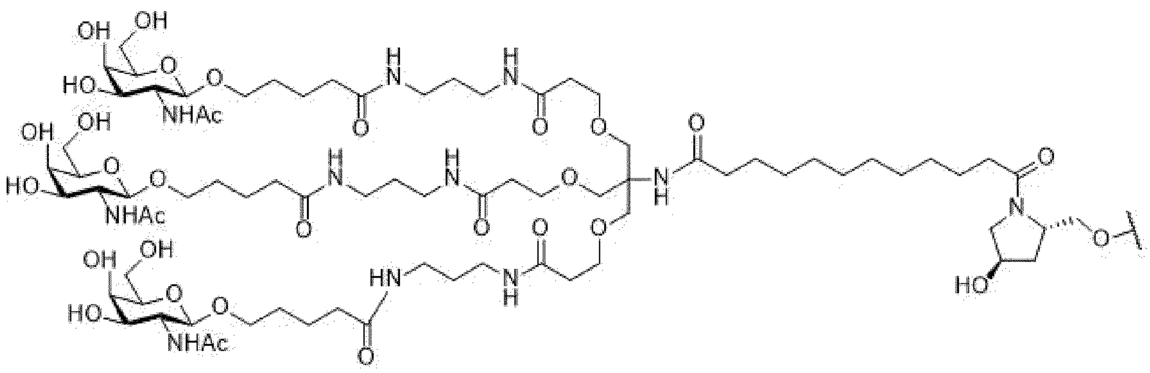

or

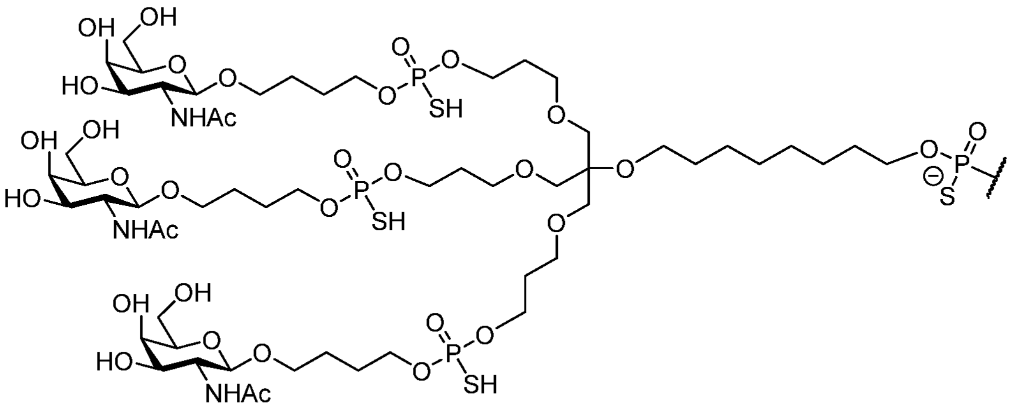

or

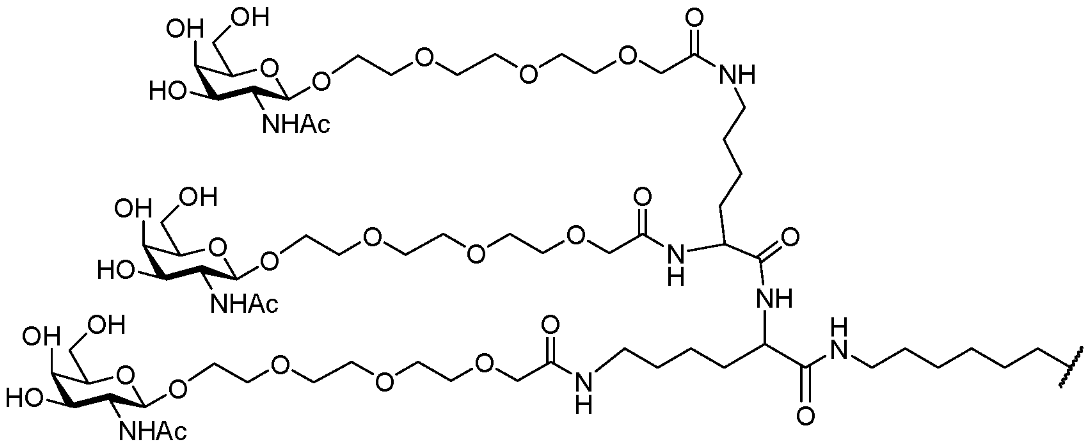

or

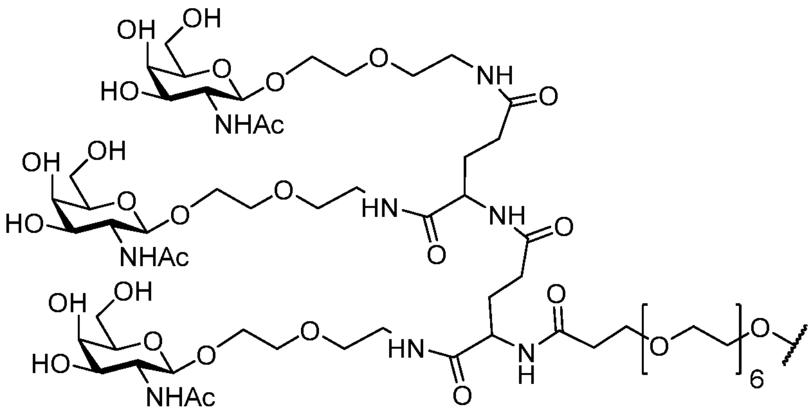

.

**11.** The biological agent or pharmaceutical composition according to claim 10, wherein the N-acetylgalactosamine derivative is conjugated to the 3'- terminus of the sense strand of the siRNA, as illustrated in the following schematic:

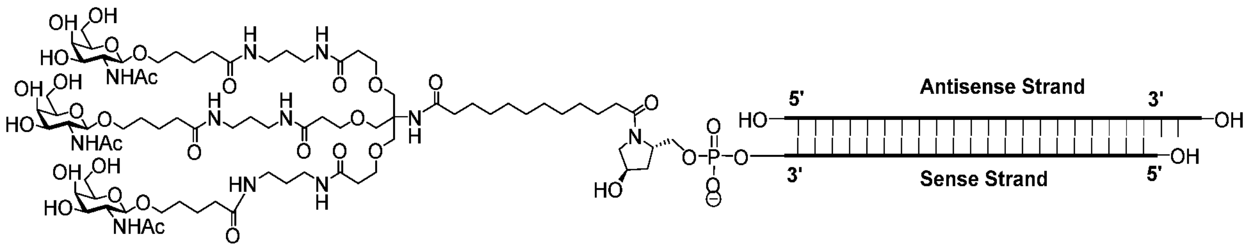


or

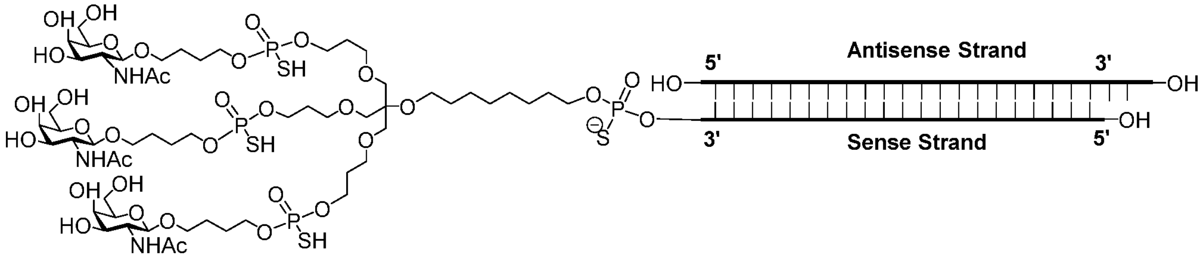


or

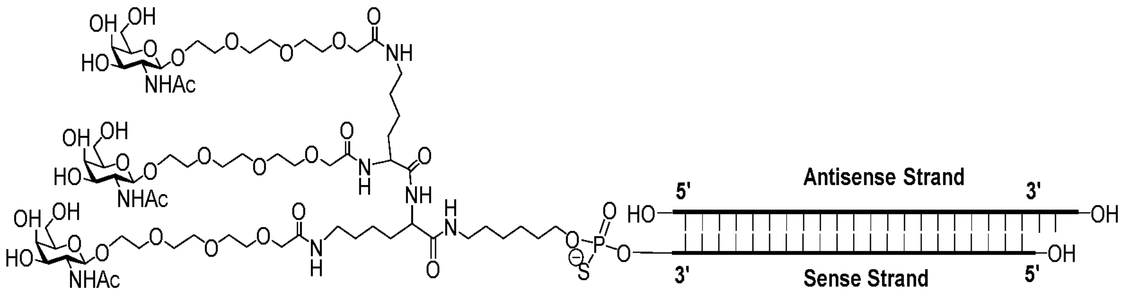


**12.** The biological agent or pharmaceutical composition according to any one of claims 8 to 11, for use in the manufacture of a medicament for the inhibition of TMPRSS6 expression in cells.

**13.** The biological agent or pharmaceutical composition according to any one of claims 8 to 11, for use in the manufacture of a medicament for preventing or treating a disorder associated with iron excess or iron overload.

**14.** The use according to claim 12 or 13, wherein the disorder associated with TMPRSS6 expression or diseases associated with iron excess or iron overload are selected from polycythemia,thalassemia,hemochromatosis, myelodysplastic syndrome, porphyria cutanea tarda, aplastic anemia, sideroblastic anemia, iron-refractory iron deficiency anemia, hereditary anemia, severe chronic hemolytic anemia, Parkinson's disease, Alzheimer's disease, Friedreich's ataxia, or microcytic anemia, transfusion-induced iron overload, and iron overload associated with non-alcoholic fatty liver disease (NAFLD).

**15.** The use according to claim 14, wherein the thalassemia is selected from $\alpha$-thalassemia, $\beta$-thalassemia, or $\delta$-thalassemia.

**16.** The use according to claim 14, wherein the polycythemia is polycythemia vera.

**17.** The use according to claim 14, wherein the hereditary anemia is selected from sickle-cell anemia, thalassemia, Fanconi anemia, Diamond-Blackfan anemia, Shwachman-Diamond syndrome, red blood cell membrane disorders, glucose-6-phosphate dehydrogenase deficiency, and hereditary hemorrhagic telangiectasia.

**18.** A method of inhibiting TMPRSS6 expression in a cell, the method comprising: (a) contacting the cell with the siRNA or the pharmaceutically acceptable salt thereof according to claim 1, or the biological agent or pharmaceutical

composition according to claim 8; and (b) maintaining the cell produced in step (a) for a time sufficient to obtain degradation of the mRNA transcript of a TMPRSS6 gene, thereby inhibiting expression of the TMPRSS6 gene in the cell.

**19.** A method of treating a subject suffering from a TMPRSS6-related disorder or a disease associated with iron excess or iron overload, comprising administering to the subject a therapeutically effective amount of an siRNA or a pharmaceutically acceptable salt thereof according to claim 1, or the biological agent or pharmaceutical composition according to claim 8, thereby treating the subject.

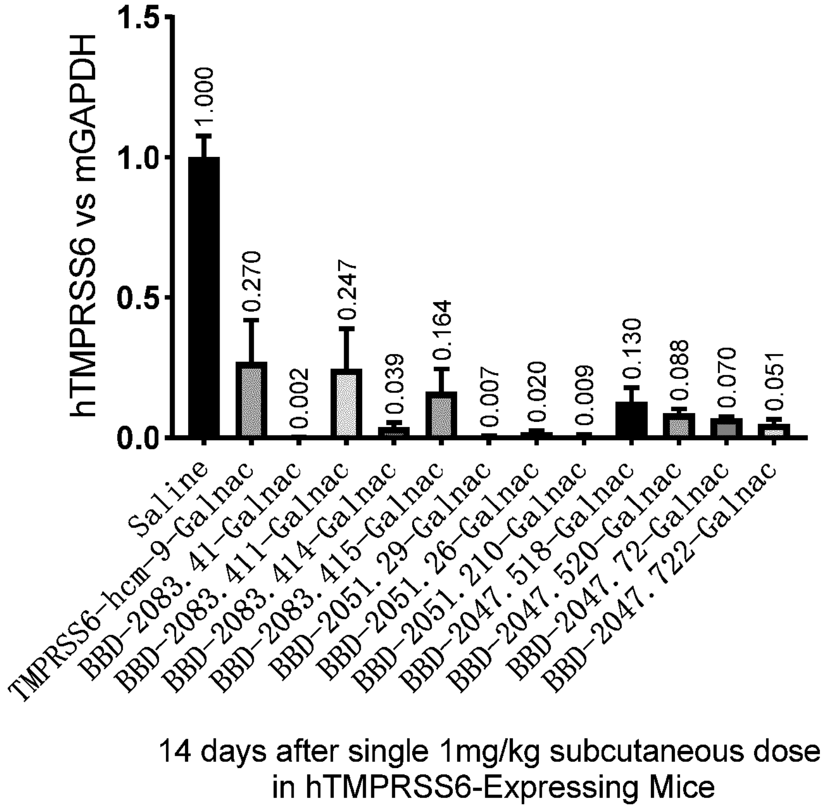
hTMPRSS6 vs mGAPDH
1.5
1.0
0.5
0.0
1.000
0.270
0.002
0.247
0.039
0.164
0.007
0.020
0.009
0.130
0.088
0.070
0.051
Saline
TMPRSS6-hcm-9-Galnac
BBD-2083. 41-Galnac
BBD-2083. 411-Galnac
BBD-2083. 414-Galnac
BBD-2083. 415-Galnac
BBD-2051. 29-Galnac
BBD-2051. 26-Galnac
BBD-2051. 210-Galnac
BBD-2047. 518-Galnac
BBD-2047. 520-Galnac
BBD-2047. 72-Galnac
BBD-2047. 722-Galnac
14 days after single 1mg/kg subcutaneous dose
in hTMPRSS6-Expressing Mice

FIG. 1

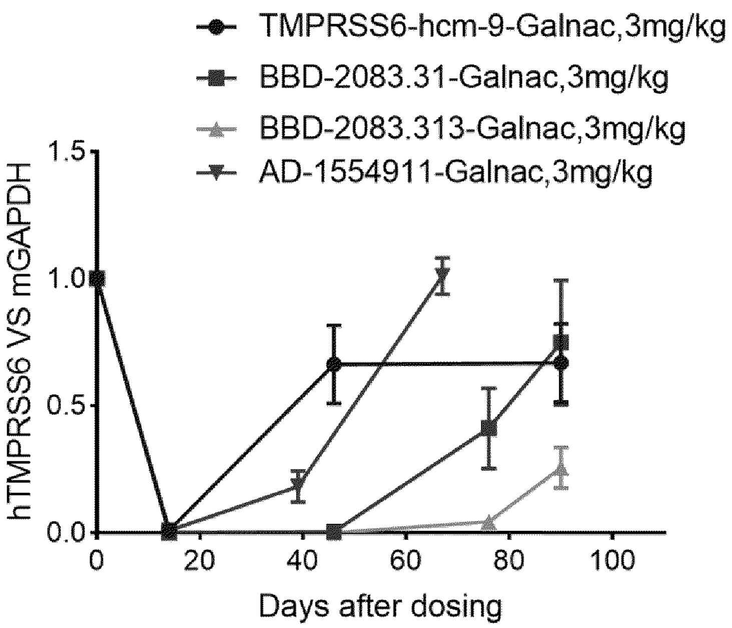
TMPRSS6-hcm-9-Galnac,3mg/kg
BBD-2083.31-Galnac,3mg/kg
BBD-2083.313-Galnac,3mg/kg
AD-1554911-Galnac,3mg/kg
hTMPRSS6 VS mGAPDH
1.5
1.0
0.5
0.0
0
20
40
60
80
100
Days after dosing

FIG. 2

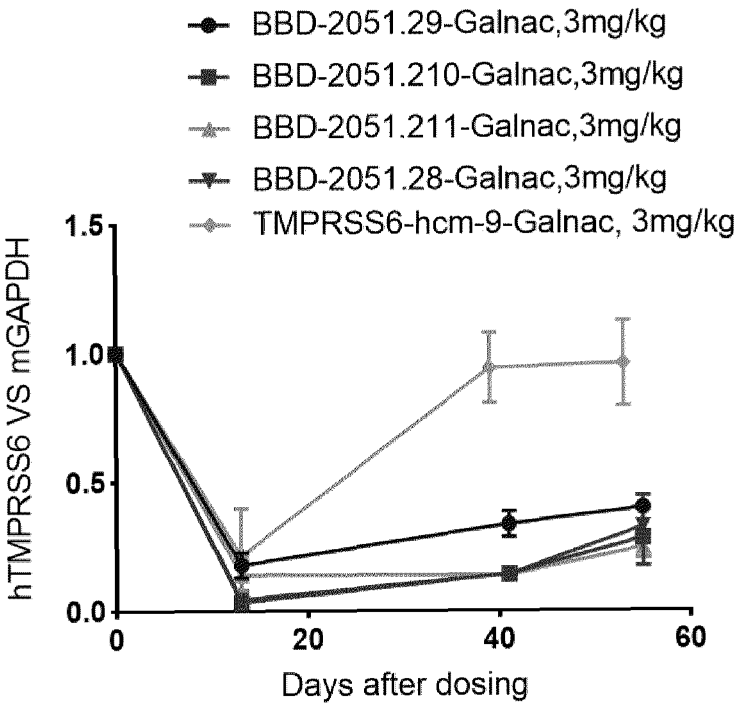
BBD-2051.29-Galnac,3mg/kg
BBD-2051.210-Galnac,3mg/kg
BBD-2051.211-Galnac,3mg/kg
BBD-2051.28-Galnac,3mg/kg
TMPRSS6-hcm-9-Galnac, 3mg/kg
hTMPRSS6 VS mGAPDH
1.5
1.0
0.5
0.0
0
20
40
60
Days after dosing

FIG. 3

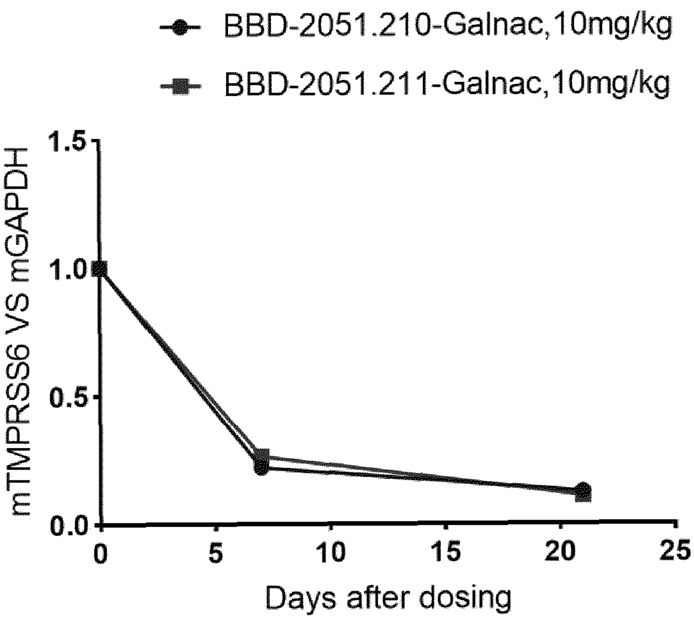
BBD-2051.210-Galnac,10mg/kg
BBD-2051.211-Galnac,10mg/kg
mTMPRSS6 VS mGAPDH
1.5
1.0
0.5
0.0
0
5
10
15
20
25
Days after dosing

FIG. 4

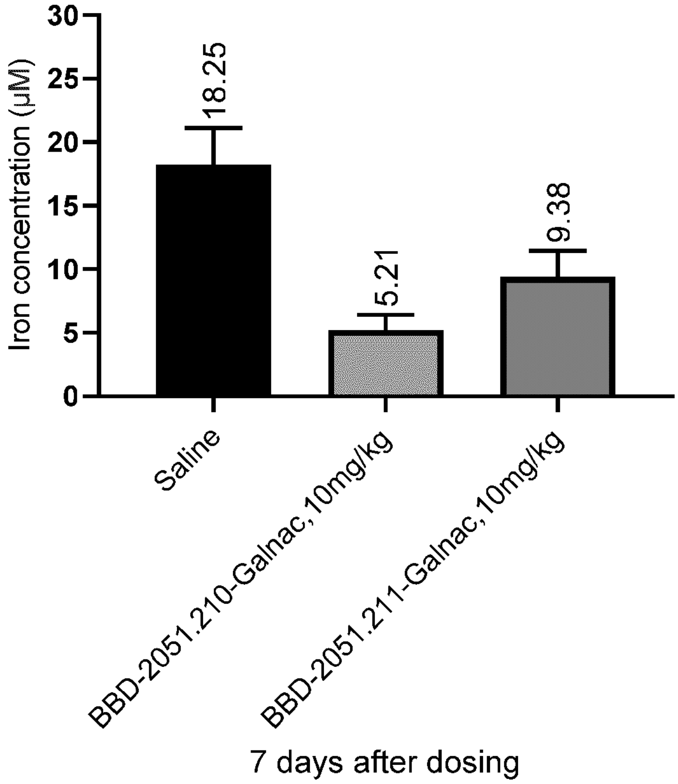
Iron concentration (μM)
30
25
20
15
10
5
0
18.25
5.21
9.38
Saline
BBD-2051.210-Galnac,10mg/kg
BBD-2051.211-Galnac,10mg/kg
7 days after dosing

FIG. 5

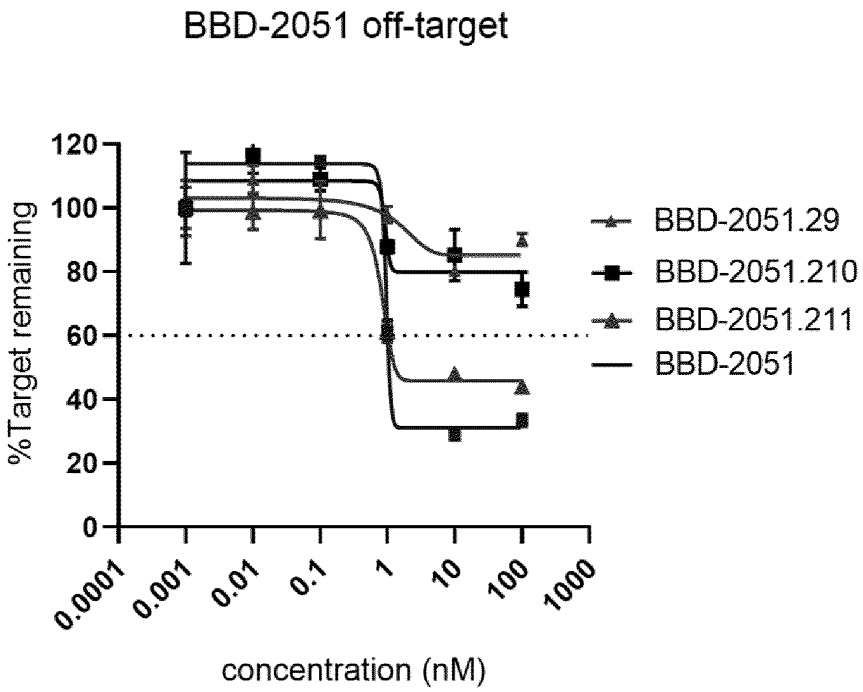
BBD-2051 off-target
%Target remaining
120
100
80
60
40
20
0
0.0001
0.001
0.01
0.1
1
10
100
1000
concentration (nM)
BBD-2051.29
BBD-2051.210
BBD-2051.211
BBD-2051

FIG. 6

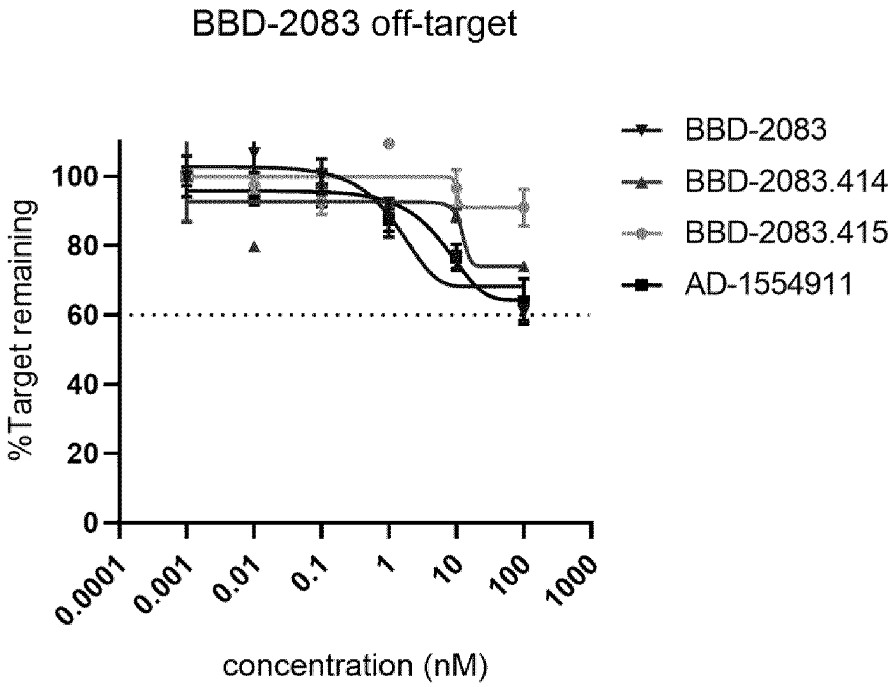
BBD-2083 off-target
%Target remaining
100
80
60
40
20
0
0.0001
0.001
0.01
0.1
1
10
100
1000
concentration (nM)
BBD-2083
BBD-2083.414
BBD-2083.415
AD-1554911

FIG. 7

## INTERNATIONAL SEARCH REPORT

International application No.
**PCT/CN2023/095104**

**A. CLASSIFICATION OF SUBJECT MATTER**

C12N15/113(2010.01)i; A61K31/713(2006.01)i; A61P7/00(2006.01)i; A61P7/06(2006.01)i

According to International Patent Classification (IPC) or to both national classification and IPC

**B. FIELDS SEARCHED**

Minimum documentation searched (classification system followed by classification symbols)

IPC: C12N, A61K, A61P

Documentation searched other than minimum documentation to the extent that such documents are included in the fields searched

Electronic data base consulted during the international search (name of data base and, where practicable, search terms used)

CNABS, SIPOABS, DWPI, CNTXT, WOTXT, USTXT, EPTXT, JPTXT, KRTXT, CNKI, Web of Science, Elsevier Science, STN, GenBank, EMBL, 中国专利生物序列检索系统, China Patent Biological Sequence Search System, 必应搜索, Bing Search, 超星读秀, CHAOXING DUXIU: 跨膜丝氨酸蛋白酶6, 蛋白裂解酶-2, 间质蛋白酶-2, RNA干扰, 小干扰RNA, Transmembrane protease serine 6, Matriptase-2, RNA interference, Small Interfering RNA, TMPRSS6, MTP-2, RNAi, siRNA, dsRNA, 对SEQ ID NOs: 95, 96, 103, 104, 167, 168, 173-176进行序列检索, search for SEQ ID NOs: 95, 96, 103, 104, 167, 168 and 173-176

**C. DOCUMENTS CONSIDERED TO BE RELEVANT**

| Category* | Citation of document, with indication, where appropriate, of the relevant passages | Relevant to claim No. |
|---|---|---|
| A | CN 113164768 A (SILENCE THERAPEUTICS GMBH) 23 July 2021 (2021-07-23)<br>see claims 6-7, and description, paragraphs 10-11, table 1, and sequence listing | 1-17 |
| A | CN 105452463 A (ALNYLAM PHARMACEUTICALS INC.) 30 March 2016 (2016-03-30)<br>see claims 1-3, tables 2 and 4, and sequence listing | 1-17 |
| A | CN 107429250 A (IONIS PHARMACEUTICALS, INC.) 01 December 2017 (2017-12-01)<br>see description, tables 1, 2 and 3 | 1-17 |
| A | CN 110573619 A (SILENCE THERAPEUTICS GMBH) 13 December 2019 (2019-12-13)<br>see claims 1-3 | 1-17 |
| A | US 2014194489 A1 (ALNYLAM PHARMACEUTICALS INC. et al.) 10 July 2014 (2014-07-10)<br>see claims 1-14 | 1-17 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 September 2023** | **30 September 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/095104**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**

International application No.
**PCT/CN2023/095104**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **18-19**
because they relate to subject matter not required to be searched by this Authority, namely:

The subject matter of claims 18-19 is a method for treatment of the human or animal body, which falls within subject matter for which no search is required (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/095104**

| Patent document cited in search report | Publication date (day/month/year) | Patent family member(s) | Publication date (day/month/year) |
|---|---|---|---|
| CN 113164768 A | 23 July 2021 | BR 112021001181 A2 | 27 April 2021 |
| | | JP 2021531029 A | 18 November 2021 |
| | | EP 3598995 A1 | 29 January 2020 |
| | | WO 2020021108 A2 | 30 January 2020 |
| | | WO 2020021108 A3 | 30 April 2020 |
| | | EP 3826720 A2 | 02 June 2021 |
| | | CA 3106854 A1 | 30 January 2020 |
| | | AU 2019308710 A1 | 04 March 2021 |
| | | KR 20210040079 A | 12 April 2021 |
| | | US 2022331351 A1 | 20 October 2022 |
| CN 105452463 A | 30 March 2016 | AU 2023201014 A1 | 25 May 2023 |
| | | IL 285780 A | 30 September 2021 |
| | | IL 285780 B | 01 July 2022 |
| | | EP 3587578 A1 | 01 January 2020 |
| | | AP 201508862 A0 | 30 November 2015 |
| | | CL 2015003440 A1 | 09 September 2016 |
| | | SG 11201510565 TA | 28 January 2016 |
| | | CA 3178370 A1 | 27 November 2014 |
| | | JP 2019214587 A | 19 December 2019 |
| | | JP 2023061967 A | 02 May 2023 |
| | | IL 280153 A | 01 March 2021 |
| | | IL 280153 B | 30 September 2021 |
| | | AP 201508862 D0 | 30 November 2015 |
| | | US 2016145629 A1 | 26 May 2016 |
| | | US 9783806 B2 | 10 October 2017 |
| | | KR 20210037730 A | 06 April 2021 |
| | | KR 102365809 B1 | 23 February 2022 |
| | | AU 2014268529 A1 | 17 December 2015 |
| | | AU 2014268529 B2 | 30 April 2020 |
| | | AU 2014268529 C1 | 01 October 2020 |
| | | KR 20220025273 A | 03 March 2022 |
| | | KR 102486617 B1 | 12 January 2023 |
| | | MY 181888 A | 12 January 2021 |
| | | BR 112015029276 A2 | 19 September 2017 |
| | | BR 112015029276 B1 | 12 July 2022 |
| | | TW 202124715 A | 01 July 2021 |
| | | EP 3828276 A1 | 02 June 2021 |
| | | US 2019211341 A1 | 11 July 2019 |
| | | US 10913950 B2 | 09 February 2021 |
| | | NZ 714577 A | 26 November 2021 |
| | | IL 293657 A | 01 August 2022 |
| | | KR 20160009687 A | 26 January 2016 |
| | | KR 102234623 B1 | 02 April 2021 |
| | | WO 2014190157 A1 | 27 November 2014 |
| | | WO 2014190157 A8 | 19 November 2015 |
| | | US 2021254076 A1 | 19 August 2021 |
| | | SG 10202103166 XA | 29 April 2021 |
| | | UY 35583 A | 31 October 2014 |
| | | JP 2021075554 A | 20 May 2021 |
| | | EP 2999786 A1 | 30 March 2016 |

Form PCT/ISA/210 (patent family annex) (July 2022)

**INTERNATIONAL SEARCH REPORT**
**Information on patent family members**

International application No.
**PCT/CN2023/095104**

| Patent document cited in search report | Publication date (day/month/year) | Patent family member(s) | Publication date (day/month/year) |
|---|---|---|---|
| | | CL 2021002998 A1 | 09 September 2022 |
| | | US 2020392511 A1 | 17 December 2020 |
| | | US 10988768 B2 | 27 April 2021 |
| | | RU 2015154738 A | 27 June 2017 |
| | | TW 201536916 A | 01 October 2015 |
| | | TWI 727917 B | 21 May 2021 |
| | | HK 1221978 A1 | 16 June 2017 |
| | | JP 2016520593 A | 14 July 2016 |
| | | US 2018087058 A1 | 29 March 2018 |
| | | CA 2912834 A1 | 27 November 2014 |
| | | IL 242701 B | 31 January 2021 |
| | | AU 2020207874 A1 | 13 August 2020 |
| | | AU 2020207874 B2 | 24 November 2022 |
| | | EA 201592225 A1 | 29 April 2016 |
| CN 107429250 A | 01 December 2017 | US 2022259600 A1 | 18 August 2022 |
| | | KR 20170129263 A | 24 November 2017 |
| | | KR 102539587 B1 | 01 June 2023 |
| | | CA 2978100 A1 | 06 October 2016 |
| | | HK 1249140 A1 | 26 October 2018 |
| | | AU 2016244106 A1 | 03 August 2017 |
| | | AU 2016244106 B2 | 19 May 2022 |
| | | JP 2021074010 A | 20 May 2021 |
| | | JP 7037681 B2 | 16 March 2022 |
| | | EP 3277817 A1 | 07 February 2018 |
| | | EP 3277817 A4 | 05 December 2018 |
| | | JP 2022104917 A | 12 July 2022 |
| | | IL 286669 A | 31 October 2021 |
| | | US 2018105817 A1 | 19 April 2018 |
| | | US 10415038 B2 | 17 September 2019 |
| | | US 2020190522 A1 | 18 June 2020 |
| | | IL 253509 A0 | 28 September 2017 |
| | | IL 253509 B1 | 01 April 2023 |
| | | IL 253509 B2 | 01 August 2023 |
| | | BR 112017015864 A2 | 31 July 2018 |
| | | JP 2018511307 A | 26 April 2018 |
| | | JP 6833705 B2 | 24 February 2021 |
| | | RU 2017137410 A | 06 May 2019 |
| | | RU 2017137410 A3 | 21 January 2020 |
| | | MX 2017012426 A | 26 January 2018 |
| | | WO 2016161429 A1 | 06 October 2016 |
| CN 110573619 A | 13 December 2019 | AU 2019247637 A1 | 22 October 2020 |
| | | BR 112019019921 A2 | 22 April 2020 |
| | | CA 3095523 A1 | 10 October 2019 |
| | | EP 3607068 A1 | 12 February 2020 |
| | | EP 3607068 B1 | 02 November 2022 |
| | | DK 3607068 T3 | 23 January 2023 |
| | | KR 20190137842 A | 11 December 2019 |
| | | PL 3607068 T3 | 20 March 2023 |
| | | ES 2936109 T3 | 14 March 2023 |
| | | JP 2020512838 A | 30 April 2020 |

Form PCT/ISA/210 (patent family annex) (July 2022)

**INTERNATIONAL SEARCH REPORT**
**Information on patent family members**

International application No.
**PCT/CN2023/095104**

| Patent document cited in search report | Publication date (day/month/year) | Patent family member(s) | Publication date (day/month/year) |
|---|---|---|---|
| | | JP 7247167 B2 | 28 March 2023 |
| | | RS 63887 B1 | 28 February 2023 |
| | | CA 3057561 A1 | 11 October 2018 |
| | | FI 3607068 T3 | 19 February 2023 |
| | | HRP 20221547 T1 | 03 March 2023 |
| | | LT 3607068 T | 10 February 2023 |
| | | JP 2023068035 A | 16 May 2023 |
| | | SI 3607068 T1 | 28 February 2023 |
| | | AU 2018247924 A1 | 14 November 2019 |
| | | AU 2018247924 B2 | 02 February 2023 |
| | | WO 2018185240 A1 | 11 October 2018 |
| | | US 2020208158 A1 | 02 July 2020 |
| | | US 11174483 B2 | 16 November 2021 |
| | | IL 269576 A | 28 November 2019 |
| | | US 2022025377 A1 | 27 January 2022 |
| | | JP 2021520207 A | 19 August 2021 |
| | | EP 4219714 A2 | 02 August 2023 |
| | | EP 4219714 A3 | 30 August 2023 |
| | | IL 277636 A | 30 November 2020 |
| US 2014194489 A1 | 10 July 2014 | JP 2017070309 A | 13 April 2017 |
| | | JP 6416295 B2 | 31 October 2018 |
| | | RU 2013148024 A | 10 May 2015 |
| | | RU 2702501 C2 | 08 October 2019 |
| | | US 2016145626 A1 | 26 May 2016 |
| | | US 10100312 B2 | 16 October 2018 |
| | | AU 2012236700 A1 | 03 October 2013 |
| | | AU 2012236700 B2 | 15 June 2017 |
| | | MX 2013011177 A | 06 December 2013 |
| | | MX 343008 B | 21 October 2016 |
| | | EP 3674409 A1 | 01 July 2020 |
| | | CA 2831284 A1 | 04 October 2012 |
| | | BR 112013025006 A2 | 17 January 2017 |
| | | BR 112013025006 B1 | 15 June 2021 |
| | | US 2019119685 A1 | 25 April 2019 |
| | | US 11198876 B2 | 14 December 2021 |
| | | KR 20140031877 A | 13 March 2014 |
| | | KR 102104401 B1 | 27 April 2020 |
| | | MX 360349 B | 30 October 2018 |
| | | JP 2014518612 A | 07 August 2014 |
| | | JP 6108628 B2 | 05 April 2017 |
| | | AU 2017203417 A1 | 08 June 2017 |
| | | AU 2017203417 B2 | 01 August 2019 |
| | | JP 2021006029 A | 21 January 2021 |
| | | JP 7150789 B2 | 11 October 2022 |
| | | AU 2019257363 A1 | 14 November 2019 |
| | | AU 2019257363 B2 | 16 December 2021 |
| | | JP 2022180576 A | 06 December 2022 |
| | | PH 12017501501 A1 | 14 January 2019 |
| | | US 9175290 B2 | 03 November 2015 |
| | | AU 2022201595 A1 | 31 March 2022 |

Form PCT/ISA/210 (patent family annex) (July 2022)

**INTERNATIONAL SEARCH REPORT**
**Information on patent family members**

International application No.
**PCT/CN2023/095104**

| Patent document cited in search report | Publication date (day/month/year) | Patent family member(s) | Publication date (day/month/year) |
|---|---|---|---|
| | | US 2022251570 A1 | 11 August 2022 |
| | | WO 2012135246 A2 | 04 October 2012 |
| | | WO 2012135246 A3 | 01 May 2014 |
| | | EP 2691121 A2 | 05 February 2014 |
| | | EP 2691121 A4 | 26 August 2015 |
| | | KR 20210082542 A | 05 July 2021 |
| | | KR 102365961 B1 | 23 February 2022 |
| | | JP 2019000123 A | 10 January 2019 |
| | | SG 10201602369 PA | 30 May 2016 |
| | | KR 20200046114 A | 06 May 2020 |
| | | KR 102271245 B1 | 02 July 2021 |
| | | SG 193923 A1 | 29 November 2013 |
| | | KR 20220025937 A | 03 March 2022 |
| | | KR 102481317 B1 | 26 December 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN CN2022112033373
- CN CN2023100881658
- US 11174483 B2


### Non-patent literature cited in the description


- **JACKSON et al.** *RNA*, 2006, vol. 12, 1179-1187
- **WEINGARTNER et al.** *Mol Ther Nucleic Acids*, 2020, vol. 21, 242-250
- **CAO** ; **KAN**. *Cold Spring Harb Perspect Med*, 2013, vol. 3, a011775
- **RIVELLA**. *Haematologica*, 2015, vol. 100, 418-430
- **MUSALLAM et al.** *Am J Hematol*, 2021, vol. 96, 1518-1531
- **RICHARD et al.** *Am J Hematol*, 2020, vol. 95, 68-77
- **NUALKAEW et al.** *Mol Ther*, 2021, vol. 29, 2841-2853
- **BELIVEAU et al.** *Cell Chem Biol*, 2019, vol. 26, 1559-1572
- **GINZBURG** ; **RIVELLA**. *Blood*, 2011, vol. 118, 4321-4330
- **LONGO et al.** *Int J Mol Sci*, 2021, vol. 22